(19)

Europäisches
Patentamt
European
Patent Office
Office européen
des brevets

(11) **EP 3 928 793 A1**

(12) **EUROPEAN PATENT APPLICATION**
published in accordance with Art. 153(4) EPC

(43) Date of publication:
**29.12.2021 Bulletin 2021/52**

(21) Application number: **20759108.2**

(22) Date of filing: **19.02.2020**

(51) Int Cl.:
$A61K\ 39/395^{(2006.01)}$          $A61K\ 45/00^{(2006.01)}$
$A61P\ 35/00^{(2006.01)}$          $A61P\ 43/00^{(2006.01)}$
$C07K\ 16/28^{(2006.01)}$          $C12Q\ 1/02^{(2006.01)}$
$C12Q\ 1/06^{(2006.01)}$          $G01N\ 33/48^{(2006.01)}$
$G01N\ 33/49^{(2006.01)}$          $G01N\ 33/68^{(2006.01)}$

(86) International application number:
**PCT/JP2020/006620**

(87) International publication number:
**WO 2020/171141 (27.08.2020 Gazette 2020/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.02.2019 JP 2019028507**
        **14.11.2019 JP 2019206277**

(71) Applicant: **Saitama Medical University**
**Iruma-gun, Saitama 350-0495 (JP)**

(72) Inventor: **KAGAMU Hiroshi**
**Iruma-gun, Saitama 350-0495 (JP)**

(74) Representative: **Uexküll & Stolberg**
**Partnerschaft von**
**Patent- und Rechtsanwälten mbB**
**Beselerstraße 4**
**22607 Hamburg (DE)**

(54) **METHOD AND COMPOSITION FOR PREDICTING LONG-TERM SURVIVAL IN CANCER IMMUNOTHERAPY**

(57)    Provided is a peripheral blood biomarker for predicting the long-term survival/need for therapeutic intervention in cancer immunotherapy. The present invention provides a method that uses a composition of a cell subpopulation in a sample obtained from a subject as an indicator to predict the long-term survival of the subject in cancer immunotherapy. The long-term survival/need for therapeutic intervention in a subject in cancer immunotherapy can be predicted by comparing the level of a CD4+ T cell subpopulation that correlates with dendritic cell stimulation in an antitumor immune response or a dendritic cell subpopulation that correlates with dendritic cell stimulation in an antitumor immune response with a reference standard.

FIG.9

**EP 3 928 793 A1**

**Description**

[Technical Field]

The present invention is associated with the field of cancer therapy. In particularly, the present invention relates to prediction of long-term survival in cancer immunotherapy.

[Background Art]

Efficacy of immune checkpoint inhibitors whose mechanism of action is PD-1/PD-L1 inhibition has been demonstrated in many carcinomas such as melanoma, lung cancer, head and neck cancer, urological cancer, and gastric cancer, so that immune checkpoint inhibitors are covered under insurance in Japan. It is demonstrated in clinical trials that long-term survival can be achieved in 10 to 20% of cases regardless of carcinoma. It is reported in a study on lung cancer that such long-term survival attained progression free survival of 5 years or longer even after discontinuing therapy in 2 years. While some factors such as tumor PD-L1 and tumor mutation burden have been studied as a biomarker associated with short-term response, biomarkers for predicting a long-term survival group have not been studied whatsoever.

The percentage of tumor PD-L1 expression is used in lung cancer as a biomarker for predicting short-term response to a PD-1/PD-L1 inhibitor. However, the correlation with response in cancer other than lung cancer is not clear, and AUC is only about 0.6 to 0.7 in ROC analysis for lung cancer. A fundamental study reports that an antitumor effect of a PD-1/PD-L1 inhibitor is also achieved even when using a tumor with PD-L1 knocked out by genome editing. Since an antitumor effect is eliminated by knocking out PD-L1 of a host, it is understood that PD-L1 expression on an antigen presenting cell is important. Although tumor mutation burden is a promising biomarker for predicting short-term response, AUC is only about 0.6 to 0.7 in ROC analysis.

The response rate is not necessarily high for treatment using cancer immunotherapy alone. For example, anti-PD-1 antibodies appear to have achieved significant clinical success, but about 40% of patients are found to be a part of a "non-responder group" whose disease progresses within three months in nearly all anti-PD-1 antibody clinical trials in view of data on progression free survival (PFS). Means for improving a low response rate with monotherapy includes combination therapy. While development of therapy concomitantly using a PD-1 inhibitor with a cytotoxic anticancer agent or other immune checkpoint inhibitor is ongoing, combination therapy faces a problem of being toxic.

[Summary of Invention]

[Solution to Problem]

The inventor has already discovered that each of the three groups, for which therapeutic effects from cancer immunotherapy (e.g., anti-PD-1 therapy or anti-PD-LI therapy) fall under progressive disease (PD), stable disease (SD), or response (complete response (CR) + partial response (PR)), exhibits different immunological states. The inventor has already provided a method of predicting a response to cancer immunotherapy as one of progressive disease (PD), stable disease (SD), and response (complete response (CR) + partial response (PR)) when cancer immunotherapy is administered to a subject (it should be noted that the present invention can detect a population of subjects to be the same as a partial response group (PR) when a complete response group (CR) is included in addition to a partial response group (PR) or when a complete response group (CR) is included without a partial response group (PR)).

The present invention provides a method of using a composition of a cell subpopulation in a sample obtained from a subject as an indicator for predicting long-term survival in cancer immunotherapy in the subject. The presence/absence and/or degree of long-term survival in cancer immunotherapy in a subject can be predicted by comparing the amount of a specific cell subpopulation described herein with a baseline.

Examples of cell subpopulations that can be used as an indicator in the present invention include, but are not limited to, a CD4$^+$ T cell subpopulation correlated with dendritic cell stimulation in an antitumor immune response, a dendritic cell subpopulation correlated with dendritic cell stimulation in an antitumor immune response, and a CD8$^+$ T cell subpopulation correlated with dendritic cell stimulation in an antitumor immune response. A CD4$^+$ T cell subpopulation correlated with dendritic cell stimulation in an antitumor immune response is, for example, a cell subpopulation within a CD62L$^{low}$CD4$^+$ T cell population (e.g., CD62L$^{low}$CD4$^+$ T cell subpopulation itself, ICOS$^+$CD62L$^{low}$CD4$^+$ T cell subpopulation, and the like). A dendritic cell subpopulation correlated with dendritic cell stimulation in an antitumor immune response is, for example, an HLA-DR$^+$CD141$^+$CD11c$^+$ cell subpopulation or the like. A CD8$^+$ T cell subpopulation correlated with dendritic cell stimulation in an antitumor immune response is, for example, a CD137$^+$CD62L$^{low}$CD8$^+$ T subpopulation or the like.

Another embodiment of the invention can provide an indicator of whether therapeutic intervention should be

2

administered to a subject or when therapeutic intervention should be administered by showing a prediction of long-term survival in cancer immunotherapy in the subject. It is understood that it can be advantageous to administer therapeutic intervention against cancer when long-term survival in caner immunotherapy is not predicted, but a biomarker for predicting long-term survival in cancer immunotherapy did not exist up to this point.

Typically, therapeutic intervention can be coadministered with one or more additional agents. Alternatively, therapeutic intervention can be combined with radiation therapy. One or more additional agents can be any chemotherapeutic drug, or a second immune checkpoint inhibitor can be included. Alternatively, another cancer therapy used in therapeutic intervention can be other cancer immunotherapy (e.g., adoptive cell transfer), hyperthermia therapy, surgical procedure, or the like. Preferably, therapeutic intervention is combined with radiation therapy or cancer therapy comprising administration of an anticancer agent such as a chemotherapeutic agent.

Examples of embodiments of the inventions are shown in the following items.

(Item 1)

A method of using a composition of a cell subpopulation in a sample obtained from a subject as an indicator for predicting long-term survival in cancer immunotherapy in the subject, comprising:

a step of analyzing the composition of the cell subpopulation in the sample obtained from the subject;
wherein long-term survival in cancer immunotherapy in the subject is predicted by comparing an amount of a CD4+ T cell subpopulation correlated with dendritic cell stimulation in an antitumor immune response in the sample with a baseline.

(Item 2)

A method of using a composition of a cell subpopulation in a sample obtained from a subject as an indicator for predicting long-term survival in cancer immunotherapy in the subject, comprising:

a step of analyzing the composition of the cell subpopulation in the sample obtained from the subject;
wherein long-term survival in cancer immunotherapy in the subject is predicted by comparing an amount of a dendritic cell subpopulation correlated with dendritic cell stimulation in an antitumor immune response in the sample with a baseline.

(Item 3)

A method of using a composition of a cell subpopulation in a sample obtained from a subject as an indicator for predicting long-term survival in cancer immunotherapy in the subject, comprising:

a step of analyzing the composition of the cell subpopulation in the sample obtained from the subject;
wherein long-term survival in cancer immunotherapy in the subject is predicted by comparing an amount of a CD8+ T cell subpopulation correlated with dendritic cell stimulation in an antitumor immune response in the sample with a baseline.

(Item 4)

The method of any one of items 1 to 3, wherein the long-term survival in cancer immunotherapy in the subject is predicted by comparing at least two amounts selected from the group consisting of an amount of a CD4+ T cell subpopulation correlated with dendritic cell stimulation in an antitumor immune response, an amount of a dendritic cell subpopulation correlated with dendritic cell stimulation in an antitumor immune response, and an amount of a CD8+ T cell subpopulation correlated with dendritic cell stimulation in an antitumor immune response in the sample with a baseline.

(Item 5)

The method of item 1 or 4, wherein the CD4+ T cell subpopulation correlated with dendritic cell stimulation in an antitumor immune response is a cell subpopulation within a CD62L$^{low}$CD4+ T cell population.

(Item 6)

The method of item 5, wherein the CD4+ T cell subpopulation correlated with dendritic cell stimulation in an antitumor immune response is a CD62L$^{low}$CD4$^+$ T cell subpopulation.

(Item 7)

The method of item 5, wherein the CD4$^+$ T cell subpopulation correlated with dendritic cell stimulation in an antitumor immune response is an ICOS$^+$CD62L$^{low}$CD4$^+$ T cell subpopulation.

(Item 8)

The method of item 2 or 4, wherein the dendritic cell subpopulation correlated with dendritic cell stimulation in an antitumor immune response is an HLA-DR$^+$CD141$^+$CD11c$^+$ cell subpopulation.

(Item 9)

The method of item 3 or 4, wherein the CD8$^+$ T cell subpopulation correlated with dendritic cell stimulation in an antitumor immune response is a cell subpopulation within a CD62L$^{low}$CD8$^+$ T cell population.

(Item 10)

The method of item 9, wherein the CD8$^+$ T cell subpopulation correlated with dendritic cell stimulation in an antitumor immune response is a CD137$^+$CD62L$^{low}$CD8$^+$ T cell subpopulation.

(Item 11)

A method of using a relative value with respect to amounts (X, Y) selected from the group consisting of:

an amount of a CD4$^+$ T cell subpopulation correlated with dendritic cell stimulation in an antitumor immune response;
an amount of a dendritic cell subpopulation correlated with dendritic cell stimulation by a CD4$^+$ T cell in an antitumor immune response;
an amount of a CD8$^+$ T cell subpopulation correlated with dendritic cell stimulation in an antitumor immune response;
an amount of regulatory T cells or a CD4$^+$ T cell subpopulation correlated with regulatory T cells; and
an amount of an ICOS$^+$CD62L$^{low}$CD4$^+$ T cell subpopulation; as an indicator for predicting long-term survival in cancer immunotherapy in the subject; comprising:

a step of measuring X; and
a step of measuring Y;
wherein comparison of a relative value of X to Y with a baseline is used as an indicator for predicting long-term survival in cancer immunotherapy in the subject.

(Item 12)

The method of item 11, wherein the amounts (x) and (Y) are each selected from the group consisting of:

an amount of a CD62L$^{low}$CD4$^+$ T cell subpopulation;
an amount of a CCR7$^-$CD4$^+$ T cell subpopulation;
an amount of a LAG-3$^+$CD62L$^{low}$CD4$^+$ T cell subpopulation;
an amount of an ICOS$^+$CD62L$^{low}$CD4$^+$ T cell subpopulation;
an amount of a PD-1$^+$CD62L$^{low}$CD4$^+$ T cell subpopulation;
an amount of a Foxp3$^+$CD25$^+$CD4$^+$ T cell subpopulation;
an amount of an HLA-DR$^+$ dendritic cell subpopulation;
an amount of a CD80$^+$ dendritic cell subpopulation;
an amount of a CD86$^+$ dendritic cell subpopulation;
an amount of a PD-L1$^+$ dendritic cell subpopulation;
an amount of a CD62L$^{low}$CD8$^+$ T cell subpopulation;
an amount of a CD137$^+$CD8$^+$ T cell subpopulation; and
an amount of a CD28$^+$CD62L$^{low}$CD8$^+$ T cell subpopulation.

(Item 13)

The method of item 11, wherein

the amount (X) is an amount of a $CD62L^{low}CD4^+$ T cell subpopulation, and
(Y) is an amount of a $Foxp3^+CD25^+CD4^+$ T cell subpopulation.

(Item 14)

The method of any one of items 11 to 13, wherein the relative value is X/Y.

(Item 15)

The method of any one of items 11 to 13, wherein the relative value is $X^2/Y$.

(Item 16)

The method of any one of items 1 to 15, wherein the sample is a peripheral blood sample.

(Item 17)

The method of any one of items 1 to 16, wherein the baseline is an amount of the cell subpopulation in the sample of the subject before the cancer immunotherapy or a predetermined value.

(Item 18)

The method of any one of items 1 to 17, wherein the amount of the cell subpopulation in the sample which is greater than the baseline indicates that long-term survival in cancer immunotherapy in the subject is predicted.

(Item 19)

The method of any one of items 1 to 17, wherein the amount of the cell subpopulation in the sample which is less than the baseline indicates that long-term survival in cancer immunotherapy in the subject is not predicted.

(Item 20)

The method of any one of items 1 to 19, wherein no prediction of long-term survival in cancer immunotherapy in the subject further indicates that combination therapy should be administered to the subject.

(Item 21)

The method of any one of items 1 to 20 further defined as a method of using a composition of a cell subpopulation in a sample obtained at a plurality of points in time from a subject as an indicator for predicting long-term survival in cancer immunotherapy in the subject, the method comprising a step of analyzing the composition of the cell subpopulation in the sample obtained at the plurality of points in time from the subject.

(Item 22)

The method of item 15, wherein long-term survival is predicted if $X^2/Y$ is about 324 or greater.

(Item 23)

A pharmaceutical composition comprising an immune checkpoint inhibitor for treating cancer in a subject, wherein the pharmaceutical composition is administered to a subject predicted to have long-term survival in cancer immunotherapy in the subject by the method of any one of items 1 to 18 and 21 to 22.

(Item 24)

The pharmaceutical composition of item 23, wherein the immune checkpoint inhibitor is a PD-1 inhibitor and/or a PD-L1 inhibitor.

(Item 25)

A combination drug comprising an immune checkpoint inhibitor for treating cancer in a subject, wherein the combination drug is administered to a subject not predicted to have long-term survival in cancer immunotherapy in the subject by the method of any one of items 1 to 22.

(Item 26)

The combination drug of item 25, wherein the immune checkpoint inhibitor is a PD-1 inhibitor and/or a PD-L1 inhibitor.

(Item 27)

The combination drug of item 25, comprising a drug selected from the group consisting of a chemotherapeutic agent and additional cancer immunotherapy.

(Item 28)

A kit for determining whether long-term survival in cancer immunotherapy in a subject is predicted, comprising a detecting agent for a combination or markers selected from the group consisting of:

*a combination of CD4 and CD62L;
*a combination of CD4 and CCR7;
*a combination of CD4, CD62L, and LAG-3;
*a combination of CD4, CD62L, and ICOS;
*a combination of CD4, CD62L, and CD25;
*a combination of CD4, CD127, and CD25;
*a combination of CD4, CD45RA, and Foxp3;
*a combination of CD4, CD25, and Foxp3;
*a combination of CD11c, CD141, and HLA-DR;
*a combination of CD11c, CD141, and CD80;
*a combination of CD11c, CD123, and HLA-DR;
*a combination of CD11c, CD123, and CD80;
*a combination of CD8 and CD62L;
*a combination of CD8 and CD137; and
*a combination of CD28, CD62L, and CD8.

(Item 29)

A kit for determining whether therapeutic intervention is needed in cancer immunotherapy in a subject, comprising a detecting agent for a combination or markers selected from the group consisting of:

*a combination of CD4 and CD62L;
*a combination of CD4 and CCR7;
*a combination of CD4, CD62L, and LAG-3;
*a combination of CD4, CD62L, and ICOS;
*a combination of CD4, CD62L, and CD25;
*a combination of CD4, CD127, and CD25;
*a combination of CD4, CD45RA, and Foxp3;
*a combination of CD4, CD25, and Foxp3;
*a combination of CD11c, CD141, and HLA-DR;
*a combination of CD11c, CD141, and CD80;
*a combination of CD11c, CD123, and HLA-DR;

*a combination of CD11c, CD123, and CD80;
*a combination of CD8 and CD62L;
*a combination of CD8 and CD137; and
*a combination of CD28, CD62L, and CD8.

(Item 30)

A method of using a composition of a subpopulation in a sample obtained from a subject as an indicator of a need for therapeutic intervention in cancer immunotherapy in the subject, comprising:

a step of analyzing the composition of the cell subpopulation in the sample obtained from the subject;
wherein an indicator of a need for therapeutic intervention in cancer immunotherapy in the subject is provided by comparing an amount of a CD4$^+$ T cell subpopulation correlated with dendritic cell stimulation in an antitumor immune response in the sample with a baseline.

(Item 31)

The method of item 30, wherein the CD4$^+$ T cell subpopulation correlated with dendritic cell stimulation in an antitumor immune response is a cell subpopulation within a CD62L$^{low}$CD4$^+$ T cell population.

(Item 32)

The method of item 30, wherein the CD4$^+$ T cell subpopulation correlated with dendritic cell stimulation in an antitumor immune response is a CD62L$^{low}$CD4$^+$ T cell subpopulation.

(Item 33)

A method of using a relative value with respect to amounts (X, Y) selected from the group consisting of:

an amount of a CD4$^+$ T cell subpopulation correlated with dendritic cell stimulation in an antitumor immune response;
an amount of a dendritic cell subpopulation correlated with dendritic cell stimulation by a CD4$^+$ T cell in an antitumor immune response;
an amount of a CD8$^+$ T cell subpopulation correlated with dendritic cell stimulation in an antitumor immune response;
an amount of regulatory T cells or a CD4$^+$ T cell subpopulation correlated with regulatory T cells; and
an amount of an ICOS$^+$CD62L$^{low}$CD4$^+$ T cell subpopulation; as an indicator of a need for therapeutic intervention in cancer immunotherapy in the subject; comprising:

a step of measuring X; and
a step of measuring Y;
wherein comparison of a relative value of X to Y with a baseline is used as an indicator of a need for therapeutic intervention in cancer immunotherapy in the subject.

(Item 34)

The method of item 33, wherein the amounts (x) and (Y) are each selected from the group consisting of:

an amount of a CD62L$^{low}$CD4$^+$ T cell subpopulation;
an amount of a CCR7$^-$CD4$^+$ T cell subpopulation;
an amount of a LAG-3$^+$CD62L$^{low}$CD4$^+$ T cell subpopulation;
an amount of an ICOS$^+$CD62L$^{low}$CD4$^+$ T cell subpopulation;
an amount of a PD-1$^+$CD62L$^{low}$CD4$^+$ T cell subpopulation;
an amount of a Foxp3$^+$CD25$^+$CD4$^+$ T cell subpopulation;
an amount of an HLA-DR$^+$ dendritic cell subpopulation;
an amount of a CD80$^+$ dendritic cell subpopulation;
an amount of a CD86$^+$ dendritic cell subpopulation;
an amount of a PD-L1$^+$ dendritic cell subpopulation;
an amount of a CD62L$^{low}$CD8$^+$ T cell subpopulation;
an amount of a CD137$^+$CD8$^+$ T cell subpopulation; and

an amount of a $CD28^+CD62L^{low}CD8^+$ T cell subpopulation.

(Item 35)

The method of item 33, wherein

the amount (X) is an amount of a $CD62L^{low}CD4^+$ T cell subpopulation, and
(Y) is an amount of a $Foxp3^+CD25^+CD4^+$ T cell subpopulation.

(Item 36)

The method of any one of items 33 to 35, wherein the relative value is X/Y.

(Item 37)

The method of any one of items 33 to 35, wherein the relative value is $X^2/Y$.

(Item 38)

The method of any one of items 30 to 37, wherein the therapeutic intervention is radiation therapy.

(Item 39)

The method of any one of items 30 to 37, wherein the therapeutic intervention is chemotherapeutic agent therapy.

(Item 40)

The method of item 37, wherein $X^2/Y$ of less than about 324 is an indicator of a need for therapeutic intervention.

(Item 41)

The method of item 37, wherein $X^2/Y$ of about 174 or greater and less than about 324 is an indicator of a need for therapeutic intervention, wherein the therapeutic intervention comprises chemotherapy, radiation therapy, a surgical procedure, hyperthermia therapy, or additional cancer immunotherapy in addition to cancer immunotherapy being administered.

(Item 42)

The method of item 37, wherein $X^2/Y$ of less than about 174 is an indicator of a need for therapeutic intervention, wherein the therapeutic intervention comprises discontinuation of cancer immunotherapy being administered, or chemotherapy, radiation therapy, a surgical procedure, hyperthermia therapy, or additional cancer immunotherapy in addition to cancer immunotherapy being administered.

(Item 43)

A combination drug comprising an immune checkpoint inhibitor for treating cancer in a subject, wherein the combination drug is administered to a subject determined as needing therapeutic intervention in cancer immunotherapy in the subject by the method of any one of items 30 to 42.

(Item 44)

The combination drug of item 43, wherein the immune checkpoint inhibitor is a PD-1 inhibitor and/or a PD-L1 inhibitor.

(Item 45)

The combination drug of item 43, comprising a drug selected from the group consisting of a chemotherapeutic agent and additional cancer immunotherapy.

(Item 46)

A method of using a composition of a cell subpopulation in a sample obtained from a subject who is a cancer patient before therapy as an indicator for determining a therapeutic strategy for the subject, comprising:

a step of measuring an amount of a $CD62L^{low}CD4^+$ T cell subpopulation in the sample obtained from the subject (X) and an amount of a $Foxp3^+CD25^+CD4^+$ T cell subpopulation (Y);
a step of finding a relative value $X^2/Y$; and
a step selected from the group consisting of:

(a) a step of setting threshold value $\alpha$ for relative value $X^2/Y$ and determining a subject as a non-responder to cancer immunotherapy if $X^2/Y$ is less than threshold value $\alpha$;
(b) a step of setting threshold values $\alpha$ and $\beta$ for relative value $X^2/Y$ wherein $\alpha < \beta$, and determining a subject as a short-term responder to cancer immunotherapy if $X^2/Y$ is threshold value $\alpha$ or greater and less than threshold value $\beta$; or
(c) a step of setting threshold value $\beta$ for relative value $X^2/Y$ and determining a subject as a long-term responder to cancer immunotherapy if $X^2/Y$ is threshold value $\beta$ or greater.

(Item 47)

The method of item 46, wherein threshold value $\beta$ is a value that is at least 50 greater than threshold value $\alpha$.

(Item 48)

The method of item 47, wherein

threshold value $\alpha$ is a value within a range from 100 to 400, and
threshold value $\beta$ is a value within a range from 150 to 450.

(Item 49)

A product comprising a package insert describing the method of any one of items 46 to 48, and an immune checkpoint inhibitor.

[Advantageous Effects of Invention]

The present invention can predict long-term survival in cancer immunotherapy. This allows determination of whether therapeutic intervention should be administered in cancer immunotherapy or when therapeutic intervention should be administered.

[Brief Description of Drawings]

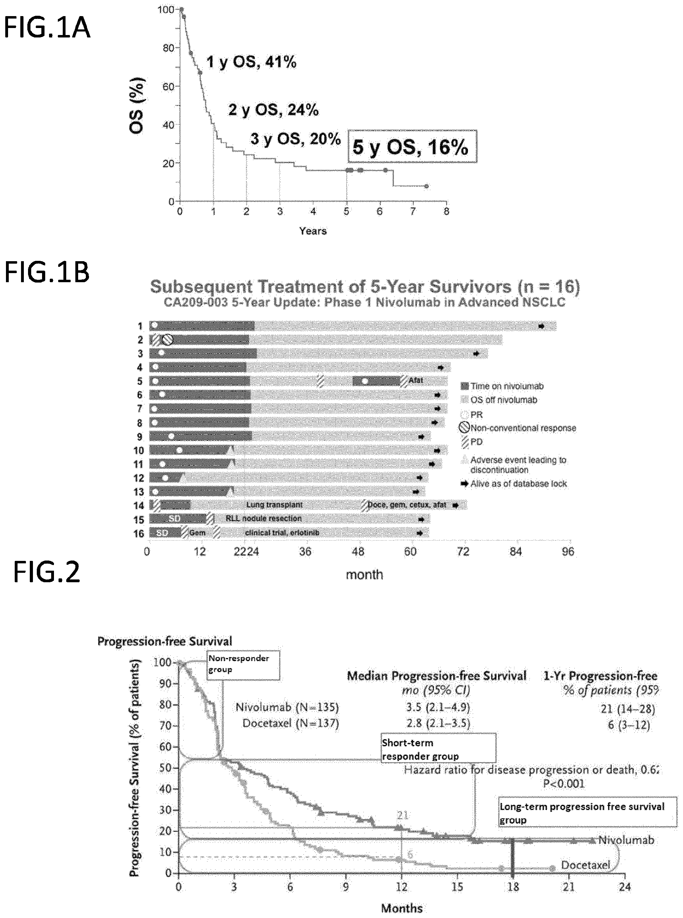

[Figure 1A] Figure **1A** is a diagram shown in Brahmer et al. (2017 AARC), i.e., a graph showing overall survival (OS, %).
[Figure 1B] Figure 1B is a diagram shown in Brahmer et al. (2017 AARC), i.e., a graph showing treatment of each patient (n = 16).
[Figure 2] Figure 2 is a graph modified from Brahmer et al. (N Eng J Med 2015: 373: 123-135).

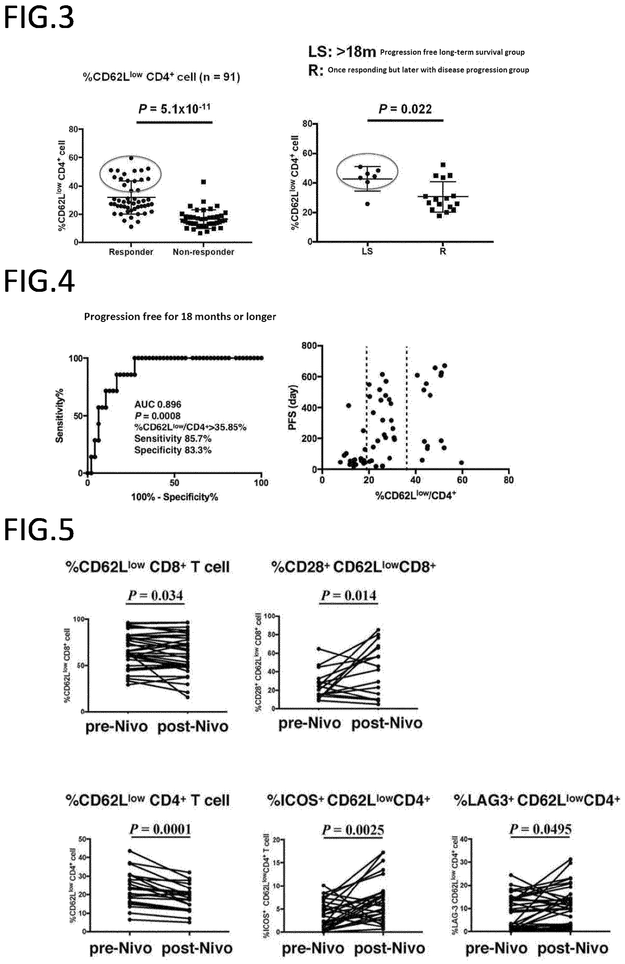

[Figure 3] The left diagram in Figure 3 is a diagram showing the percentage of $CD62L^{low}CD4^+$ T cells in non-responder groups with early progression in disease and other responder groups.
[Figure 4] Figure 4 is a diagram showing ROC analysis and PFS plot for the percentage of $CD62L^{low}CD4^+$ T cells.
[Figure 5] Figure 5 is the result of measuring the change in various markers from before nivolumab therapy (pre-Nivo) to after (post-Nivo).

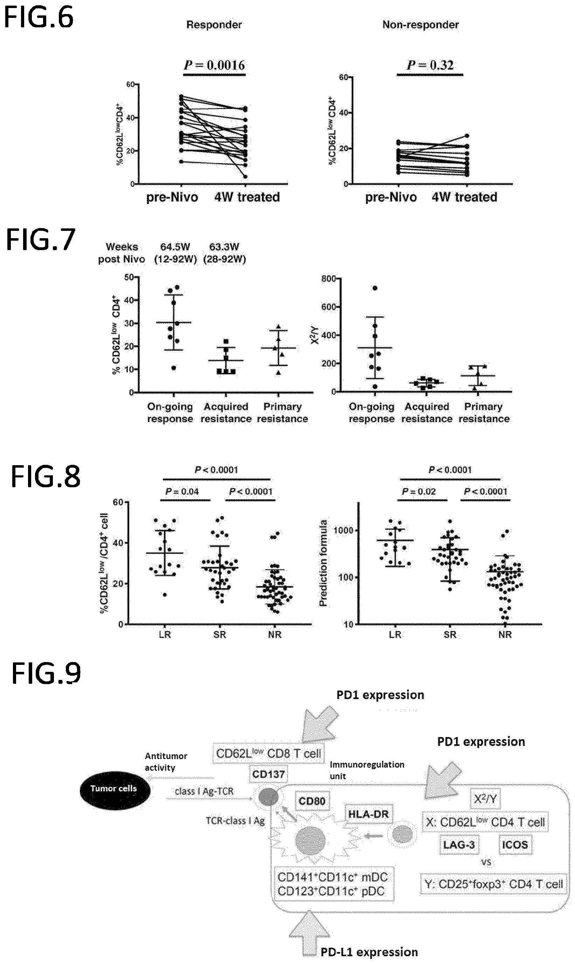

[Figure 6] Figure 6 is a graph showing the percentage of $CD62L^{low}CD4^+$ T cells before nivolumab therapy (pre-Nivo) and at 4 weeks after therapy (4W treated) in a responder group (Responder, left graph of Figure 6) and non-responder group (Non-responder, right graph of Figure 6).
[Figure 7] The left graph of Figure 7 is a graph showing the percentage of $CD62L^{low}CD4^+$ T cells for each of: patient group resistant to therapy as of the start of therapy (Primary resistance, right side of graph); patient group at an average of 63.3 weeks (28 to 92 weeks) after starting therapy, which has acquired therapeutic resistance after the start of therapy (Acquired resistance, middle of graph); and patient group at an average of 64.5 weeks (12 to 92 weeks) after starting therapy, which is still responsive to therapy after the start of therapy (On-going response, left side of graph). The right graph in Figure 7 is a graph showing $X^2/Y$ wherein "X" is the percentage of $CD62L^{low}CD4^+$ T cells and "Y" is the percentage of $CD25^+FOXP3^+CD4^+$ T cells for each of: patient group resistant to therapy as of the start of therapy (Primary resistance, right side of graph); patient group at an average of 63.3 weeks (28 to 92 weeks) after starting therapy, which has acquired therapeutic resistance after the start of therapy (Acquired resistance, middle of graph); and patient group at an average of 64.5 weeks (12 to 92 weeks) after starting therapy, which is still responsive to therapy after the start of therapy (On-going response, left side of graph).

[Figure 8] Figure **8** is a graph showing the percentage of $CD62L^{low}CD4^+$ T cells for long-term progression free survival group (LR), short-term responder group (SR), and non-responder group (NR) (left graph of Figure 8) and a graph showing $X^2/Y$ wherein "X" is the percentage of $CD62L^{low}CD4^+$ T cells and "Y" is the percentage of $CD25^+FOXP3^+CD4^+$ T cells (Figure **8,** right).

[Figure 9] Figure **9** is a schematic diagram describing the mechanism associated with the present invention.

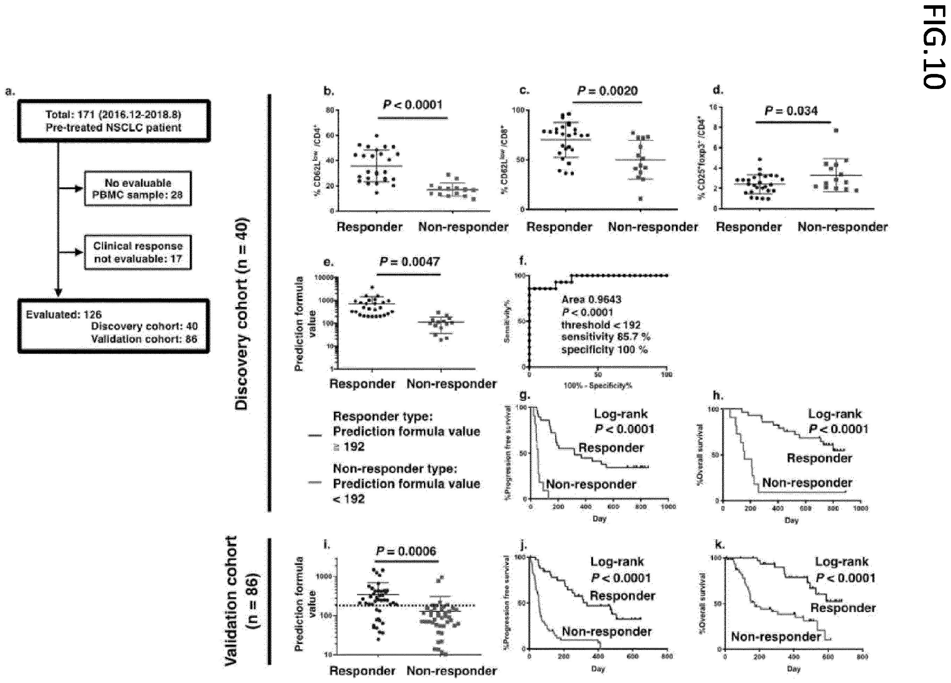

[Figure 10] Figure **10** shows the correlation between a T cell subpopulation and NSCLC patients responsive to nivolumab therapy. a: CONSORT diagram. Informed consent was obtained from 171 NSCLC patients. A peripheral blood sample was not collected before nivolumab therapy from 28 patients. Image evaluation was not performed in week 9 for 17 patients. b to d: difference between subpopulations of peripheral blood mononuclear cells (PBMC) in responders achieving PR or SD and non-responders exhibiting progression of disease by week 9 after nivolumab therapy. PBMCs were stained using FITC-conjugated anti-CD4, PE-conjugated anti-CD62L, and PE-Cy5-conjugated anti-CD8 mAb, or FITC-conjugated anti-CD4, PE-conjugated anti-FOXP3, and PE-Cy5 conjugated anti-CD25 mAb. Panels b and c: the ratios of $CD62L^{low}$ cells in entire $CD4^+$ cell population and entire $CD8^+$ cell population, respectively. Panel d: the ratio of $CD25^+FOXP3^+$ cells in entire $CD4^+$ cell population. e: value of prediction formula for patients of a discovery cohort. Prediction formula ($X^2/Y$) is based on the ratio of $CD62L^{low}$ cells (X) and ratio of $CD25^+FOXP3^+$ cells (Y) in the entire $CD4^+$ cell population. f: receiver operating characteristic curve of a formula for predicting non-responders in a discovery cohort (n = 40). Sensitivity and specificity were 85.7% and 100% at a threshold value (192) of the prediction formula (P < 0.0001). g: Progression free survival (PFS) curve for patients of a discovery cohort diagnosed as a non-responder or responder based on a threshold value (192) of the prediction formula. h: overall survival (OS) curve of a discovery cohort. i: value of a prediction formula for a patient of a validation cohort. j: PFS curve for a patient of a validation cohort. k: OS curve for a patient of a validation cohort. Data is shown as mean value $\pm$ standard error for the mean value, and the symbols indicate the value for individual patients in panels b to e and i. The statistical significance of the differences was evaluated using two-sided Student's t-test (b to e and i) and logrank test (g, h, j, and k).

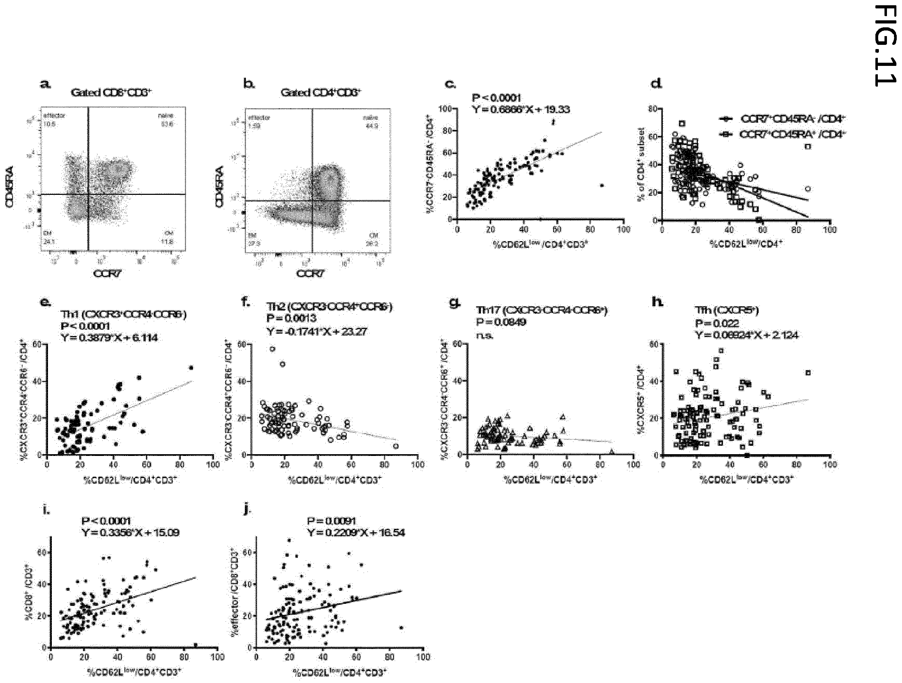

[Figure 11] Figure **11** is a diagram showing the correlation between $CD62L^{low}CD4^+$ T cells and other T cell subpopulations. a and b: CCR7 and CD45RA expression in gated $CD8^+CD3^+$ cells and $CD4^+CD3^+$ cells in PBMCs. c and d: linear correlation between the ratio of $CD62L^{low}CD4^+$ cells and the ratio of $CCR7^- CD45RA^-$ cells (c) and linear correlation between the ratio of $CD62L^{low}CD4^+$ cells and the ratio of $CCR7^+CD45RA^-$ cells or the ratio of $CCR7^+CD45RA^+$ cells (d) in the entire $CD4^+$ cell population. e to h: linear correlation between the ratio of $CD62L^{low}CD4^+$ cells and the ratio of $CXCR3^+CCR4^-CCR6^-$ cells, $CXCR3^-CCR4^+CCR6^-$ cells, $CXCR3^-CCR4^-CCR6^+$ cells, or $CXCR5^+$ cells in the entire $CD4^+$ cell population. i and j: linear correlation between the ratio of $CD62L^{low}CD4^+$ cells and the ratio of $CD8^+CD3^+$ cells and (effector) $CCR7^-CD45RA^+CD8^+$ cells.

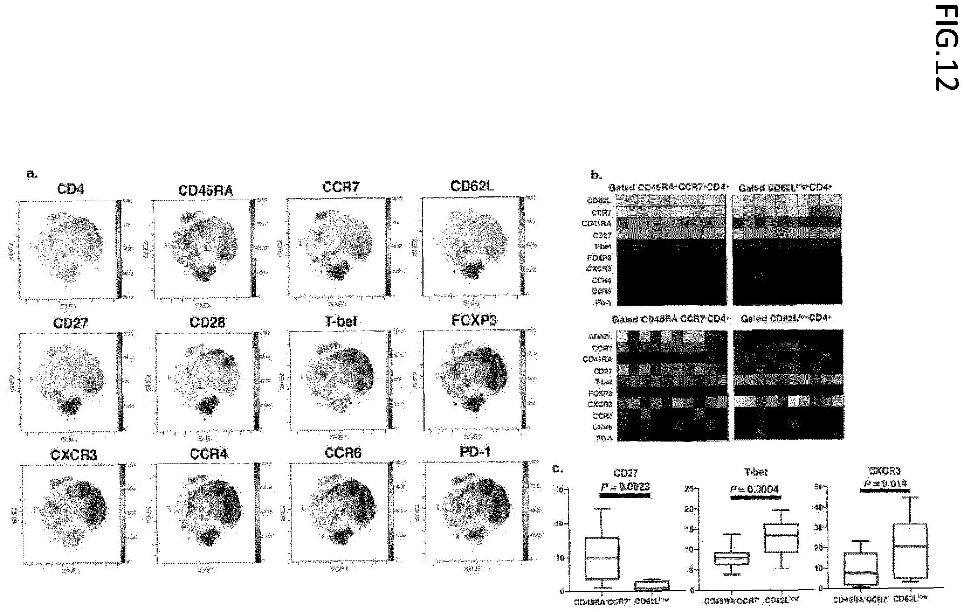

[Figure 12] Figure **12** is a diagram showing mass cytometry and gene expression analysis on $CD4^+$ T cells. a: representative example of viSNE analysis on gated $CD4^+CD3^+$ cells by unsupervised clustering based on expression of 29 types of molecules (CD3, CD4, CD8, CD19, CD27, CD28, CD45RA, CD62L, CD69, CD80, CD90, CD103, CD134, CD137, CD152, CD154, CD183, CD194, CD196, CD197, CD223, CD273, CD274, CD278, CD279, T-bet, BCL-6, FOXP3, and TIM-3). b: expression of CD62L, CCR7, CD45RA, CD27, T-bet, FOXP3, CXCR3, CCR4, CCR6, and PD-1 is shown for gated $CD45RA^+CCR7^+$, $CD45RA^-CCR7^-$, $CD62L^{high}$, and $CD62L^{low}CD4^+CD3^+$ T cells (n = 10). c: comparison of CD27, T-bet, and CXCR3 expression between gated $CD45RA^-CCR7^-$ and $CD62L^{low}CD4^+CD3^+$ T cells (n = 10) .

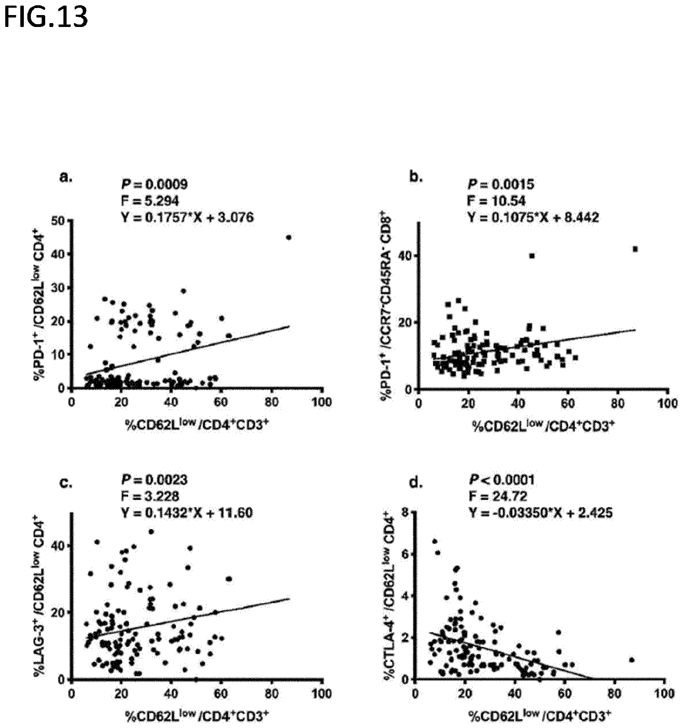

[Figure 13] Figure **13** is a diagram showing the correlation between a $CD62L^{low}CD4^+$ T cell subpopulation and PD-1, LAG3, and CTLA-4 expression, and the status of dendritic cells. a to d: linear correlation between the ratio of $CD62L^{low}CD4^+$ cells and the ratio of $PD-1^+CD62L^{low}CD4^+$ cells, $PD-1^+CCR7^- CD45RA^-CD8^+$ cells, $LAG-3^+CD62L^{low}CD4^+$ cells, or $CTLA-4^+CD62L^{low}CD4^+$ cells.

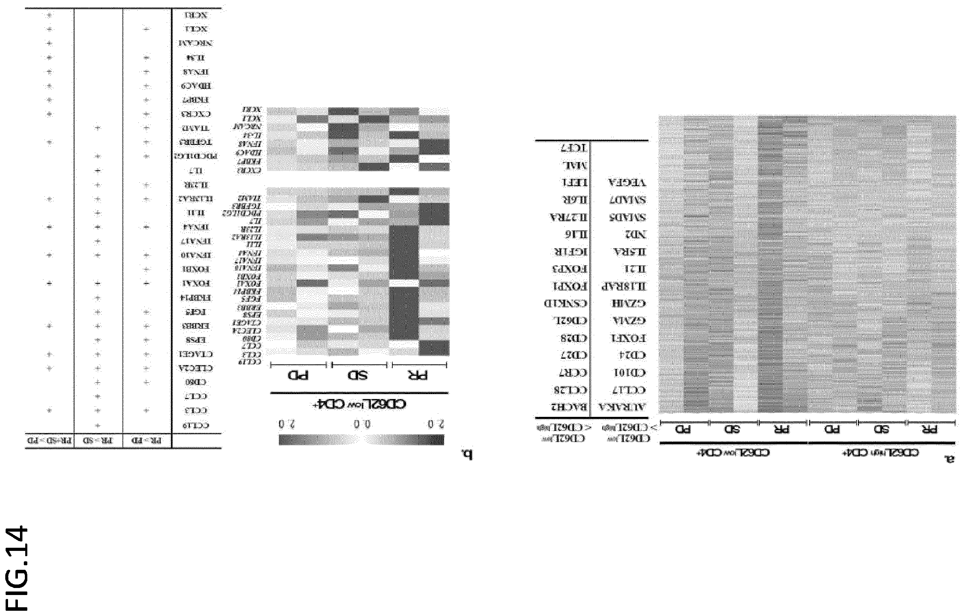

[Figure 14] Figure **14** shows gene expression corresponding to an excellent response to nivolumab therapy. Gene expression data was compared between $CD62L^{high}CD4^+$ T cells and $CD62L^{low}CD4^+$ T cells from partial response (PR), stable disease (SD), and progressive disease (PD) patients to obtain signatures. Genes that are signatures compared between PR and SD, PR and PD, SD and PD, PR + SD and PD, and PR and SD + PD derived cells (1884, 1826, 1410, 1167, and 1513 genes, respectively) were combined with all 3458 genes in a. 30 immunity related genes exhibiting different expressions between CD62L$^{low}$CD4$^+$ T cells and CD62L$^{high}$CD4$^+$ T cells are shown. Gene expression of 30 out of 53 genes that are known to be associated with antitumor immunity among the signatures described above are shown in b from the viewpoint of response to nivolumab therapy. The level of gene expression in CD62L$^{low}$CD4$^+$ T cells is shown. These genes had relatively high gene expression in PR relative to PD, in PR relative to SD, and in PR and SD relative to PD.

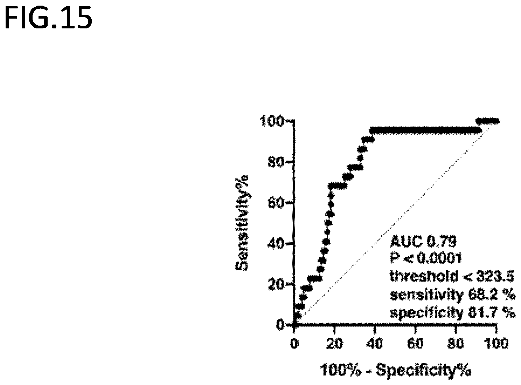

[Figure 15] Figure **15** shows a subpopulation of CD62L$^{low}$CD4$^+$ T cells in long-term survivors vs. short-term responders. A receiver operating characteristic curve of a formula for predicting long-term responders (n = 126). Sensitivity and specificity were 68.2% and 81.7% at a threshold value (323.5) of the prediction formula (P < 0.0001).

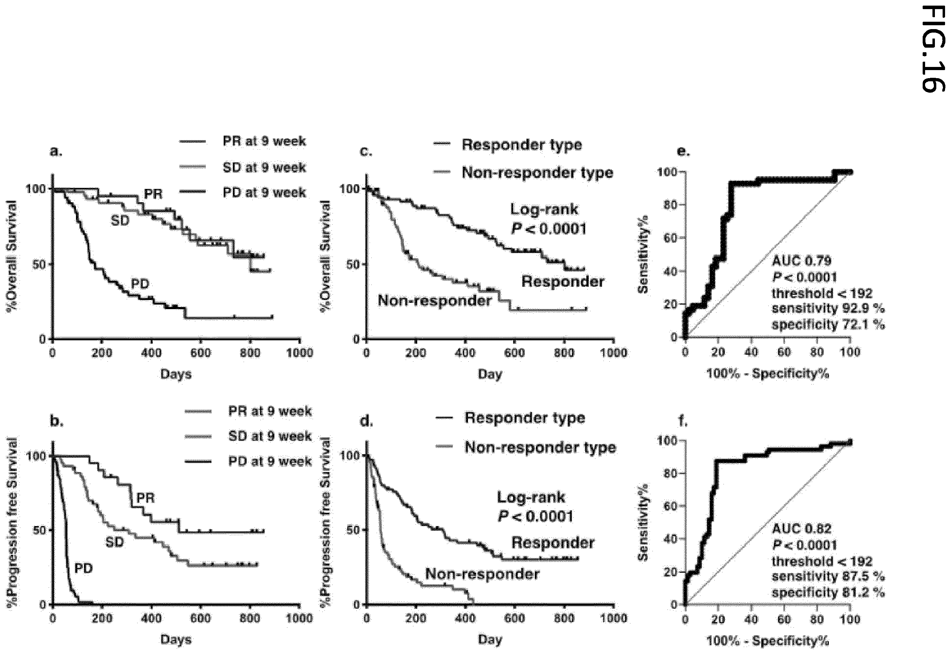

[Figure 16] Figure **16** is a diagram showing the survival period of patients after nivolumab therapy. (a) Overall survival curve and (b) progression free period curve for three patient subgroups (n = 126 in total) exhibiting progressive disease (PD), stable disease (SD), or partial response (PR) during the first tumor response evaluation at week 9 after nivolumab therapy. (c) OS curve and (d) PFS curve for patients diagnosed as a non-responder or responder (n = 143 in total) based on a threshold value (192) of the prediction formula including patients whose tumor response could not be evaluated at week 9. (e and f) receiver operating characteristic curves (ROC) for a formula for predicting non-responders in the validation cohort (n = 86) and all patients (n = 126).

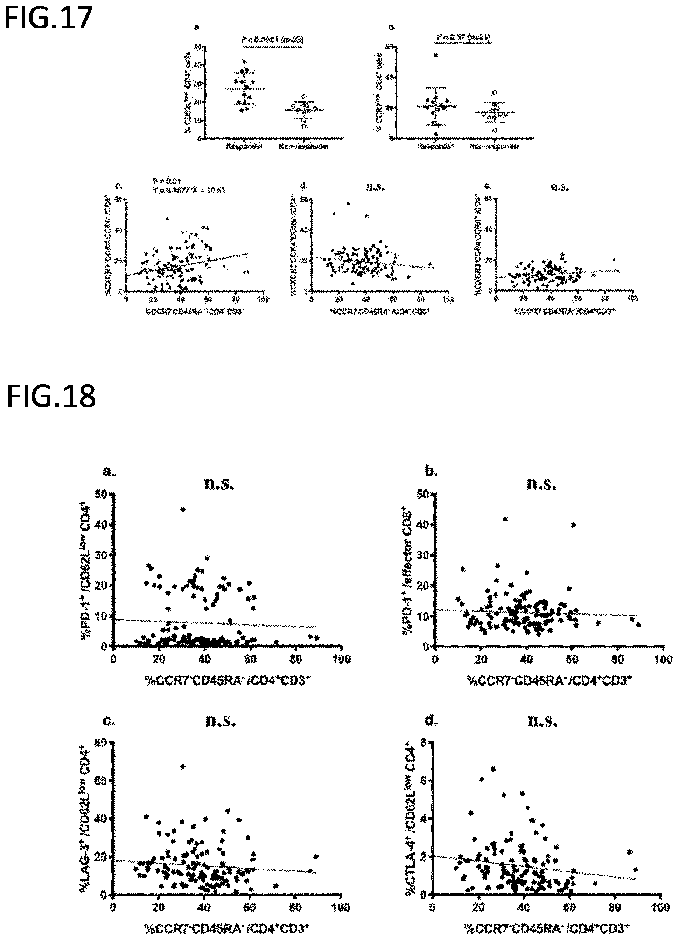

[Figure 17] Figure **17** is a diagram showing the correlation between ratios of subpopulations of immune system cells and correlation between CD62L$^{low}$ cells and CCR7$^-$CD45RA$^-$ cells in the entire CD4$^+$ T cell population and a value of the prediction formula. c to e: correlation between the ratio of CCR7$^-$CD45RA$^-$ cells in the entire CD4$^+$ cell population and the ratio of CXCR3$^+$CCR4$^-$CCR6$^-$ cells, CXCR3$^-$CCR4$^+$CCR6$^-$ cells, or CXCR3$^-$CCR4$^-$CCR6$^+$ cells in the entire CD4$^+$ cell population. a and b: ratios of CD62L$^{low}$ cells and CCR7$^-$CD45RA$^-$ cells in the entire CD4$^+$ T cell population obtained from 23 patients. Data is shown as mean value ± standard error for the mean value, and the symbols indicate the value for individual patients. The statistical significance of differences was evaluated using two-sided Student's t-test.

[Figure 18] Figure **18** is a diagram showing the correlation between the ratios of T cell subpopulations. Linear correlation of the ratio of CCR7$^-$CD45RA$^-$ cells in the entire CD4$^+$ T cell population with respect to: (a and b) the ratio of PD-1$^+$ cells in the entire CD62L$^{low}$CD4$^+$ cell population and the entire CCR7$^-$CD45RA$^-$CD8$^+$ cell population; and (c and d) the ratio of LAG-3$^+$ cells and the ratio of CTLA-4$^+$ cells in the entire CD62L$^{low}$CD4$^+$ cell population.

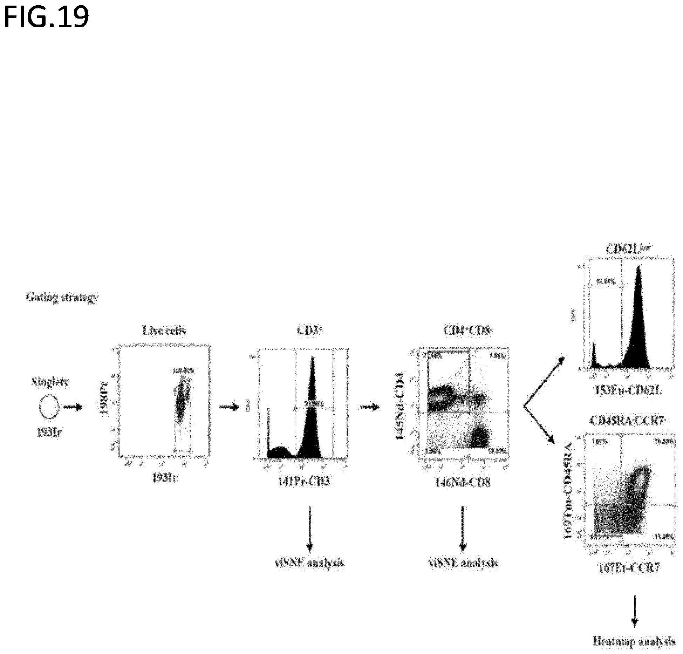

[Figure 19] Figure **19** is a diagram showing the gating strategy of mass cytometry analysis. The inventors used a normalization algorithm that recognizes the signal intensity of metal embedded polypropylene EQ™ Four Element Calibration Bead. After normalization, the beads were removed, and singlets were gated with $^{191}$Ir. Viable cells were gated with $^{191}$Ir and $^{198}$Pt.

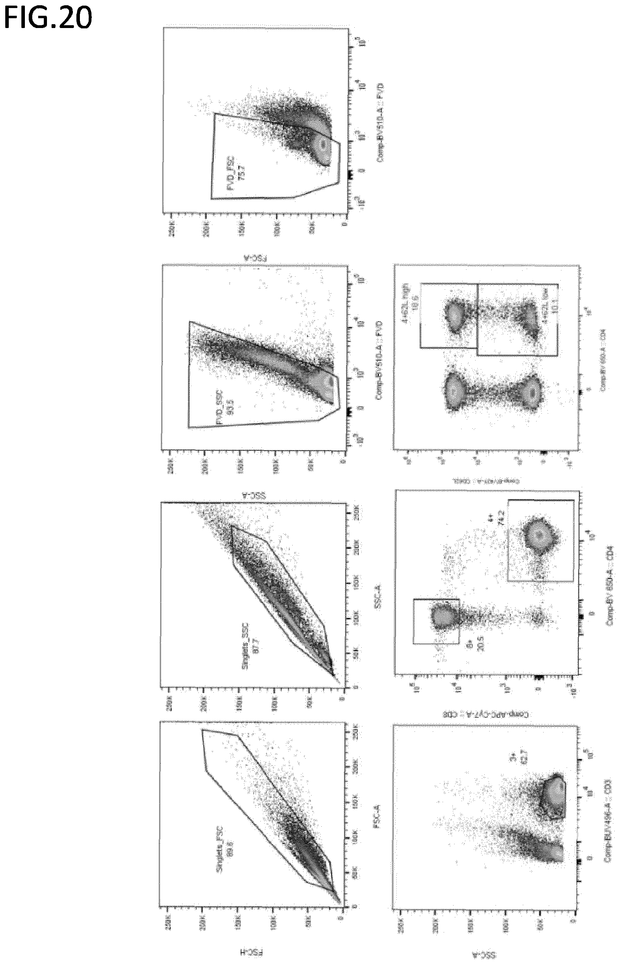

[Figure 20] Figure **20** is a diagram showing the gating strategy for LSR Fortessa analysis. Viable singlets were gated using FSC, SSC, and FVD staining. CD3$^+$ cells were gated as T cells.

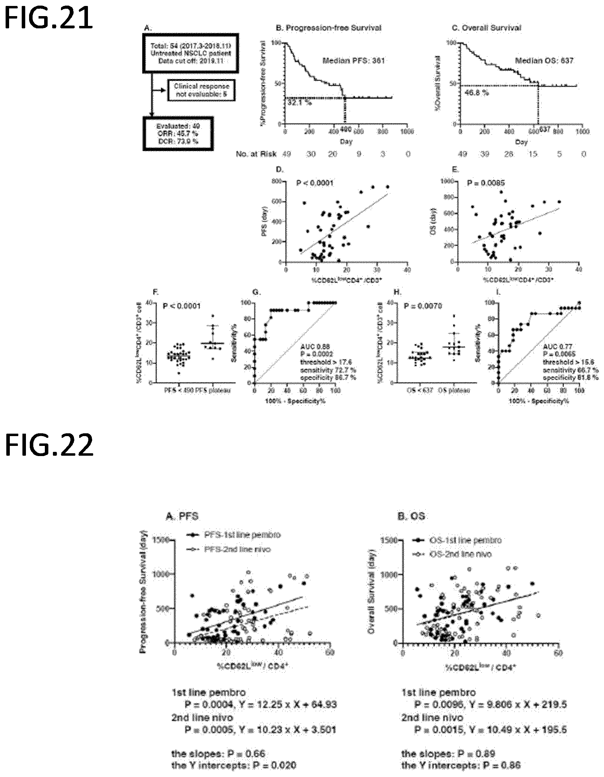

[Figure 21] Figure **21** is a diagram showing a result from using pembrolizumab as the first-line therapy. A: analyzed patient group. B: progression free survival (PFS) curve for pembrolizumab therapy. C: overall survival (OS) curve for pembrolizumab therapy. D: Results of ROC analysis with the horizontal axis showing CD62L$^{low}$CD4$^+$/CD3$^+$ and the vertical axis showing PFS. E: Results of ROC analysis with the horizontal axis showing CD62L$^{low}$CD4$^+$/CD3$^+$ and the vertical axis showing OS. F: Results of plotting CD62L$^{low}$CD4$^+$/CD3$^+$ in the PFS < 490 group and the PFS ≥ 490 group. G: Results of ROC analysis with CD62L$^{low}$CD4$^+$/CD3$^+$ > 17.6 as the threshold value for PFS. H: Results of plotting CD62L$^{low}$CD4$^+$/CD3$^+$ in the OS < 637 group and the OS ≥ 637 group. I: Results of ROC analysis with CD62L$^{low}$CD4$^+$/CD3$^+$ > 15.6 as the threshold value for OS.

[Figure 22] Figure **22A** and Figure **22B** are graphs showing results of comparing patients who have undergone first-line therapy using pembrolizumab (•) and patients who have undergone second-line therapy using nivolumab (o). The effect of nivolumab therapy on treated non-small cell lung cancer and the effect of pembrolizumab therapy on untreated PD-L1 > 50% non-small cell lung cancer would be nearly the same when adjusted with %CD62Llow/CD4+ (PFS is excellent in the PD-L1 > 50% group, but the ratio of increase in PFS for each %CD62Llow/CD4+ is the same).

[Description of Embodiments]

The present invention is described hereinafter while showing the best mode thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence.

The definitions of the terms and/or the detailed basic technology that are particularly used herein are described hereinafter as appropriate.

(Definitions)

As used herein, "long-term responder" refers to a patient with a progression free survival period of 500 days or longer such as a patient without any progression over 500 days or longer after nivolumab therapy. Since a patient who is expected to be a long-term responder is predicted to have long-term survival through cancer immunotherapy, clinicians can determine that cancer immunotherapy should be discontinued with minimum administration (e.g., one administration).

As used herein, "short-term responder" refers to a patient with a progression free survival period of less than 500 days such as a patient with progression in less than 500 days after nivolumab therapy. Since a patient who is expected to be a short-term responder is predicted to have no expectation of an effect through cancer immunotherapy, or attain somewhat of an effect but unable to attain long-term survival through cancer immunotherapy, clinicians can (1) consider concomitant use of another therapeutic method while continuing to further administer therapy, or (2) consider changing the therapy to another therapeutic method and/or concomitant use of another therapeutic method.

As used herein, "biomarker" refers to characteristics that can be objectively measured and evaluated as an indicator of a normal biological process, pathological process, or a pharmacological response to therapeutic intervention.

As used herein, "cancer" refers to malignant tumor, which is highly atypic, expands faster than normal cells, and can destructively infiltrate or metastasize surrounding tissue, or the presence thereof. In the present invention, cancer includes, but is not limited to, solid cancer and hematopoietic tumor.

As used herein, "cancer immunotherapy" refers to a method of treating cancer using a biological defense mechanism such as the immune mechanism of organisms.

As used herein, "antitumor immune response" refers to any immune response against tumor in a live organism.

As used herein, "dendritic cell stimulation in an antitumor immune response" refers to any phenomenon that stimulates dendritic cells, which occurs in the process of an immune response against tumor in a live organism. Such stimulation can be a direct or indirect factor for inducing an antitumor immune response. Although not limited to the following, dendritic cell stimulation in an antitumor immune response is typically applied by $CD4^+$ T cells (e.g., effector T cells), which results in dendritic cells stimulating $CD8^+$ T cells, and the stimulated $CD8^+$ T cells exerting an antitumor effect.

As used herein, "correlation" refers to two matters having a statistically significant correlated relationship. For example, "relative amount of B correlated with A" refers to the relative amount of B being statistically significantly affected (e.g., increase or decrease) when A occurs.

As used herein, "flow cytometry" refers to a technology of measuring the number of cells, individuals, and other biological particles suspended in a liquid and individual physical/chemical/biological attributes.

As used herein, "immune activation" refers to enhancement in the immune function for eliminating foreign objects in the body. Immune activation can be indicated by an increase in the amount of any factor (e.g., immune cell or cytokine) that has a positive effect on immune function.

As used herein, "cell subpopulation" refers to any group of cells with some type of a common feature in a cell population including cells with diverse properties. For cell subpopulations with a specific name that is known in the art, a specific cell subpopulation can be mentioned by using such a term or by describing any property (e.g., expression of a cell surface marker).

As used herein, the "amount" of a certain cell subpopulation encompasses the absolute number of certain cells and relative amount as the ratio in a cell population. For example, "amount of a $CD62L^{low}CD4^+$ T cell subpopulation" as used herein can be a relative amount with respect to the amount of $CD3^+$ cells, $CD4^+$ cells, or $CD3^+CD4^+$ cells. As used herein, "percentage of cells" refers to the amount of the cell subpopulation. For example, "percentage of $CD62L^{low}CD4^+$ T cells" refers to the amount of $CD62L^{low}CD4^+$ T cell subpopulation relative to a $CD3^+$ cell subpopulation, $CD4^+$ cell subpopulation, or $CD3^+CD4^+$ cell subpopulation.

As used herein, the term "relative amount" with regard to cells can be interchangeably used with "ratio". Typically, the terms "relative amount" and "ratio" refer to the number of cells constituting a given cell subpopulation (e.g., $CD62L^{low}CD4^+$ T cell subpopulation) with respect to the number of cells constituting a specific cell population (e.g., $CD4^+$ T cell population).

As used herein, "baseline" refers to the amount that is the subject of comparison for determining the increase or decrease in the amount of a marker described herein. When determining the increase/decrease of a certain amount after a certain treatment (e.g., cancer immunotherapy) relative to before the certain treatment, "baseline" can be, for example, said amount before treatment.

As used herein, the term "about", when used to qualify a numerical value, is used to mean that the described numerical value encompasses a range of values up to $\pm$ 10%.

As used herein, "threshold value" refers to a value that is set for a variable, which gives some type of a meaning when the variable is greater than or less than the threshold value. A threshold value is also referred to as a cut-off value herein.

As used herein, "non-responder group" refers to a group of subjects determined as progressive disease (PD) when the therapeutic effect from undergoing cancer therapy is determined in accordance with RECIST ver 1.1. A non-responder group is also referred to as a PD group, progressive group, or NR (Non-responder), which are interchangeably used herein.

As used herein, "partial responder group" refers to a group of subjects determined as partial response (PR) when the therapeutic effect from undergoing cancer therapy is determined in accordance with RECIST ver 1.1. A partial responder group is also referred to as a PR group, which is interchangeably used herein.

As used herein, "stable group" refers to a group of subjects determined as stable disease (SD) when the therapeutic effect from undergoing cancer therapy is determined in accordance with RECIST ver 1.1. A "stable group" is also referred to as an SD group, intermediate group, or IR (Intermediate Responder), which are interchangeably used herein.

As used herein, "complete responder group" refers to a group of subjects determined as complete response (CR) when the therapeutic effect from undergoing cancer therapy is determined in accordance with RECIST ver 1.1. A "complete responder group" is also referred to as a CR group, which is interchangeably used herein. If a population of subjects includes a complete responder group (CR) in addition to a partial responder group (PR) or includes a complete responder group (CR) without including a partial responder group (PR), the population is detected in the same manner as a partial responder group (PR) in the present invention.

As used herein, "responder group" is used to comprehensively refer to a "partial responder group" and "complete responder group", and is also referred to as a "good responder group" or "GR".

As used herein, "non-responder group threshold value" refers to a threshold value used to distinguish a non-responder group from a stable group + responder group in a given population of subjects. When selecting a non-responder group in a given population of subjects, a non-responder group threshold value is selected to achieve a predetermined sensitivity and specificity.

As used herein, "responder group threshold value" refers to a threshold value used to distinguish a stable group and a responder group in a given population of subjects or in a given population of subjects from which a non-responder group is excluded using a non-responder group threshold value. When selecting a responder group in a given population of subjects or in a given population of subjects from which a non-responder group is excluded using a non-responder group threshold value, a responder group threshold value is selected to achieve a predetermined sensitivity and specificity.

As used herein, "long-term survival threshold value" refers to a threshold value used to identify a subject predicted to have long-term survival in a given population of subjects or in a given population of subjects from which a non-responder group is excluded using a non-responder group threshold value. When selecting or predicting a long-term survivor in a given population of subjects or in a given population of subjects from which a non-responder group is excluded using a non-responder group threshold value, a long-term survival threshold value is selected to achieve a predetermined sensitivity and specificity.

As used herein, "therapeutic intervention" refers to any therapy administered, after administering a certain therapy or concurrently with a certain therapy, by targeting the same disease as said therapy. As a therapeutic intervention, therapy that has been administered once can be repeated, or therapy which is different from therapy that has been administered once can be administered. Examples of therapeutic intervention when cancer immunotherapy has been administered include a therapeutic method combining said cancer immunotherapy with another cancer therapy. Typically, therapeutic intervention can be co-administration of one or more additional agents. Alternatively, combination therapy can be a combination with radiation therapy. One or more additional agents can be any chemotherapeutic drug, or a second immune checkpoint inhibitor can be included. Examples of another cancer therapy used in combination therapy include, but are not limited to, other cancer immunotherapy (e.g., adoptive cell transfer), hyperthermia therapy, surgical procedure, and the like.

Preferably, therapeutic intervention is administered when a given composition of a cell subpopulation in a subject is shown to be above (or below) the non-responder group threshold value or responder group threshold value as a non-responder group or a responder group, or when a given composition of a cell subpopulation in a subject is above (or below) the baseline so that long-term survival is not predicted. To determine whether therapeutic intervention is needed, the change in a given composition of a cell subpopulation in a subject over time can be measured. Therapeutic intervention can be administered in order to increase the amount of a CD4$^+$ T cell subpopulation correlated with dendritic cell stimulation in an antitumor immune response in a sample. The amount of a CD4$^+$ T cell subpopulation is not limited, but is typically selected from the group consisting of:

an amount of a CD62L$^{low}$CD4$^+$ T cell subpopulation;
an amount of a CCR7$^-$CD4$^+$ T cell subpopulation;

an amount of a LAG-3$^+$CD62L$^{low}$CD4$^+$ T cell subpopulation;
an amount of an ICOS$^+$CD62L$^{low}$CD4$^+$ T cell subpopulation; and
an amount of a PD-1$^+$CD62L$^{low}$CD4$^+$ T cell subpopulation.

"Sensitivity" refers to the ratio of the number of subjects with a given feature among selected subjects to the total number of subjects with a given feature in a subject population when selecting a subject with a given feature in a population of subjects. If, for example, subjects with a given feature in a population of subjects are all selected, sensitivity is 100%. If half of the subjects with a given feature in a population of subjects is selected, sensitivity is 50%. If a subject with a given feature in a population of subjects is not selected at all, sensitivity is 0%. Sensitivity is determined as, for example, (number of subjects with a given feature in selected subjects)/(total number of subjects with a given feature in a subject population). When it is desirable to find subjects in a certain state (e.g., long-term survival as a result of cancer immunotherapy), determination with high sensitivity means that such subjects are likely determined to be in such a state with certainty.

"Specificity" refers to the ratio of the number of subjects with a given feature among selected subjects to the total number of selected subjects when selecting a subject with a given feature in a subject population. If, for example, candidates selected from a population of subjects all have a given feature, specificity is 100%. If half of the candidates selected from a population of subjects has a given feature, specificity is 50%. If none of the candidates selected from a population of subjects has a given feature, specificity is 0%. Specificity is determined as, for example, (number of subjects with a given feature in selected subjects)/(total number of selected subjects). Determination with high specificity means that the probability of incorrectly determining a subject who is not in a certain state (e.g., responder to cancer immunotherapy) to be in another state (e.g., long-term survival as a result of caner immunotherapy) is low.

(Marker)

T cell subpopulations that have a strong positive correlation with a CD62L$^{low}$CD4$^+$ cell subpopulation are type 1 helper CD4$^+$ T cells (Th1), effector memory CD4$^+$ T cells, CD8$^+$ T cells, and effector CD8$^+$ T cells. They are cell subpopulations that are important for the cell killing function in cell-mediated immunity. Meanwhile, type 2 helper CD4$^+$ T cells (Th2) and regulatory T cells have a negative correlation. These are known as cell subpopulations that suppress cell-mediated immunity. Accordingly, an increase in the CD62L$^{low}$CD4$^+$ cell subpopulation indicates activation of antitumor cell-mediated immunity and a decrease in a cell subpopulation that obstructs such activation. The CD62L$^{low}$CD4$^+$ cell subpopulation controls the antitumor immune function by having a significant correlation with LAG3, ICOS, PD-1, or CTLA-4 expression on CD4$^+$ T cells or CD8$^+$ T cells. Specifically, an increase in the CD62L$^{low}$CD4$^+$ cell subpopulation is correlated with an increase in PD-1, LAG-3, or ICOS expression and a decrease in CTLA-4 expression. This indicates that antitumor immunity is primarily regulated by PD-1 or LAG-3, and is thus understood to be associated with the efficacy of immune checkpoint inhibition therapy thereof. Furthermore, the HLA-DR$^+$CD141$^+$CD11c$^+$ dendritic cell subpopulation and CD62L$^{low}$CD4$^+$ cell subpopulation have a positive correlation. This is understood such that expression of an MHC class II restricted antigen by an activated dendritic cell results in an increase in the CD62L$^{low}$CD4$^+$ cell subpopulation which recognizes MHC class II restricted antigens. It is understood that the cell subpopulation is a CD4$^+$ T cell subpopulation correlated with dendritic cell stimulation in tumor immune response. It is understood that the HLA-DR$^+$CD141$^+$CD11c$^+$ dendritic cell subpopulation is a dendritic cell subpopulation correlated with dendritic cell stimulation in an antitumor immune response. When a cell subpopulation is expressed herein, CD62L$^{low}$/CD4$^+$ cells, for example, means the ratio of CD62L$^{low}$CD4$^+$ T cells to CD4$^+$ T cells, wherein the cells described in the numerator comprise all of the features of the cells described in the denominator. As used herein, CD62L$^{low}$CD4$^+$/CD3$^+$ cells, for example, can be CD62L$^{low}$/CD4$^+$CD3$^+$ cells. Both cells indicate the ratio of CD62L$^{low}$CD4$^+$CD3$^+$ cells. The ratio can also be expressed as CD62L$^{low}$/CD4$^+$ with the parent population as CD4$^+$.

The results described above suggest that long-term survival in cancer immunotherapy can be predicted by using a CD4$^+$ T cell subpopulation correlated with dendritic cell stimulation in an antitumor immune response and/or a dendritic cell subpopulation correlated with dendritic cell stimulation in an antitumor immune response.

An embodiment of the invention provides a method of using a composition of a cell subpopulation in a subject who has undergone cancer immunotherapy as an indicator for predicting long-term survival in cancer immunotherapy. The method can comprise analyzing a composition of a cell subpopulation in a sample. The composition of a cell subpopulation can be analyzed by any method described herein or any method that is known to those skilled in the art. The method can be an in vitro or in silico method. One embodiment of the invention indicates the presence/absence of immune activation in a subject by comparing an amount of a cell subpopulation with a suitable baseline. In particular, a cell subpopulation that correlates with dendritic cell stimulation in an antitumor immune response can be used as the cell subpopulation.

(1. CD4$^+$ T cell subpopulation correlated with dendritic cell stimulation in an antitumor immune response)

In one embodiment, the indicator cell subpopulation is a CD4$^+$ T cell subpopulation correlated with dendritic cell stimulation in an antitumor immune response. CD62L$^{low}$CD4$^+$ T cells play a role in the stimulation of dendritic cells in antitumor immunity. It is understood that a CD4$^+$ T cell subpopulation correlated with dendritic cell stimulation in an antitumor immune response can also be used as an indicator for predicting long-term survival in caner immunotherapy.

Examples of a CD4$^+$ T cell subpopulation correlated with dendritic cell stimulation in an antitumor immune response include, but are not limited to, a CD4$^+$ T cell subpopulation with decreased expression of a homing molecule to a secondary lymphoid organ, CD4$^+$ T cell subpopulation primed by an effector T cell, CD4$^+$ T cell subpopulation primed by antigen recognition, and regulatory T cell subpopulation.

Examples of a CD4$^+$ T cell subpopulation correlated with dendritic cell stimulation include, but are not limited to, a CD62L$^{low}$CD4$^+$ T cell subpopulation, CCR7$^-$CD4$^+$ T cell subpopulation, LAG-3$^+$CD62L$^{low}$CD4$^+$ T cell subpopulation, ICOS$^+$CD62L$^{low}$CD4$^+$ T cell subpopulation, CCR4$^+$CD25$^+$CD4$^+$ T cell subpopulation, CD45RA$^-$CD4$^+$ T cell subpopulation, CD45RO$^+$CD4$^+$ T cell subpopulation, CD62L$^{high}$CD25$^+$CD4$^+$ T cell subpopulation, CD127$^+$CD25$^+$CD4$^+$ T cell subpopulation, CD45RA$^-$Foxp3$^+$CD4$^+$ T cell subpopulation, Foxp3$^+$CD25$^+$CD4$^+$ T cell subpopulation, and the like.

A CD4$^+$ T cell subpopulation correlated with dendritic cell stimulation in an antitumor immune response can be, for example, a cell subpopulation within a CD62L$^{low}$CD4$^+$ T cell population. Examples of a CD4$^+$ T cell subpopulation correlated with dendritic cell stimulation in an antitumor immune response include, but are not limited to, a CD62L$^{low}$CD4$^+$ T cell subpopulation (i.e., CD62L$^{low}$CD4$^+$ T cell population itself), ICOS$^+$CD62L$^{low}$CD4$^+$ T cell subpopulation, PD-1$^+$CD62L$^{low}$CD4$^+$ T cell subpopulation, LAG-3$^+$CD62L$^{low}$CD4$^+$ T cell subpopulation, and the like.

For the cell subpopulations described above, the amount of expression of a suitable surface marker molecule in a suitable cell can be used as an indicator instead of, or in addition to, the amount of the cell subpopulation. For example, the amount of expression of ICOS, PD-1, LAG-3, or the like expressed in a CD62L$^{low}$CD4$^+$ T cell can be used as an indicator.

(2. Amount of dendritic cell subpopulation correlated with dendritic cell stimulation in an antitumor immune response)

In one embodiment, an indicator cell subpopulation is a dendritic cell subpopulation correlated with dendritic cell stimulation in an antitumor immune response. For example, in an embodiment herein, an increase in an HLA-DR$^+$CD141$^+$CD11c$^+$ cell subpopulation after cancer immunotherapy relative to before cancer immunotherapy is observed. HLA-DR mediates stimulation of dendritic cells by a CD4$^+$ T cell. It is understood that a dendritic cell subpopulation correlated with dendritic cell stimulation in an antitumor immune response can also be used as an indicator for predicting long-term survival in caner immunotherapy.

Examples of a dendritic cell subpopulation correlated with dendritic cell stimulation in an antitumor immune response include, but are not limited to, a dendritic cell subpopulation that increases due to an increase in a cell subpopulation with decreased expression of a homing molecule in a CD4$^+$ T cell population, dendritic cell subpopulation that increases due to an increase in a CD4$^+$ T cell subpopulation primed by an effector T cell in a CD4$^+$ T cell population, and dendritic cell subpopulation that increases due to an increase in a CD4$^+$ T cell subpopulation primed by antigen recognition in a CD4$^+$ T cell population. Examples of dendritic cell subpopulations include, but are not limited to, HLA-DR$^+$ dendritic cell subpopulations, CD80$^+$ dendritic cell subpopulations, CD86$^+$ dendritic cell subpopulations, and PD-L1$^+$ dendritic cell subpopulations. Examples of dendritic cells include, but are not limited to, myeloid dendritic cells (mDC, CD141$^+$CD11c$^+$ dendritic cells) and plasmacytoid dendritic cells (pDC, CD123$^+$CD11c$^+$ dendritic cells).

Examples of a dendritic cell subpopulation correlated with dendritic cell stimulation in an antitumor immune response include an HLA-DR$^+$CD141$^+$CD11c$^+$ cell subpopulation. For the cell subpopulation described above, an amount of expression of a suitable surface marker molecule in a suitable cell can be used as an indicator instead of, or in addition to, the amount of the cell subpopulation. For example, the amount of expression of HLA-DR or the like expressed in a CD141$^+$CD11c$^+$ can be used as an indicator.

(3. Amount of CD8$^+$ T cell subpopulation correlated with dendritic cell stimulation in an antitumor immune response)

In one embodiment, an indicator cell subpopulation is a CD8$^+$ T cell subpopulation correlated with dendritic cell stimulation in an antitumor immune response. Dendritic cells which have been stimulated by CD4$^+$ T cells stimulate CD8$^+$ T cells, and stimulated CD8$^+$ T cells ultimately exert antitumor activity. CD137 on a CD8$^+$ T cell mediates stimulation of a CD8$^+$ T cell by a dendritic cell. It is understood that a CD8$^+$ T cell subpopulation correlated with dendritic cell stimulation in an antitumor immune response can also be used as an indicator for predicting long-term survival in caner immunotherapy.

Examples of a CD8$^+$ T cell subpopulation correlated with dendritic cell stimulation in an antitumor immune response include, but are not limited to, a CD8$^+$ T cell subpopulation that increases due to an increase in a cell subpopulation with decreased expression of a homing molecule in a CD4+ T cell population, CD8+ T cell subpopulation that increases due to an increase in a CD4+ T cell subpopulation primed by an effector T cell in a CD4+ T cell population, CD8+ T cell subpopulation that increases due to an increase in a CD4+ T cell subpopulation primed by antigen recognition in a CD4+ T cell population, CD8+ T cell subpopulation that increases due to an increase in an HLA-DR+ dendritic cell subpopulation in a dendritic cell population, CD8+ T cell subpopulation that increases due to an increase in a CD80+ dendritic cell subpopulation in a dendritic cell population, and CD8+ T cell subpopulation that increases due to an increase in a PD-L1+ dendritic cell subpopulation in a dendritic cell population. Furthermore, examples of CD8+ T cell subpopulations correlated with dendritic cell stimulation in an antitumor immune response include, but are not limited to, CD62L$^{low}$CD8+ T cell subpopulation, CD137+CD8+ T cell subpopulation, and CD28+CD62L$^{low}$CD8+ T cell subpopulation.

For the cell subpopulation described above, an amount of expression of a suitable surface marker molecule in a suitable cell can be used as an indicator instead of, or in addition to, the amount of the cell subpopulation. For example, the amount of expression of CD137 or the like expressed in a CD62L$^{low}$CD8+ T cell can be used as an indicator.

The amount of cell subpopulation described herein can be used as an indicator by combining a plurality of amounts. Combining indicators can improve the accuracy of prediction of long-term progression free survival. One embodiment can indicate the presence/absence of immune activation in a subject by comparing at least two amounts selected from the group consisting of an amount of a CD4+ T cell subpopulation correlated with dendritic cell stimulation in an antitumor immune response, an amount of a dendritic cell subpopulation correlated with dendritic cell stimulation in an antitumor immune response, and an amount of a CD8+ T cell subpopulation correlated with dendritic cell stimulation in an antitumor immune response in a sample with a baseline.

One embodiment of the invention is a method of using an amount selected from:

an amount of a CD4+ T cell subpopulation correlated with dendritic cell stimulation in an antitumor immune response;
an amount of a dendritic cell subpopulation correlated with dendritic cell stimulation by a CD4+ T cell in an antitumor immune response;
an amount of a CD8+ T cell subpopulation correlated with dendritic cell stimulation in an antitumor immune response;
an amount of regulatory T cell subpopulation or an amount of a CD4+ T cell subpopulation correlated with regulatory T cells; or
an amount of an ICOS+CD62L$^{low}$CD4+ T cell subpopulation; in a subject as a variable (indicator) of a formula for predicting long-term progression free survival. In one embodiment, variables (X, Y) in the invention are each selected from the group consisting of:

an amount of a CD62L$^{low}$CD4+ T cell subpopulation;
an amount of a CCR7-CD4+ T cell subpopulation;
an amount of a LAG-3+CD62L$^{low}$CD4+ T cell subpopulation;
an amount of an ICOS+CD62L$^{low}$CD4+ T cell subpopulation;
an amount of a PD-1+CD62L$^{low}$CD4+ T cell subpopulation;
an amount of a CD45RA-CD4+ T cell subpopulation;
an amount of a CD45RO+CD4+ T cell subpopulation;
an amount of a CCR4+CD25+CD4+ T cell subpopulation;
an amount of a CD62L$^{high}$CD25+CD4+ T cell subpopulation;
an amount of a CD127+CD25+CD4+ T cell subpopulation;
an amount of a CD45RA-Foxp3+CD4+ T cell subpopulation;
an amount of a Foxp3+CD25+CD4+ T cell subpopulation;
an amount of a HLA-DR+ dendritic cell subpopulation;
an amount of a CD80+ dendritic cell subpopulation;
an amount of a CD86+ dendritic cell subpopulation;
an amount of a PD-L1+ dendritic cell subpopulation;
an amount of a CD62L$^{low}$CD8+ T cell subpopulation;
an amount of a CD137+CD8+ T cell subpopulation; and
an amount of a CD28+CD62L$^{low}$CD8+ T cell subpopulation

For example, the method of the invention can use a value selected from the group consisting of:

an amount of a CD4+ T cell subpopulation correlated with dendritic cell stimulation in an antitumor immune response;
an amount of a dendritic cell subpopulation correlated with dendritic cell stimulation by a CD4+ T cell in an antitumor immune response; and
an amount of a CD8+ T cell subpopulation correlated with dendritic cell stimulation in an antitumor immune response;
as (X). The method of the invention can also use a value selected from the group consisting of:

an amount of a CD62L$^{low}$CD4$^+$ T cell subpopulation;
an amount of a CCR7$^-$CD4$^+$ T cell subpopulation;
an amount of a LAG-3$^+$CD62L$^{low}$CD4$^+$ T cell subpopulation;
an amount of an ICOS$^+$CD62L$^{low}$CD4$^+$ T cell subpopulation;
an amount of a PD-1$^+$CD62L$^{low}$CD4$^+$ T cell subpopulation;
an amount of a CD45RA$^-$CD4$^+$ T cell subpopulation;
an amount of a CD45RO$^+$CD4$^+$ T cell subpopulation;
an amount of a HLA-DR$^+$ dendritic cell subpopulation;
an amount of a CD80$^+$ dendritic cell subpopulation;
an amount of a CD86$^+$ dendritic cell subpopulation;
an amount of a PD-L1$^+$ dendritic cell subpopulation;
an amount of a CD62L$^{low}$CD8$^+$ T cell subpopulation;
an amount of a CD137$^+$CD8$^+$ T cell subpopulation; and
an amount of a CD28$^+$CD62L$^{low}$CD8$^+$ T cell subpopulation; as (X) to calculate variables (X, Y).

For example, the method of the invention can use an amount of a regulatory T cell subpopulation or an amount of a CD4$^+$ T cell subpopulation correlated with regulatory T cells as (Y) to calculate variables (X, Y). The method of the invention can also use a value selected from the group consisting of:

an amount of a CCR4$^+$CD25$^+$CD4$^+$ T cell subpopulation;
an amount of a CD62L$^{high}$CD25$^+$CD4$^+$ T cell subpopulation;
an amount of a CD127$^+$CD25$^+$CD4$^+$ T cell subpopulation;
an amount of a CD45RA$^-$Foxp3$^+$CD4$^+$ T cell subpopulation; and
an amount of a Foxp3$^+$CD25$^+$CD4$^+$ T cell subpopulation; as (Y) to calculate variables (X, Y).

The method of the invention can use, for example, comparison of a relative value of X to Y with a threshold value, comprising a step of measuring amount (X) of CD4$^+$CD62L$^{low}$ T cells and a step of measuring amount (Y) of Foxp3$^+$CD25$^+$CD4$^+$ T cells, as an indicator for predicting long-term progression free survival. As (Y), an amount of a regulatory T cell subpopulation or an amount or ratio of a CD4$^+$ T cell subpopulation correlated with regulatory T cells can be used. In particular, a study of the ratio of CD62L$^{low}$CD4$^+$ T cells/regulatory T cells as a biomarker has not been reported up to this point, but the inventor found that this is very useful as a biomarker for predicting long-term progression free survival with respect to cancer immunotherapy.

The present invention can also use comparison of a relative value of X to Y with a threshold value, comprising a step of measuring an amount of a CD80$^+$ dendritic cell subpopulation (X) and a step of measuring an amount of a CD28$^+$CD62L$^{low}$CD8$^+$ T cell subpopulation (Y), as an indicator for predicting long-term progression free survival.

Since the inventor has found that a plurality of indicators are independently correlated with long-term progression free survival, a plurality of indicators can be combined and used as an indicator of long-term progression free survival. When two or more indicators are combined as an indicator of long-term progression free survival, an indicator expressed by a formula using any number of variables can be used. When using a plurality of indicators ($X_1$, $X_2$, $X_3$ ... $X_n$), examples of indicators of long-term progression free survival include, but are not limited to the following:

$$F = a_1 X_1^{b1} + a_2 X_2^{b2} + a_3 X_3^{b3} \ldots + a_n X_n^{bn}$$

$$F = X_1^{c1} \star X_2^{c2} \star X_3^{c3} \ldots \star X_n^{cn}$$

wherein each of a, b, and c is any real number. Long-term progression free survival can be predicted from the result of comparing a value (indicator) calculated from such a formula with a threshold value. Each coefficient can be determined from multivariate analysis (e.g., estimation by logistic regression) using discriminant analysis for the novel indicators found by the inventor to predict long-term progression free survival as a result of cancer immunotherapy on a subject.

Typically, long-term progression free survival can be predicted by formula F(X, Y) using two indicators (X, Y) described herein as variables. In a specific embodiment, the formula is a relative value of X to Y.

As a relative value of X to Y, any function of X and Y (F(X, Y)) can be used. In particular, when it is understood that X is positively correlated with long-term progression free survival and Y is negatively correlated with long-term progression free survival, any function of X and Y (F(X, Y)), which monotonically increases with respect to X and monotonically decreases with respect to Y, can be used, but the function is not limited thereto. When two or more variables representing long-term progression free survival are given, a formula indicating long-term progression free survival can be found through regression by calculating the contribution of each variable to long-term progression free survival.

Examples of formula F(X, Y) indicating long-term progression free survival include, but are not limited to the following.

$$F = aX^r + bY^s$$

$$F = X^r * Y^s$$

wherein a, b, r, s are any real number.

As r and s, an integer can be used to simplify a formula. In some embodiments, examples of relative values of X to Y include, but are not limited to, $X^n/Y^m$ (wherein n and m are any real number such as any integer) such as X/Y and $X^2/Y$. If each factor of X and Y indicates long-term progression free survival to therapy from different mechanisms, combining such indicators can improve the accuracy of prediction for long-term progression free survival. Testing by the inventor demonstrated that long-term progression free survival can be predicted as a result of cancer immunotherapy on a subject by using a formula with r and s in the range of -5 to 5.

A threshold value can be determined while taking sensitivity and specificity into consideration. Sensitivity and specificity can be sensitivity and specificity for the detection of long-term progression free survival. In one embodiment, it is preferable to set a threshold value resulting in both sensitivity and specificity of 100% for the biomarker of the invention. When two or more indicators described as a biomarker of the invention are used, a threshold value can be determined for each of the indicators. If necessary, threshold values can be distinguished for use as a first threshold value, second threshold value, third threshold value, fourth threshold value, or the like.

A threshold value can be determined so that the sensitivity would be greater than about 90% for the detection of long-term progression free survival. In another embodiment, a threshold value can be determined so that the sensitivity would be about 100% for the detection of long-term progression free survival. In still another embodiment, a threshold value can be determined so that the specificity would be greater than about 90% for the detection of long-term progression free survival. In still another embodiment, a threshold value can be determined so that the specificity would be about 100% for the detection of long-term progression free survival.

A value determined by performing an analysis known in the art in a reference subject group can be used as a threshold value. Examples of such analysis include, but are not limited to, machine learning and regression analysis. A threshold value can be obtained by, for example, ROC analysis using a discriminant created by regression analysis. An excellent threshold value for one or both parameters can be set while taking sensitivity and specificity in ROC analysis into consideration.

In one embodiment, the composition of T cells of a subject is a composition of T cells in a sample obtained from the subject. Preferably, the sample is a peripheral blood sample. Since a biomarker provided in the present invention can be measured using a peripheral blood sample, such a biomarker has a significant advantage in clinical application in that the biomarker can be used noninvasively at a low cost over time.

In one embodiment, cancer immunotherapy comprises administration of an immune checkpoint inhibitor. The biomarker of the invention can, in particular, accurately predict long-term progression free survival of a subject against such cancer immunotherapy.

In a preferred embodiment of the invention, X (percentage of CD62L$^{low}$CD4$^+$ cells) and Y (percentage of CD25$^+$FOXP3$^+$CD4$^+$ T cells) can be used. In a preferred embodiment of the invention, $X^2/Y$ can be utilized as a function using X and Y. For example in an especially preferred embodiment of the invention, $X^2/Y$ can be calculated using X (percentage of CD62L$^{low}$CD4$^+$ cells) and Y (percentage of CD25$^+$FOXP3$^+$CD4$^+$ T cells) for each patient of a patient population and a specific numerical value can be set as a threshold value by a known method. For example, a non-responder group threshold value is "α" and long-term survival threshold value is "β". Next, a sample of a subject is measured. The value of $X^2/Y$ of the subject can be compared with the size of the values of α and β to determine that:

*$X^2/Y < \alpha$: no effect can be expected from cancer immunotherapy. Change in therapy to another therapeutic method or concomitant use with another therapeutic method should be considered.

*$\alpha \leq X^2/Y < \beta$: certain effect is attained by cancer immunotherapy, but therapy should be continued and concomitant use with another therapeutic method should be considered to attain long-term survival.

*$\beta < X^2/Y$: since long-term survival is predicted from cancer immunotherapy, cancer immunotherapy should be discontinued at the minimum (e.g., at one administration).

The numerical values "α" and "β" can be determined while envisioning adjustment of sensitivity and specificity.

Although not particularly limited, about 100, about 120, about 140, about 160, about 180, about 200, about 220, or about 240 can be used as preferred α. More preferred α is about 170, about 180, about 190, or about 200. α can also be about 192.

Although not particularly limited, β can be a numerical value that is, for example, at least 50, preferably at least 70, and more preferably at least 90 greater than α. Preferred β is a numerical value that is at least 50 greater than α. About 150, about 170, about 190, about 210, about 230, about 250, about 270, about 290, about 300, about 320, about 340, about 360, about 380, about 400, about 420, or about 440 can be used. More preferred β is about 310, about 320, about 330, or about 340. β can also be about 324.

Although not particularly limited, a value within the range from about 100 to 400, preferably a value within the range from about 100 to 200, such as a value within the range from about 100 to 110, from about 110 to 120, from about 120 to 130, from about 130 to 140, from about 140 to 150, from about 150 to 160, from about 160 to 170, from about 170 to 180, from about 180 to 190, from about 190 to 200, from about 200 to 210, from about 210 to 220, from about 220 to 230, or from about 230 to 240 can be set as α.

Although not particularly limited, a value which is at least 50 greater than α and is within the range from about 150 to 450, preferably a value within the range from about 300 to 440 such as from about 300 to 310, from about 310 to 320, from about 320 to 330, from about 330 to 340, from about 340 to 350, from about 350 to 360, from about 360 to 370, from about 370 to 380, from about 380 to 390, from about 390 to 400, from about 400 to 410, from about 410 to 420, from about 420 to 430, or from about 430 to 440 can be set as β.

The preferred embodiment of the invention can indicate that therapeutic intervention should be administered when a subject is indicated as a part of a non-responder group, such as when a discriminant is less than a non-responder group threshold value. If a subject is a part of a non-responder group, therapy that is not cancer immunotherapy (e.g., chemotherapy, radiation therapy, surgical procedure, hyperthermia therapy, or the like) can be administered, or additional cancer immunotherapy (e.g., immune checkpoint inhibitor, adoptive cell transfer, or the like) can be administered instead of, or in addition to, cancer immunotherapy being administered as therapeutic intervention. As the therapy that is combined, any therapy described herein can be administered.

A preferred embodiment of the invention considers that therapeutic intervention should be administered when it is indicated that a subject is not a long-term responder or when long-term survival is not attained such as when a discriminant is less than a long-term survival threshold value. In such a case, it can be distinguished whether a discriminant is less than the long-term survival threshold value, and whether the discriminant is less than the non-responder group threshold value or greater than or equal to the non-responder group threshold value. If a subject is indicated as not attaining long-term survival, but is not a part of a non-responder group (short-term responder), therapy that is not cancer immunotherapy (e.g., chemotherapy, radiation therapy, surgical procedure, hyperthermia therapy, or the like) can be administered, or additional cancer immunotherapy (e.g., immune checkpoint inhibitor, adoptive cell transfer, or the like) can be administered in addition to cancer immunotherapy being administered as therapeutic intervention. Typically, concomitant use of another chemotherapeutic drug or a second immune checkpoint inhibitor with an immune checkpoint inhibitor that is already being administered can be considered. Any therapy described herein can be administered as the therapy being combined.

In one embodiment, an immune checkpoint inhibitor comprises a PD-1 inhibitor or a PD-L1 inhibitor. Examples of PD-1 inhibitors include, but are not limited to, anti-PD-1 antibodies that inhibit interaction (e.g., binding) of PD-1 and PD-L1 such as nivolumab, pembrolizumab, spartalizumab, and cemiplimab. Examples of PD-L1 inhibitors include, but are not limited to, anti-PD-Ll antibodies that inhibit interaction (e.g., binding) of PD-1 and PD-L1 such as durvalumab, atezolizumab, and avelumab.

Another aspect of the invention provides a method of predicting long-term progression free survival against cancer immunotherapy of a subject using a composition of T cells of the subject to treat a subject with cancer. Alternatively, a method of treating cancer in a subject with a specific T cell composition or a composition therefor is provided. Cancer immunotherapy, especially immune checkpoint inhibition therapy is known to result in a large difference in responsiveness for each subject. Administration of cancer immunotherapy by selecting a subject with a biomarker of the invention can significantly improve the probability of achieving a therapeutic effect such as tumor regression.

One embodiment of the invention provides a method of treating a subject with cancer, comprising:

(1) a step of determining a relative amount selected from the group consisting of:

a relative amount of a CD4$^+$ T cell subpopulation correlated with dendritic cell stimulation in an antitumor immune response;
a relative amount of a dendritic cell subpopulation correlated with dendritic cell stimulation by a CD4$^+$ T cell in an antitumor immune response; and
a relative amount of a CD8$^+$ T cell subpopulation correlated with dendritic cell stimulation in an antitumor immune response;

in CD4$^+$ T cells in a sample derived from the subject and

(2) a step of determining that the subject is a part of a long-term progression free survival group against cancer immunotherapy when the relative amount is higher than a threshold value, and a step of administering the cancer immunotherapy to the subject when the subject is determined to be a part of a long-term progression free survival group.

An embodiment of the invention provides a method of using a composition of a cell subpopulation in a sample obtained from a subject as an indicator for predicting long-term survival in cancer immunotherapy in the subject. The method comprises a step of analyzing the composition of the cell subpopulation in the sample obtained from the subject. Long-term survival in cancer immunotherapy in the subject is predicted by comparing an amount of a CD4$^+$ T cell subpopulation correlated with dendritic cell stimulation in an antitumor immune response in the sample with a baseline. In this regard, the amount (or relative amount) of a CD4$^+$ T cell subpopulation is selected from the group consisting of:

a ratio of a CD62L$^{low}$ T cell subpopulation in CD4$^+$ T cells;
a ratio of a CCR7$^-$ T cell subpopulation in CD4$^+$ T cells;
a ratio of a CD45RA$^-$ T cell subpopulation in CD4$^+$ T cells;
a ratio of a CD45RO$^+$ T cell subpopulation in CD4$^+$ T cells;
a ratio of a LAG-3$^+$ T cell subpopulation in CD62L$^{low}$CD4$^+$ T cells;
a ratio of an ICOS$^+$ cell subpopulation in CD62L$^{low}$CD4$^+$ T cells;
a ratio of a PD-1$^+$ cell subpopulation in CD62L$^{low}$CD4$^+$ T cells;
a ratio of a CCR4$^+$CD25$^+$ cell subpopulation in CD4$^+$ T cells;
a ratio of a CD62L$^{high}$CD25$^+$ cell subpopulation in CD4$^+$ T cells;
a ratio of a CD127$^+$CD25$^+$ cell subpopulation in CD4$^+$ T cells;
a ratio of a CD45RA$^-$Foxp3$^+$ cell subpopulation in CD4$^+$ T cells;
a ratio of a Foxp3$^+$CD25$^+$ cell subpopulation in CD4$^+$ T cells;
a ratio of a HLA-DR$^+$ subpopulation in dendritic cells;
a ratio of a CD80$^+$ subpopulation in dendritic cells;
a ratio of a CD86$^+$ subpopulation in dendritic cells;
a ratio of a PD-L1$^+$ subpopulation in dendritic cells;
a ratio of a CD62L$^{low}$ cell subpopulation in CD8$^+$ T cells;
a ratio of a CD137$^+$ cell subpopulation in CD8$^+$ T cells; and
a ratio of a CD28$^+$ cell subpopulation in CD62L$^{low}$CD8$^+$ T cells.

Preferably, the amount (or relative amount) is selected from the group consisting of:

a ratio of a CD62L$^{low}$ cell subpopulation in CD4$^+$ T cells;
a ratio of a CCR7$^-$ cell subpopulation in CD4$^+$ T cells;
a ratio of a CD45RA$^-$ cell subpopulation in CD4$^+$ T cells;
a ratio of a CD45RO$^+$ cell subpopulation in CD4$^+$ T cells;
a ratio of a LAG-3$^+$ cell subpopulation in CD62L$^{low}$CD4$^+$ T cells;
a ratio of an ICOS$^+$ cell subpopulation in CD62L$^{low}$CD4$^+$ T cells;
a ratio of a HLA-DR$^+$ subpopulation in dendritic cells;
a ratio of a CD80$^+$ subpopulation in dendritic cells;
a ratio of a CD86$^+$ subpopulation in dendritic cells;
a ratio of a PD-L1$^+$ subpopulation in dendritic cells;
a ratio of a CD62L$^{low}$ cell subpopulation in CD8$^+$ T cells;
a ratio of a CD137$^+$ cell subpopulation in CD8$^+$ T cells; and
a ratio of a CD28$^+$ cell subpopulation in CD62L$^{low}$CD8$^+$ T cells.

Another embodiment of the invention provides a method of treating a subject with cancer, comprising: a step of determining a ratio of Foxp3$^+$CD25$^+$ T cells in CD4$^+$ T cells in a sample derived from the subject; a step of determining that the subject is a part of a long-term progression free survival group against cancer immunotherapy when the ratio of Foxp3$^+$CD25$^+$ T cells in CD4$^+$ T cells is lower than a threshold value; and a step of administering the cancer immunotherapy to the subject when the subject is determined to be a part of a long-term progression free survival group against cancer immunotherapy. Another embodiment of the invention provides a method of treating a subject with cancer, comprising: a step of determining a ratio of Foxp3$^+$CD25$^+$ T cells in CD4$^+$ T cells in a sample derived from the subject; and a step of administering cancer immunotherapy to the subject determined to be a part of a long-term progression free survival group by a step of determining that the subject is a part of a long-term progression free survival group against the cancer immunotherapy when the ratio of Foxp3$^+$CD25$^+$ T cells in CD4$^+$ T cells is lower than a threshold value.

Another embodiment of the invention provides a method of treating a subject with cancer, comprising:

(1) a step of determining amounts (X, Y) selected from the group consisting of:

a relative amount of a CD4$^+$ T cell subpopulation correlated with dendritic cell stimulation in an antitumor immune response;
a relative amount of a dendritic cell subpopulation correlated with dendritic cell stimulation by a CD4$^+$ T cell in an antitumor immune response;
a relative amount of a CD8$^+$ T cell subpopulation correlated with dendritic cell stimulation in an antitumor immune response;
an amount of regulatory T cells or a CD4$^+$ T cell subpopulation correlated with regulatory T cells; and
an amount of an ICOS$^+$CD62L$^{low}$CD4$^+$ T cell subpopulation;

(2) a step of determining that the subject is a part of a long-term progression free survival group against cancer immunotherapy by using a comparison of a relative amount of X to Y with a threshold value; and
(3) a step of administering the cancer immunotherapy to the subject when the subject is determined to be a part of a long-term progression free survival group against cancer immunotherapy.

Another embodiment of the invention provides a method of treating a subject with cancer, comprising:

a step of administering cancer immunotherapy to the subject determined to be a part of a long-term progression free survival group against the cancer immunotherapy by (1) a step of determining amounts (X, Y) selected from the group consisting of:
a relative amount of a CD4$^+$ T cell subpopulation correlated with dendritic cell stimulation in an antitumor immune response;
a relative amount of a dendritic cell subpopulation correlated with dendritic cell stimulation by a CD4$^+$ T cell in an antitumor immune response;
a relative amount of a CD8$^+$ T cell subpopulation correlated with dendritic cell stimulation in an antitumor immune response;
an amount of regulatory T cells or a CD4$^+$ T cell subpopulation correlated with regulatory T cells; and
an amount of an ICOS$^+$CD62L$^{low}$CD4$^+$ T cell subpopulation; and
(2) determining that the subject is a part of a long-term progression free survival group against cancer immunotherapy by using a comparison of a relative amount of X to Y with a threshold value (non-responder group threshold value).

For example, the amounts (X) and (Y) are selected from the group consisting of:

an amount of a CD62L$^{low}$CD4$^+$ T cell subpopulation;
an amount of a CCR7$^-$CD4$^+$ T cell subpopulation;
an amount of a LAG-3$^+$CD62L$^{low}$CD4$^+$ T cell subpopulation;
an amount of a PD-1$^+$CD62L$^{low}$CD4$^+$ T cell subpopulation;
an amount of a CD45RA$^-$CD4$^+$ T cell subpopulation;
an amount of a CD45RO$^+$CD4$^+$ T cell subpopulation;
an amount of a HLA-DR$^+$ dendritic cell subpopulation;
an amount of a CD80$^+$ dendritic cell subpopulation;
an amount of a CD86$^+$ dendritic cell subpopulation;
an amount of a PD-L1$^+$ dendritic cell subpopulation;
an amount of a CD137$^+$CD8$^+$ T cell subpopulation;
an amount of a CD62L$^{low}$CD8$^+$ T cell subpopulation;
an amount of a CD28$^+$CD62L$^{low}$CD8$^+$ T cell subpopulation;
an amount of a Foxp3$^+$CD25$^+$CD4$^+$ T cell subpopulation;
an amount of a CD62L$^{high}$CD25$^+$CD4$^+$ T cell subpopulation;
an amount of a CD45RA$^-$Foxp3$^+$CD4$^+$ T cell subpopulation;
an amount of a CCR4$^+$CD25$^+$CD4$^+$ T cell subpopulation; and
an amount of a CD127$^+$CD25$^+$CD4$^+$ T cell subpopulation.

For example, the method of the invention can use a value selected from the group consisting of:

an amount of a CD4$^+$ T cell subpopulation correlated with dendritic cell stimulation in an antitumor immune response;
an amount of a dendritic cell subpopulation correlated with dendritic cell stimulation by a CD4$^+$ T cell in an antitumor immune response; and
an amount of a CD8$^+$ T cell subpopulation correlated with dendritic cell stimulation in an antitumor immune response;
as (X). The method of the invention can also use a value selected from the group consisting of:
an amount of a CD62L$^{low}$CD4$^+$ T cell subpopulation;
an amount of a CCR7$^-$CD4$^+$ T cell subpopulation;
an amount of a LAG-3$^+$CD62L$^{low}$CD4$^+$ T cell subpopulation;
an amount of a ICOS$^+$CD62L$^{low}$CD4$^+$ T cell subpopulation;
an amount of a PD-1$^+$CD62L$^{low}$CD4$^+$ T cell subpopulation;
an amount of a CD45RA$^-$CD4$^+$ T cell subpopulation;
an amount of a CD45RO$^+$CD4$^+$ T cell subpopulation;
an amount of a HLA-DR$^+$ dendritic cell subpopulation;
an amount of a CD80$^+$ dendritic cell subpopulation;
an amount of a CD86$^+$ dendritic cell subpopulation;
an amount of a PD-L1$^+$ dendritic cell subpopulation;
an amount of a CD62L$^{low}$CD8$^+$ T cell subpopulation;
an amount of a CD137$^+$CD8$^+$ T cell subpopulation; and
an amount of a CD28$^+$CD62L$^{low}$CD8$^+$ T cell subpopulation; as (X) to calculate variables (X, Y).

For example, the method of the invention can use an amount of regulatory T cells or a CD4$^+$ T cell subpopulation correlated with regulatory T cells as (Y) to calculate variables (X, Y). The method of the invention can also use a value selected from the group consisting of:

an amount of a CCR4$^+$CD25$^+$CD4$^+$ T cell subpopulation;
an amount of a CD62L$^{high}$CD25$^+$CD4$^+$ T cell subpopulation;
an amount of a CD127$^+$CD25$^+$CD4$^+$ T cell subpopulation;
an amount of a CD45RA$^-$Foxp3$^+$CD4$^+$ T cell subpopulation; and
an amount of a CD4$^+$Foxp3$^+$CD25$^+$ T cell subpopulation; as (Y) to calculate variables (X, Y).

Another aspect of the invention provides a kit for predicting long-term progression free survival against cancer immunotherapy of a subject comprising a detecting agent for one or more cell surface markers selected from CD4, CD25, CD62L, Foxp3, and the like, such as a combination of markers selected from the group consisting of:

*a combination of CD4 and CD62L;
*a combination of CD4, CD45RA, and CCR7;
*a combination of CD4, CD45RO, and CCR7;
*a combination of CD4, CD62L, and LAG-3;
*a combination of CD4, CD62L, and ICOS;
*a combination of CD4, CD62L, and PD-1;
*a combination of CD4, CD62L, and CD25;
*a combination of CD4, CD127, and CD25;
*a combination of CD4, CD45RA, and Foxp3;
*a combination of CD4, CD45RO, and Foxp3;
*a combination of CD4, CD25, and Foxp3;
*a combination of CD11c, CD141, and HLA-DR;
*a combination of CD11c, CD141, and CD80;
*a combination of CD11c, CD123, and HLA-DR;
*a combination of CD11c, CD123, and CD80;
*a combination of CD8 and CD62L;
*a combination of CD8 and CD137; and
*a combination of CD28, CD62L, and CD8.

Preferably, a kit comprises detecting agents for each of CD4 and CD62L. Such a combination of detecting agents can be used to determine a T cell composition of a subject. Such a kit can be used to measure a ratio of a specific T cell subpopulation as a novel biomarker described herein in a subject.
One embodiment of the invention is a kit comprising a detecting agent for a cell surface marker for predicting a response to cancer immunotherapy of a subject. The inventor discovered that these cell surface markers expressed by T cells of a subject are related to long-term progression free survival against cancer immunotherapy of the subject. It is understood therefrom that a kit comprising a detecting agent for these cell surface markers is useful for predicting long-term progression free survival against cancer immunotherapy. A kit preferably comprises a detecting agent for CD4 and CD62L. A kit more preferably comprises a detecting agent for CD4, CD25, CD62L and Foxp3. In one embodiment, a detecting agent is an antibody. Preferably, an antibody facilitates detection of a suitably labeled marker.

Another aspect of the invention is a composition comprising an immune checkpoint inhibitor for treating cancer in a subject predicted to be a part of a long-term progression free survival group. The present invention can also provide a product comprising a package insert and an immune checkpoint inhibitor. A package insert can describe an instruction for using an immune checkpoint inhibitor in accordance with one or more steps of the method described in the present specification.

One embodiment of the invention is a composition comprising an immune checkpoint inhibitor for treating cancer in a subject predicted to be a part of a long-term progression free survival group, wherein a relative amount selected from the group consisting of:

a relative amount of a CD4$^+$ T cell subpopulation correlated with dendritic cell stimulation in an antitumor immune response;
a relative amount of a dendritic cell subpopulation correlated with dendritic cell stimulation by a CD4$^+$ T cell in an antitumor immune response; and
a relative amount of a CD8$^+$ T cell subpopulation correlated with dendritic cell stimulation in an antitumor immune response;
is greater than or equal to a threshold value in the subject.

For example, this relative amount is typically selected from the group consisting of:

a ratio of a CD62L$^{low}$ cell subpopulation in CD4$^+$ T cells;
a ratio of a CCR7$^-$ cell subpopulation in CD4$^+$ T cells;
a ratio of a LAG-3$^+$ cell subpopulation in CD62L$^{low}$CD4$^+$ T cells;
a ratio of an ICOS$^+$ cell subpopulation in CD62L$^{low}$CD4$^+$ T cells;
a ratio of a PD-1$^+$ cell subpopulation in CD62L$^{low}$CD4$^+$ T cells;
a ratio of a CD62L$^{high}$CD25$^+$ cell subpopulation in CD4$^+$ T cells;
a ratio of a CD127$^+$CD25$^+$ cell subpopulation in CD4$^+$ T cells;
a ratio of a CD45RA$^-$Foxp3$^+$ cell subpopulation in CD4$^+$ T cells;
a ratio of a Foxp3$^+$CD25$^+$ cell subpopulation in CD4$^+$ T cells;
a ratio of a HLA-DR$^+$ subpopulation in dendritic cells;
a ratio of a CD80$^+$ subpopulation in dendritic cells;
a ratio of a CD86$^+$ subpopulation in dendritic cells;
a ratio of a PD-L1$^+$ subpopulation in dendritic cells;
a ratio of a CD62L$^{low}$ subpopulation in CD8$^+$ T cells;
a ratio of a CD137$^+$ subpopulation in CD8$^+$ T cells; and
a ratio of a CD28$^+$ cell subpopulation in CD62L$^{low}$CD8$^+$ T cells.

A still another embodiment of the invention is a composition comprising an immune checkpoint inhibitor for treating cancer in a subject predicted to be a part of a long-term progression free survival, wherein the subject is a subject selected by comparing amounts (X, Y) selected from the group consisting of:

an amount of a CD4$^+$ T cell subpopulation correlated with dendritic cell stimulation in an antitumor immune response;
an amount of a dendritic cell subpopulation correlated with dendritic cell stimulation by a CD4$^+$ T cell in an antitumor immune response;
an amount of a CD8$^+$ T cell subpopulation correlated with dendritic cell stimulation in an antitumor immune response;
an amount of regulatory T cells or a CD4$^+$ T cell subpopulation correlated with regulatory T cells; and
an amount of an ICOS$^+$CD62L$^{low}$CD4$^+$ T cell subpopulation; in a sample derived from the subject,
a relative amount of X to Y, and a threshold value. The amounts (X, Y) are typically selected from the group consisting of:

an amount of a CD62L$^{low}$CD4$^+$ T cell subpopulation;
an amount of a CCR7$^-$CD4$^+$ T cell subpopulation;
an amount of a LAG-3$^+$CD62L$^{low}$CD4$^+$ T cell subpopulation;
an amount of an ICOS$^+$CD62L$^{low}$CD4$^+$ T cell subpopulation;

an amount of a PD-1$^+$CD62L$^{low}$CD4$^+$ T cell subpopulation;
an amount of a CCR4$^+$CD25$^+$CD4$^+$ T cell subpopulation;
an amount of a CD45RA$^-$CD4$^+$ T cell subpopulation;
an amount of a CD45RO$^+$CD4$^+$ T cell subpopulation;
an amount of a CD62L$^{high}$CD25$^+$CD4$^+$ T cell subpopulation;
an amount of a CD127$^+$CD25$^+$CD4$^+$ T cell subpopulation;
an amount of a CD45RA$^-$Foxp3$^+$CD4$^+$ T cell subpopulation;
an amount of a Foxp3$^+$CD25$^+$CD4$^+$ T cell subpopulation;
an amount of a HLA-DR$^+$ dendritic cell subpopulation;
an amount of a CD80$^+$ dendritic cell subpopulation;
an amount of a CD86$^+$ dendritic cell subpopulation;
an amount of a PD-L1$^+$ dendritic cell subpopulation;
an amount of a CD62L$^{low}$CD8$^+$ T cell subpopulation;
an amount of a CD137$^+$CD8$^+$ T cell subpopulation; and
an amount of a CD28$^+$CD62L$^{low}$CD8$^+$ T cell subpopulation.

For example, the method of the invention can use a value selected from the group consisting of:

an amount of a CD4$^+$ T cell subpopulation correlated with dendritic cell stimulation in an antitumor immune response;
an amount of a dendritic cell subpopulation correlated with dendritic cell stimulation by a CD4$^+$ T cell in an antitumor immune response; and
an amount of a CD8$^+$ T cell subpopulation correlated with dendritic cell stimulation in an antitumor immune response;
as (X). The method of the invention can also use a value selected from the group consisting of:

an amount of a CD62L$^{low}$CD4$^+$ T cell subpopulation;
an amount of a CCR7$^-$CD4$^+$ T cell subpopulation;
an amount of a LAG-3$^+$CD62L$^{low}$CD4$^+$ T cell subpopulation;
an amount of an ICOS$^+$CD62L$^{low}$CD4$^+$ T cell subpopulation;
an amount of a PD-1$^+$CD62L$^{low}$CD4$^+$ T cell subpopulation;
an amount of a CD45RA$^-$CD4$^+$ T cell subpopulation;
an amount of a CD45RO$^+$CD4$^+$ T cell subpopulation;
an amount of a HLA-DR$^+$ dendritic cell subpopulation;
an amount of a CD80$^+$ dendritic cell subpopulation;
an amount of a CD86$^+$ dendritic cell subpopulation;
an amount of a PD-L1$^+$ dendritic cell subpopulation;
an amount of a CD62L$^{low}$CD8$^+$ T cell subpopulation;
an amount of a CD137$^+$CD8$^+$ T cell subpopulation; and
an amount of a CD28$^+$CD62L$^{low}$CD8$^+$ T cell subpopulation as (X) to calculate variables (X, Y).

For example, the method of the invention can use an amount of a regulatory T cell subpopulation or an amount of a CD4$^+$ T cell subpopulation correlated with regulatory T cells as (Y) to calculate variables (X, Y). The method of the invention can also use a value selected from the group consisting of:

an amount of a CCR4$^+$CD25$^+$CD4$^+$ T cell subpopulation;
an amount of a CD62L$^{high}$CD25$^+$CD4$^+$ T cell subpopulation;
an amount of a CD127$^+$CD25$^+$CD4$^+$ T cell subpopulation;
an amount of a CD45RA$^-$Foxp3$^+$CD4$^+$ T cell subpopulation; and
an amount of a CD4$^+$Foxp3$^+$CD25$^+$ T cell subpopulation; as (Y) to calculate variables (X, Y).

The method of the invention can, for example, use a comparison of a relative value of X to Y with a threshold value, comprising a step of measuring an amount (X) of CD4$^+$CD62L$^{low}$ T cells and a step of measuring an amount (Y) of CD4$^+$Foxp3$^+$CD25$^+$ T cells, as an indicator for predicting that the subject is a part of a long-term progression free survival group against cancer immunotherapy. As (Y), an amount or ratio of regulatory T cells or a CD4$^+$ T cell subpopulation correlated with regulatory T cells can be used.

Still another embodiment of the invention is a composition comprising an immune checkpoint inhibitor for treating cancer in a subject predicted to be a part of a long-term progression free survival group, wherein the subject is a subject selected by comparing a relative amount of amounts (X, Y) selected from:

an amount of a CD4$^+$ T cell subpopulation correlated with dendritic cell stimulation in an antitumor immune response;

an amount of a dendritic cell subpopulation correlated with dendritic cell stimulation by a CD4$^+$ T cell in an antitumor immune response;

an amount of a CD8$^+$ T cell subpopulation correlated with dendritic cell stimulation in an antitumor immune response;

an amount of regulatory T cells or a CD4$^+$ T cell subpopulation correlated with regulatory T cells; and

an amount of an ICOS$^+$CD62L$^{low}$CD4$^+$ T cell subpopulation; in a sample derived from the subject with a threshold value, and a ratio of a Foxp3$^+$CD25$^+$ T cell subpopulation in CD4$^+$ T cells or a ratio of an ICOS$^+$CD62L$^{low}$CD4$^+$ T cell subpopulation in CD62L$^{low}$CD4$^+$ T cells in the sample derived from the subject is greater than or equal to a threshold value. Amounts (X) and (Y) are typically selected from the group consisting of:

an amount of a CD62L$^{low}$CD4$^+$ T cell subpopulation;
an amount of a CCR7$^-$CD4$^+$ T cell subpopulation;
an amount of a LAG-3$^+$CD62L$^{low}$CD4$^+$ T cell subpopulation;
an amount of an ICOS$^+$CD62L$^{low}$CD4$^+$ T cell subpopulation;
an amount of a PD-1$^+$CD62L$^{low}$CD4$^+$ T cell subpopulation;
an amount of a CCR4$^+$CD25$^+$CD4$^+$ T cell subpopulation;
an amount of a CD45RA$^-$CD4$^+$ T cell subpopulation;
an amount of a CD45RO$^+$CD4$^+$ T cell subpopulation;
an amount of a CD62L$^{high}$CD25$^+$CD4$^+$ T cell subpopulation;
an amount of a CD127$^+$CD25$^+$CD4$^+$ T cell subpopulation;
an amount of a CD45RA$^-$Foxp3$^+$CD4$^+$ T cell subpopulation;
an amount of a Foxp3$^+$CD25$^+$CD4$^+$ T cell subpopulation;
an amount of a HLA-DR$^+$ dendritic cell subpopulation;
an amount of a CD80$^+$ dendritic cell subpopulation;
an amount of a CD86$^+$ dendritic cell subpopulation;
an amount of a PD-L1$^+$ dendritic cell subpopulation;
an amount of a CD62L$^{low}$CD8$^+$ T cell subpopulation;
an amount of a CD137$^+$CD8$^+$ T cell subpopulation; and
an amount of a CD28$^+$CD62L$^{low}$CD8$^+$ T cell subpopulation.

For example, the composition of the invention can be targeted for administration to a subject characterized by variables (X, Y) by using a value selected from the group consisting of:

an amount of a CD4$^+$ T cell subpopulation correlated with dendritic cell stimulation in an antitumor immune response;

an amount of a dendritic cell subpopulation correlated with dendritic cell stimulation by a CD4$^+$ T cell in an antitumor immune response; and

an amount of a CD8$^+$ T cell subpopulation correlated with dendritic cell stimulation in an antitumor immune response;

as (X). The method of the invention can also target administration to a subject characterized by variables (X, Y) by using a value selected from the group consisting of:

an amount of a CD62L$^{low}$CD4$^+$ T cell subpopulation;
an amount of a CCR7$^-$CD4$^+$ T cell subpopulation;
an amount of a LAG-3$^+$CD62L$^{low}$CD4$^+$ T cell subpopulation;
an amount of an ICOS$^+$CD62L$^{low}$CD4$^+$ T cell subpopulation;
an amount of a PD-1$^+$CD62L$^{low}$CD4$^+$ T cell subpopulation;
an amount of a CD45RA$^-$CD4$^+$ T cell subpopulation;
an amount of a CD45RO$^+$CD4$^+$ T cell subpopulation;
an amount of a HLA-DR$^+$ dendritic cell subpopulation;
an amount of a CD80$^+$ dendritic cell subpopulation;
an amount of a CD86$^+$ dendritic cell subpopulation;
an amount of a PD-L1$^+$ dendritic cell subpopulation;
an amount of a CD62L$^{low}$CD8$^+$ T cell subpopulation;
an amount of a CD137$^+$CD8$^+$ T cell subpopulation; and
an amount of a CD28$^+$CD62L$^{low}$CD8$^+$ T cell subpopulation; as (X) to calculate variables (X, Y).

For example, variable (X, Y) can be calculated using an amount of a regulatory T cell subpopulation or an amount of a CD4$^+$ T cell subpopulation correlated with regulatory T cells as (Y) for the composition of the invention. The method of the invention can also target administration to a subject characterized by variables (X, Y) by using a value selected from the group consisting of:

> an amount of a CCR4$^+$CD25$^+$CD4$^+$ T cell subpopulation;
> an amount of a CD62L$^{high}$CD25$^+$CD4$^+$ T cell subpopulation;
> an amount of a CD127$^+$CD25$^+$CD4$^+$ T cell subpopulation;
> an amount of a CD45RA$^-$Foxp3$^+$CD4$^+$ T cell subpopulation; and
> an amount of a CD4$^+$Foxp3$^+$CD25$^+$ T cell subpopulation; as (Y) to calculate variables (X, Y).

The composition of the invention can be targeted for administration to a subject predicted to be a part of a long-term progression free survival group against cancer immunotherapy by comparing a relative value of X to Y with a threshold value from an amount (X) of CD4$^+$CD62L$^{low}$ T cells and an amount (Y) of CD4$^+$Foxp3$^+$CD25$^+$ T cells. As (Y), an amount or ratio of regulatory T cells or a CD4$^+$ T cell subpopulation correlated with regulatory T cells can be used.

In one embodiment, a composition comprises a PD-1 inhibitor. A PD-1 inhibitor is, for example, an anti-PD-1 antibody that inhibits binding of PD-1 and PD-L1, which can be, for example, nivolumab, pembrolizumab, spartalizumab, or cemiplimab. In another embodiment, a composition comprises a PD-L1 inhibitor. A PD-L1 inhibitor is, for example, an anti-PD-Ll antibody that inhibits binding of PD-1 and PD-L1, which can be, for example, durvalumab, atezolizumab, or avelumab. It is understood that a composition comprising these immune checkpoint inhibitors achieves a therapeutic effect at an especially high probability when administered to a subject selected with the biomarker of the invention. The composition of the invention can be concomitantly used with any other agent.

(Biomarkers of the invention)

It is understood that the biomarker of the invention is for evaluating the balance of the entire antitumor immune responses including CD4$^+$ T cells, dendritic cells, and/or CD8$^+$ T cells and for evaluating tumor immunity as a whole. For this reason, the method of the invention can be deemed as a method that is effective against a broad range of carcinomas. Since the present invention is for evaluating the balance of the entire antitumor immune responses, the invention is predicted to be effective for not only immune checkpoint inhibitors against PD-1/PD-L1, but also anticancer therapy acting against other immune checkpoints.

In the present invention, a marker that would be an indicator of effector T cells such as CCR7$^-$ can be used instead of or in addition to CD62L$^{low}$. Alternatively, CD45RA-and/or CD45RO+ can be used. For example, a ratio of a CD45RA$^-$ CD4$^+$ T cell subpopulation in CD4$^+$ T cells and/or a ratio of a CD45RO$^+$CD4$^+$ T cell subpopulation in CD4$^+$ T cells can also be used. It was elucidated that expression of LAG3, ICOS, or PD-1 can also be used in the same manner as (or can be used in addition to or in place of) CD62L$^{low}$. Likewise, it was elucidated that expression of CCR4 can be used in the same manner as (or can be used in addition to or in place of) CD62L$^{low}$.

Instead of (or in addition to) using CD4$^+$ T cells (CD62L$^{low}$CD4$^+$ T cells) used in the Examples as an indicator, the number/ratio of cells expressing HLA-DR and/or CD80 and/or CD86 in a myeloid dendritic cell (mDC) and/or plasmacytoid dendritic cell (pDC) population can be used as an indicator. It is understood that PD-L1 on dendritic cells can also be used as the marker of the invention.

Instead of (or in addition to) using CD4$^+$ T cells (CD62L$^{low}$CD4$^+$ T cells) used in the Examples as an indicator, the number/ratio of cells expressing CD137 in CD8$^+$ T cells can also be used as an indicator.

(Mechanism of the invention)

Although not wishing to be bound by any theory, Figure 9 schematically shows the antitumor immune response phenomenon local to tumor advocated by the inventors. Figure 9 describes cells that can be observed in peripheral blood, i.e., CD62L$^{low}$CD4$^+$ T cells, myeloid dendritic cells (mDC), plasmacytoid dendritic cells (pDC), and CD62L$^{low}$CD8$^+$ T cells as well as marker molecules expressed in these cells, i.e., LAG-3, ICOS, PD-1, HLA-DR, CD80, and CD137. PD-L1 is expressed in dendritic cells, and PD-1 is expressed in CD62L$^{low}$CD4$^+$ T cells and CD62L$^{low}$CD8$^+$ T cells.

It is understood that T cell composition is important in antitumor immune responses. For example, stimulation of dendritic cells by a CD62L$^{low}$CD4$^+$ T cell is important. If CD62L$^{low}$CD4$^+$ T cells are not sufficient (e.g., the balance between effector T cells and naive T cells is tilted toward naive T cells), dendritic cells cannot be adequately stimulated even with administration of an immune checkpoint inhibitor. As a result, antitumor immune responses cannot be sufficiently induced. For this reason, the ratio of CD62L$^{low}$CD4$^+$ T cells in CD4$^+$ T cells would be an indicator for predicting an antitumor effect by an immune checkpoint inhibitor. The ratio of CD45RA-negative CCR7-negative T cells in CD4$^+$ T cells indicates the balance between effector T cells and naive T cells in the same manner as CD62L. Thus, such a ratio can be used as an indicator in the present invention.

Since dendritic cell stimulation by CD4$^+$ T cells is mediated by HLA-DR, dendritic cells cannot be adequately stimulated if the ratio of HLA-DR positive cells in dendritic cells decreases, even after administering an immune checkpoint inhibitor. As a result, antitumor immune responses cannot be sufficiently induced. For this reason, the ratio of HLA-DR positive cells in dendritic cells would also be an indicator for predicting an antitumor effect by an immune checkpoint inhibitor.

Dendritic cells stimulated by CD4+ T cells stimulate CD8+ T cells, and stimulated CD8+ T cells ultimately exert antitumor activity. Since stimulation of CD8+ T cells by dendritic cells is mediated by CD80/CD86 expressed on dendritic cells and CD137 on CD8+ T cells, both the ratio of CD80 positive cells in dendritic cells and the ratio of CD137 positive cells in CD8+ T cells would be indicators for predicting an antitumor effect (long-term progression free survival) by an immune checkpoint inhibitor.

In addition to the biomarkers found from the mechanism described above, LAG-3, ICOS, PD-1, and CCR4 in CD4+ T cells would also be indicators for predicting an antitumor effect (long-term progression free survival) by an immune checkpoint inhibitor.

The present invention can compare an amount of a cell subpopulation with a suitable baseline and predict long-term survival in cancer immunotherapy in a subject by the comparison. An increase in the amount of a cell subpopulation in a sample relative to the baseline can indicate that long-term survival in cancer immunotherapy in the subject is predicted. Alternatively, no increase in the amount of a cell subpopulation in a sample relative to the baseline can indicate that long-term survival in cancer immunotherapy in the subject is not predicted.

Examples of the baseline include, but are not limited to, a corresponding amount of a cell subpopulation in a sample of a subject before cancer immunotherapy. As the baseline, a value experimentally calculated from a sample of a subject who has not undergone cancer immunotherapy can also be used.

An increase relative to a baseline can be indicated by an amount of cell subpopulation after cancer immunotherapy, which is an amount exceeding the baseline, an amount that is 1, 2, 3, 4, 5, 10, 15, 20, or 30% beyond the baseline, or an amount that is more than 1.5-fold, 2-fold, 3-fold, or 5-fold of the baseline. Typically, the amount is considered to be increased relative to the baseline if the amount exceeds the baseline value. When the baseline is experimentally computed, the amount can be considered to be increased relative to the baseline if an increase exceeding a suitable error relative to the baseline value is observed. Examples of suitable errors include 1 standard deviation, 2 standard deviations, 3 standard deviations, and greater errors.

(Radiation therapy)

An embodiment of the invention provides an indicator of radiation therapy-induced immune activation. In radiation therapy, irradiation of radiation can disrupt DNA or RNA of cancer cells to suppress cell division and/or induce apoptosis (cell death) to reduce cancer cells. Generally, radiation dose up to the maximum tolerance dose for normal cells (about 50 to 60 Gy) is divided (about 2 Gy per day) and irradiated onto tissue. While normal cells repair the disruption in genes and survive, cell death is induced in cancer cells with slower self-repairing action than normal cells from being irradiated with radiation again before the disrupted genes are repaired such that the genes cannot be repaired. This materializes tumor regression in the radiation field.

It is reported that tumor regression is induced outside of the radiation field in addition to tumor regression within the radiation field from radiation therapy. This is known as an abscopal effect. Tumor regression outside of the radiation field cannot be explained by suppression of proliferation/death of cancer cells due to radiation described above. This was understood as some type of an effect mediated by activation of the immune system, but much of the detailed mechanism is unknown. While it is understood that efficacy of cancer immunotherapy utilizing antitumor immunity can be improved by activation of the immune system by radiation therapy, a biomarker for confirming whether an abscopal effect is generated in a subject who has undergone radiation therapy had not been found. A biomarker indicating immune activation (abscopal effect) that affects the outside of the radiation field in a subject who has undergone radiation therapy is provided herein.

Radiation is roughly classified into electromagnetic waves and particle beams. Electromagnetic waves include X-rays, γ-rays, and the like. Particle beams are material particles that flow with high kinetic energy. Examples thereof include α-ray, β-ray, neutron beam, proton beam, heavy ion beam, meson beam, and the like.

Methods of irradiating radiation in radiation therapy are divided into "external irradiation" that applies radiation from the outside of the body and "internal irradiation" that applies radiation on cancer or the periphery thereof from the inside of the body. External irradiation and internal irradiation can also be combined.

External irradiation irradiates radiation through the skin from the outside of the body. A method of irradiating high energy X-rays is the most common. External irradiation includes various modes, including, but not limited to, X-ray irradiation by a LINAC (linear accelerator), three-dimensional conformal radiation therapy (3D-CRT), intensity-modulated radiation therapy (IMRT), stereotactic radiation therapy (SRT), particle beam therapy (proton beam therapy/heavy particle beam therapy), image-guided radiation therapy (IGRT), and the like.

Examples of internal irradiation modes include, but are not limited to, brachytherapy (internal radiation and intracavitary radiation), therapy using unsealed radioisotopes (internal therapy), and the like.

The mode of radiation therapy that can be within the scope of the invention is not limited, as long as radiation is irradiated in a mode that can result in immune activation. For example, the radiation field in radiation therapy can be an irradiation range including tumor tissue. Although not wishing to be bound by any theory, it is understood that tumor cells subjected to radiation therapy resulting in immunogenic cell death is important for increasing antitumor effector T cells. Examples of radiation therapy include thoracic irradiation, irradiation onto bone metastasis site, irradiation onto lymph node metastasis, irradiation onto adrenal metastasis, irradiation onto liver metastasis, irradiation onto brain metastasis, and the like.

The biomarker of the invention can be utilized for planning a schedule for radiation therapy that is intended to activate immunity. For example, no radiation therapy-induced immune activation in a subject can indicate that radiation therapy should be re-administered to a subject. Alternatively, radiation therapy-induced immune activation in a subject can indicate that radiation therapy should be discontinued.

Radiation therapy can irradiate a dose of about 1 to 3 Gy per administration about 1 to 2 times a day over 3 to 8 weeks. However, if concomitant use of small doses of multiple administrations of irradiation with cancer immunotherapy is considered, immune cells (e.g., T cells) can also be affected, so that hypofractionated radiation therapy (e.g., a small number of large doses are irradiated in 1 to 2 weeks) can be preferable.

To reduce the possibility of a side effect from radiation therapy, further radiation therapy can be withheld when it is indicated that immunity is activated. It is advantageous to activate immunity without unnecessary irradiation, especially when the dose per administration is high. In the past, it was not possible to monitor when immunity is activated, so that radiation therapy was administered in accordance with a schedule that has been empirically determined in advance. With the biomarker of the invention, a suitable timing for discontinuing radiation therapy can be determined.

(Fractionation/separation of cells)

A sample for fractionation/separation of T cells can be suitably collected from a subject using a conventional method. For example, such a sample can be collected from peripheral blood, bone marrow, tumor tissue, hematopoietic tissue, spleen, normal tissue, lymph, or the like of a subject. Sample collection from peripheral blood can be advantageous for being simple and non-invasive.

The composition of T cells in a sample of a subject can be measured by those skilled in the art using a conventional method. Generally, the number of cells that are positive for a marker (e.g., CD4) defining a cell subpopulation of interest in a sample can be measured using flow cytometry or the like. The measurement of the composition of a cell population generally uses flow cytometry, but other means may be used, such as a method using an antibody array or immunostaining on a sample comprising cells, protein expression analysis in a sample comprising cells (e.g., Western blot, mass spectrometry, HPLC, or the like), or mRNA expression analysis in a sample comprising cells (microarray, next generation sequencing, or the like).

To measure the cell count in each cell subpopulation such as CD62L$^{low}$CD4$^+$ T cell subpopulation, the cell count may be found by experimentally removing cells other than each cell subpopulation from all cells. There is a kit for the materialization thereof. For example, cells corresponding to a CD4$^+$CD62L$^{low}$ T cell subpopulation can be separated from peripheral blood without using a CD4 antibody or CD62L antibody by using a CD4$^+$ Effector Memory T cell isolation kit, human (Militenyi Biotech). This is achieved by counting and recording the total viable cell count, and counting and recording the number of cells obtained using this kit.

An antibody does not need to be used. Antibodies that can specifically recognize and bind a molecule expressed on individual cells are prepared so that they can emit color when bound to a molecule expressed on the cell surface or in cells. The antibodies are then detected to measure the number of cells that are emitting color. Since these molecules expressed on the cell surface or in the cells are proteins, mRNA encoding a protein when the protein is expressed is also formed in the cells. In other words, it is sufficient to examine mRNA in individual cells to examine the presence/absence of mRNA encoding a protein molecule of interest. This is made possible by single cell gene expression analysis, i.e., mRNA analysis at a single cell level. Examples of single cell gene expression analysis include 1) a method of next generation sequencing using Quartz-Seq, 2) a method of isolating cells using a Fluidigm C1 System or ICELL8 Single-Cell System to prepare a library with SMART-Seq v4, 3) a method of separating cells with a cell sorter and measuring the cells by quantitative PCR using an Ambion Single Cell-to-CT kit, 4) CyTOF SYSTEM (Helios), and the like.

Blood is obtained, viable cells are counted, and cells are separated with a cell sorter or the like. For example, Ambion Single Cell-to-CT kit can be used on the individual separated cells to measure the expression level of a specific gene with an apparatus for quantitative PCR. Based on the result, individual cells are examined as to which subpopulation such as the CD62L$^{low}$ CD4+ T cell subpopulation the cells fall under to count the number of cells falling under each subpopulation. Examples of candidate genes whose expression is examined include αβTCR, CD3, CD4, CD25, CTLA4, GITR, FoxP3, STAT5, FoxO1, FoxO3, IL-10, TGFbeta, IL-35, SMAD2, SMAD3, SMAD4, CD62Llow, CD44, IL-7R (CD127), IL-15R, CCR7low, BLIMP1, and the like.

Examples of genes with elevated expression in CD62L$^{low}$CD4$^+$ T cells than in CD62L$^{high}$CD4$^+$ T cells include AURAKA, CCL17, CD101, CD24, FOXF1, GZMA, GZMH, IL18RAP, IL21, IL5RA, ND2, SMAD5, SMAD7, and VEGFA (WO 2018/147291) . Expression of these genes can be studied to determine which T cell subpopulation the obtained T cells belong to and measure the amount and/or ratio of the cell subpopulation.

Examples of genes with elevated expression in CD62L$^{high}$CD4$^+$ T cells than in CD62L$^{low}$CD4$^+$ T cells include BACH2, CCL28, CCR7, CD27, CD28, CD62L, CSNK1D, FOXP1, FOXP3, IGF1R, IL16, IL27RA, IL6R, LEF1, MAL, and TCF7 (WO 2018/147291). Expression of these genes can be studied to determine which T cell subpopulation the obtained T cells belong to and measure the amount and/or ratio of the cell subpopulation.

Measurement of the ratio of cell subpopulations or comparison with a threshold value in the present invention may use a reference sample with a defined signal. Signals can be compared between a reference (e.g., particle to which a fluorescent pigment is attached) prepared to induce a fluorescent signal corresponding to a given cell subpopulation and a sample comprising a cell population to measure the amount or ratio of a cell subpopulation in the sample by comparison with a reference. Signals can also be compared between a reference (e.g., particle to which a fluorescent pigment is attached) prepared to induce a fluorescent signal corresponding to a predetermined threshold value and a sample comprising a cell population to determine the presence/absence or the amount of the marker of the invention in the T cell composition in the sample by comparison with a reference.

When determining a specific marker to be high (high expression) or low (low expression) in the present invention, those skilled in the art can use a classification baseline for expression intensity that is commonly used in the art. For example, it is possible to clearly divide CD62L into CD62L$^{low}$ and CD62L$^{high}$ using the signal intensity corresponding to a 10E2 signal when using a PE-labeled anti-human CD62L antibody as the boundary (WO 2018/147291).

The biomarker of the invention can be used to consider whether to start combination therapy or a schedule for combination therapy. If, for example, long-term survival in cancer immunotherapy in a subject is not predicted, this can suggest that combination therapy should be administered to the subject. Alternatively, if long-term survival in cancer immunotherapy in a subject is predicted, this can suggest that combination therapy should not be administered.

Alternatively, additional combination therapy can be discontinued when long-term survival is predicted as a result of combination therapy to reduce the possibility of a side effect in combination therapy.

(Cancer immunotherapy)

Cancer immunotherapy is a method of treating cancer using a biological defense mechanism of an organism. Cancer immunotherapy can be largely divided into cancer immunotherapy from strengthening the immune function against cancer and cancer immunotherapy from inhibiting the immune evasion mechanism of cancer. Cancer immunotherapy further includes active immunotherapy for activating the immune function in the body and passive immunotherapy for returning immune cells with an immune function activated or the numbers thereof expanded outside the body into the body. Whether to administer combination therapy, or a suitable timing for administering combination therapy can be found from the biomarker of the invention indicating prediction of long-term survival in cancer immunotherapy.

Examples of cancer immunotherapy include non-specific immunopotentiators, cytokine therapy, cancer vaccine therapy, dendritic cell therapy, adoptive immunotherapy, non-specific lymphocyte therapy, cancer antigen specific T cell therapy, antibody therapy, immune checkpoint inhibition therapy, and the like.

PD-1 inhibitors are representative examples of immune checkpoint inhibitors. Examples of PD-1 inhibitors include, but are not limited to, anti-PD-1 antibodies nivolumab (sold as Opdivo™) and pembrolizumab, and spartalizumab and cemiplimab. In one preferred embodiment, nivolumab can be selected.

PD-L1 inhibitors and PD-1 inhibitors can be used in the same manner in the present invention. It is understood that anti-PD-1 antibodies achieve an anticancer effect by releasing the suppression of T cell activation by a PD-1 signal. It is understood that anti-PD-LI antibodies also achieve an anticancer effect by releasing the suppression of T cell activation by a PD-1 signal. While the mechanism of PD-1 inhibiting a T cell function is not fully elucidated, it is understood that an interaction between PD-1 (programmed death 1) and PD-L1 or PD-L2 recruits a tyrosine phosphatase, SHP-1 or 2, to the cytoplasmic domain of PD-1 to inactivate a T cell receptor signaling protein ZAP70, thus suppressing activation of T cells (Okazaki, T., Chikuma, S., Iwai, Y. et al.: A rheostat for immune responses: the unique properties of PD-1 and their advantages for clinical application. Nat. Immunol., 14, 1212-1218 (2013)). This is understood to be due to recruitment of SHP-1 or 2 to a part known as an ITSM motif which dephosphorylates proximal signaling kinase of a T cell receptor in the vicinity. In other words, the memory of "being stimulated by an antigen" is erased from a T cell that has been stimulated by an antigen.

PD-1 is expressed at a high level in killer T cells and natural killer cells, which have infiltrated into a cancer tissue. It is understood that an immune response mediated by a PD-1 signal from PD-1 is attenuated by PD-L1 on tumors. While the immune response mediated by a PD-1 signal is attenuated by PD-L1, an effect of enhancing an antitumor immune response is attained by inhibiting an interaction between PD-1 and PD-L1 and/or signaling induced by an interaction by an anti-PD-1 antibody.

PD-L1 inhibitors (e.g., anti-PD-LI antibodies avelumab, durvalumab, and atezolizumab) are other examples of an immune checkpoint inhibitor.

PD-L1 inhibitors bind to and inhibit the aforementioned PD-1 pathway on the PD-L1 side to inhibit an interaction between PD-1 and PD-L1 and/or signaling induced by an interaction to induce an antitumor immune response.

CTLA-4 inhibitors (e.g., anti-CTLA-4 antibodies ipilimumab and tremelimumab) are other examples of an immune checkpoint inhibitor. CTLA-4 inhibitors activate T cells to induce an antitumor immune response. T cells are activated by an interaction of CD28 on the surface with CD80 or CD86. However, it is understood that surface expressed CTLA-4 (cytotoxic T-lymphocyte-associated antigen 4) preferentially interacts with CD80 or CD86 with higher affinity than CD20 to suppress activation, even for T cells that have been activated. CTLA-4 inhibitors induce an antitumor immune response by inhibiting CTLA-4 to prevent inhibition of an interaction between CD20 and CD80 or CD86.

In another embodiment, an immune checkpoint inhibitor may target an immune checkpoint protein such as TIM-3 (T cell immunoglobulin and mucin containing protein-3), LAG-3 (lymphocyte activation gene-3), B7-H3, B7-H4, B7-H5 (VISTA), or TIGIT (T cell immunoreceptor with Ig and ITIM domain).

It is understood that the immune checkpoints described above suppress an immune response to autologous tissue, but immune checkpoints increase in T cells when an antigen such as a virus is present in vivo for an extended period of time. It is understood that for tumor tissue, it is also an antigen which is present in vivo for an extended period of time, so that an antitumor immune response is evaded by such immune checkpoints. The aforementioned immune checkpoint inhibitors invalidate such an evasion function to achieve an antitumor effect.

In the present invention, combination therapy can be a therapy combined with another suitable cancer therapy, and typically can be co-administration of one or more additional agents. Alternatively, combination therapy can be a combination with radiation therapy. One or more additional agents can be any chemotherapeutic drug, or a second immune checkpoint inhibitor can be included. Alternatively, examples of another cancer therapy used in combination therapy include, but are not limited to, other cancer immunotherapy (e.g., adoptive cell transfer), hyperthermia therapy, surgical procedure, and the like.

One embodiment of the invention provides a composition comprising an immune checkpoint inhibitor for a patient predicted to have long-term survival in cancer immunotherapy. The composition comprising an immune checkpoint inhibitor of the invention is generally administered systemically or locally in an oral or parenteral form. It is predicted that administration of the composition comprising an immune checkpoint inhibitor of the invention to a subject by the method described herein results in long-term survival in cancer immunotherapy.

The dosage varies depending on the age, body weight, symptom, therapeutic effect, administration method, treatment time, or the like, but is generally administered, for example, orally one to several times a day in the range of 0.1 mg to 100 mg per dose per adult, or is administered parenterally (preferably intravenously) one to several times a day in the range of 0.01 mg to 30 mg per dose per adult, or is continuously administered intravenously in the range of 1 hour to 24 hours per day. Of course, the dosage varies depending on various conditions, so that an amount less than the dosage described above may be sufficient or an amount exceeding the range may be required.

For administration, a composition comprising an immune checkpoint inhibitor can have a dosage form such as a solid agent or liquid agent for oral administration or an injection, topical agent, or suppository for parenteral administration. Examples of solid agents for oral administration include tablets, pills, capsules, powder, granules, and the like. Capsules include hard and soft capsules.

The composition of the invention is one or more active ingredients (e.g., antibody to an immune checkpoint protein) that is directly used or is mixed with an excipient (lactose, mannitol, glucose, microcrystalline cellulose, starch, etc.), binding agent (hydroxypropyl cellulose, polyvinyl pyrrolidone, magnesium aluminometasilicate, etc.), disintegrant (calcium cellulose glycolate, etc.), lubricant (magnesium stearate, etc.), stabilizer, solubilizing agent (glutamic acid, aspartic acid, etc.), or the like as needed, which is formulated in accordance with a conventional method for use. The composition may also be coated with a coating agent (refined sugar, gelatin, hydroxypropyl cellulose, hydroxypropyl methyl cellulose phthalate, or the like) or coated by two or more layers as needed. Capsules made of a substance that can be absorbed such as gelatin are also encompassed.

The composition of the invention comprises a pharmaceutically acceptable aqueous agent, suspension, emulsion, syrup, elixir, or the like when formulated as a liquid agent for oral administration. In such a liquid agent, one or more active ingredients is dissolved, suspended, or emulsified in a commonly used diluent (purified water, ethanol, a mixture thereof, or the like). Such a liquid agent may also contain a humectant, suspending agent, emulsifier, sweetener, flavor, fragrance, preservative, buffer, or the like.

Examples of injections for parenteral administration include a solution, suspension, emulsion, and solid injection that is used by dissolving or suspending it in a solvent at the time of use. An injection is used by dissolving, suspending, or emulsifying one or more active ingredients into a solvent. Examples of solvents that are used include distilled water for injections, saline, vegetable oil, propylene glycol, polyethylene glycol, alcohols such as ethanol, combination thereof, and the like. Such an injection may also comprise a stabilizer, solubilizing agent (glutamic acid, aspartic acid, polysorbate 80™, or the like), suspending agent, emulsifier, analgesic, buffer, preservative, or the like. They are prepared by sterilizing or aseptic operation in the final step. It is also possible to manufacture an aseptic solid agent such as a lyophilized product, which is sterilized or dissolved in aseptic distilled water for injection or another solvent before use.

(Cancer)

Examples of target cancer in the present invention include, but are not limited to, melanoma (malignant melanoma), non-small cell lung cancer, renal cell cancer, malignant lymphoma (Hodgkin's or non-Hodgkin's lymphoma), head and neck cancer, urological cancer (bladder cancer, urothelial cancer, and prostate cancer), small cell lung cancer, thymic carcinoma, gastric cancer, esophageal cancer, esophagogastric junction cancer, liver cancer (hepatocellular carcinoma and intrahepatic cholangiocarcinoma), primary brain tumor (glioblastoma and primary central nervous system lymphoma), malignant pleural mesothelioma, gynecologic cancer (ovarian cancer, cervical cancer, and uterine cancer), soft tissue sarcoma, cholangiocarcinoma, multiple myeloma, breast cancer, colon cancer, and the like.

(Kit)

One embodiment of the invention provides a kit for determining whether long-term survival in cancer immunotherapy in a subject is predicted. A kit can comprise one or more detecting agents for a suitable molecule for detecting a cell subpopulation described herein. Such a combination of detecting agents can be used to determine the T cell composition of a subject. Such a kit can be used for measuring the ratio of a specific cell subpopulation as a novel biomarker described herein in a subject.

In one embodiment of the invention, a kit can comprise a detecting agent for

CD4 and CD62L;
(i) a marker selected from ICOS, PD-1, LAG-3 and CD28, (ii) CD4, and (iii) CD62L;
CD11c, CD141, and HLA-DR; or
CD8, CD62L, and CD137. In one embodiment, the detecting agent is an antibody. Preferably, an antibody facilitates detection of a suitably labeled marker.

[Examples]

The present invention has been described while showing preferred embodiments to facilitate understanding. While the present invention is described hereinafter based on the Examples, the above descriptions and the following Examples are provided for the sole purpose of exemplification, not limitation of the present invention. Thus, the scope of the present invention is not limited to the embodiments and Examples that are specifically described herein and is limited only by the scope of claims.

(Materials and methods)

(1) Patients

171 consecutive patients with NSCLC participated in this study from February 2016 to August 2018 at a single facility (Saitama Medical University International Medical Center). After registration, 28 patients were excluded because PBMC samples that can be assessed could not be obtained. 17 more patients were excluded because the antitumor effect could not be evaluated after 9 weeks from nivolumab therapy. Patient data was separated into two groups to obtain a discovery cohort of 40 patients and independent validation cohort of 86 patients. The feature and response of patients are listed in the following Table 1.

[Table 1]

|  |  | Discovery cohort n = 40 | Validation cohort n = 86 |
|---|---|---|---|
| Age-yr |  |  |  |
|  | Median | 67 | 69 |
|  | Range | 51-84 | 31-85 |
| Sex-no. (%) |  |  |  |
|  | Male | 26 (65) | 67 (77.9) |
|  | Female | 14 (35) | 19 (22.1) |

(continued)

| Histology-no. (%) | | |
|---|---|---|
| Sq | 10 (25) | 24 (27.9) |
| Non-Sq | 30 (75) | 62 (72.1) |
| Smoking history-no. (%) | | |
| Current or former smoker | 29 (72.5) | 68 (79.1) |
| Never smoked | 11 (27.5) | 18 (20.9) |
| Disease stage-no. (%) | | |
| c-stage III | 9 (22.5) | 18 (20.9) |
| c-stageIV | 22 (55) | 55 (64.0) |
| post-operative recurrence | 9 (22.5) | 13 (15.1) |
| Driver mutation status-no. (%) | | |
| Wild type | 33 (82.5) | 73 (84.9) |
| EGFR (19 del or L858R) | 7 (17.5) | 12 (14.0) |
| ALK | 0 (0) | 1 (1.1) |
| Objective response at 9 weeks-no. (%) | | |
| Complete or patial response | 11 (27.5) | 12 (14.0) |
| Stable disease | 15 (37.5) | 31 (36.0) |
| Progressive disease | 14 (35) | 43 (50.0) |

Sq refers to squamous epithelial cancer. c-stage refers to the clinical stage of cancer. EGFR refers to the epithelial growth factor receptor. del refers to a deletion mutation. ALK refers to anaplastic lymphoma kinase.

Patients received a dose of 3 mg/kg body weight of nivolumab every two weeks. Tumor responses were evaluated on week 9 and every 8 weeks thereafter using Response Evaluation Criteria in Solid Tumors (RECIST), version 1.1. The data collection cutoff was November 13, 2018. Samples were collected after obtaining a written informed consent approved by the institutional review board of the Saitama Medical University International Medical Center.

(2) Analysis of blood sample

Samples were collected with a heparin-added CPT Vacutainer™ tube (Becton Dickinson Vacutainer Systems, Franklin Lakes, NJ) and centrifuged at 1,500 × g for 20 minutes at room temperature to separate PBMCs from erythrocytes and granulocytes on a Ficoll gradient. PBMCs were frozen at -80°C in Cellbanker 2™ (Nippon Zenyaku Kogyo Co., Ltd., Koriyama, Japan), and the frozen cells were transferred into a liquid nitrogen tank within one week. For analysis of subpopulations of T cells, the cells were incubated for 32 to 48 hours in a medium before staining the cells.

For FACS Calibur™, the cells were stained using the following mAb: fluorescein isothiocyanate (FITC)-conjugated anti-CD3 (HIT3a) and anti-CD4 (RPA-T4), phycoerythrin (PE)-conjugated anti-CD8 (RPA-T8) and anti-CD25 (M-A251), PE-Cy7-conjugated anti-CD25 (M-A251), PE-Cy5-conjugated anti-CD62L (Dreg 56) (all from BD Pharmingen, San Diego, CA), and FITC-conjugated anti-CD62L (Dreg 56) (eBioscience, Wien, Austria). Monoclonal antibodies used in LSR Fortessa™ and mass cytometry are listed in the following Table 2.

[Table 2]

a.

| Antigen | Conjugate | Clone | Manufacturere |
|---|---|---|---|
| CD3 | BUV496 | UCHTI | BD |
| CD4 | BV650 | OKT4 | BL |
| CD8 | APC-Cy7 | SK1 | BL |
| CD16 | BUV395 | 3G8 | BD |
| CD19 | PE-Cy5.5 | SJ25C1 | eBio |
| CD25 | PE-CF594 | M-A251 | BD |
| CD28 | PerCP-Cy5.5 | CD28.2 | BL |
| CD45RA | AF700 | HI100 | BL |
| CD56 | PE-CF594 | B159 | BD |
| CD62L | BV421 | DREG-56 | BL |
| CCR4 | PE-Cy7 | L291H4 | BL |
| CCR6 | BV605 | G034E3 | BL |
| CCR7 | BV711 | G043H7 | BL |
| CTLA-4 | BV786 | BNI3 | BD |
| CXCR3 | BV785 | G025H7 | BL |
| CXCR5 | FITC | J252D4 | BL |
| Foxp3 | PE | 236A/E7 | eBio |
| ICOS | BUV395 | DX29 | BD |
| ICOS | PE-Cy7 | ISA-3 | eBio |
| Ki-67 | BV605 | Ki-67 | BL |
| LAG-3 | FITC | 17B4 | ENZ |
| PD-1 | APC | MIH4 | BD |
| Ms IgG1 $\kappa$ | BUV395 | X40 | BD |
| Ms IgG1 $\kappa$ | BV421 | MOPC-21 | BL |
| Ms IgG1 $\kappa$ | BV605 | MOPC-21 | BL |

b.

| Antigen | Conjugate | Clone | Manufacturere |
|---|---|---|---|
| CD134/OX40 | 150Nd | AC 35 | FLUIDIGM |
| Gelactin-9 | 163Dy | 9M1-3 | FLUIDIGM |
| CD141 | 173Yb | 1A4 | FLUIDIGM |
| CD123/IL-3R | 151Eu | 6H6 | FLUIDIGM |
| CD11b/Mac-1 | 167Er | ICRF44 | FLUIDIGM |
| CD33 | 169Tm | WM53 | FLUIDIGM |
| CD16 | 209Bi | 3G8 | FLUIDIGM |
| CD56/NCAM | 149Sm | NCAM16.2 | FLUIDIGM |
| CD11c | 159Tb | Bu15 | FLUIDIGM |
| CD14 | 175Lu | M5E2 | FLUIDIGM |
| HLA-DR | 170Er | L243 | FLUIDIGM |
| HLA-ABC | 144Nd | W6-32 | FLUIDIGM |
| CD40 | 165Ho | 5C3 | FLUIDIGM |
| CD137 Ligand | 158Gd | 5F4 | FLUIDIGM |
| CD86/B7.2 | 156Gd | IT2.2 | FLUIDIGM |
| CD3 | 141Pr | UCHT1 | FLUIDIGM |
| CD8 | 146Nd | RPA- T8 | FLUIDIGM |
| CD4 | 145Nd | RPA- T4 | FLUIDIGM |
| Tbet | 161Dy | 4B10 | FLUIDIGM |
| CD19 | 142Nd | HIB19 | FLUIDIGM |
| CD45RA | 169 Tm | HI100 | FLUIDIGM |
| BCL-6 | 163Dy | K112-91 | FLUIDIGM |
| FoxP3 | 159 b | 259D/C7 | FLUIDIGM |
| CD183/CXCR3 | 156Gd | G025H7 | FLUIDIGM |
| CD194/CCR4 | 149Sm | L291H4 | FLUIDIGM |

(continued)

a.

b.

| Antigen | Conjugate | Clone | Manufacturere | Antigen | Conjugate | Clone | Manufacturere |
|---|---|---|---|---|---|---|---|
| Ms IgG1 κ | BV785 | MOPC-21 | BL | CD196/CCR6 | 176Yb | G034E3 | FLUIDIGM |
| Ms IgG1 κ | FITC | MOPC-21 | BL | CD278/ICOS | 143Nd | C398.4A | FLUIDIGM |
| Ms IgG1 κ | PerCP-Cy5.5 | P3.6.2.8.1 | eBio | CD137 | 158Gd | 143Nd | FLUIDIGM |
| Ms IgG1 κ | PerCP-Cy5.5 | MOPC-21 | BL | CD69 | 144Nd | FN50 | FLUIDIGM |
| Ms IgG1 κ | PE-CF594 | X40 | BD | CD134/OX40 | 150Nd | AC 35 | FLUIDIGM |
| Ms IgG1 κ | PE-Cy7 | P3.6.2.8.1 | eBio | CD154/CD40L | 168Er | 24-31 | FLUIDIGM |
| Ms IgG1 κ | PE-Cy7 | MOPC-21 | BL | CD27 | 155Gd | L128 | FLUIDIGM |
| Ms IgG1 κ | APC | MOPC-21 | BD | CD28 | 160Gd | CD28.2 | FLUIDIGM |
| Ms IgG2a κ | BV711 | MOPC-173 | BL | CD152/CTLA-4 | 170Er | 14D3 | FLUIDIGM |
| Ms IgG2a κ | BV786 | G155-178 | BD | CD80/B7.1 | 162Dy | 2D10.4 | FLUIDIGM |
| Ms IgG2b κ | BV605 | MPC-11 | BL | CD103 | 151Eu | Ber-ACT8 | FLUIDIGM |
| | | | | CD279/PD-1 | 175Lu | EH12.2H7 | FLUIDIGM |
| | | | | TIM-3 | 154Sm | F38-2E2 | FLUIDIGM |
| | | | | CD223/LAG-3 | 165Ho | 11C3C65 | FLUIDIGM |
| | | | | CD274/PD-L1 | 148Nd | 29E.2A3 | FLUIDIGM |
| | | | | CD273/PDL2 | 172Yb | 24F.10C12 | FLUIDIGM |
| | | | | CD62L | 153Eu | DREG-56 | FLUIDIGM |
| | | | | CD 197/CCR 7 | 167Er | G043H7 | FLUIDIGM |

BD refers to a product of Becton, Dickinson and Company. BL refers to a product of BioLegend. eBIO refers to a product of eBioscience. ENZ refers to a product of Enzo Life Sciences.

Cell surface phenotypes were analyzed by direct immunofluorescent staining of $1 \times 10^6$ cells with a fluorophore-conjugated mAb. Figures **19** and **20** show the gating strategy. 10,000 cells were analyzed from each sample using FACS Calibur™ and LSR Fortessa™ flow cytometers (Becton Dickinson, Sunnyvale, CA) and FlowJo™ software. 20,000 cells were also analyzed using a CyTOF™ (Fluidigm Corp., San Francisco, CA) mass cytometer and Cytobank™ software to obtain viSNE analysis.

(3) Purification of cells

PBMCs were collected from two patients in each responder group (PR, SD, and PD). CD4+ T cells were purified by negative selection using a human CD4+ T cell isolation kit (Dynal Biotech, Oslo, Norway). CD4+ T cells were separated into CD62L$^{high}$ cells and CD62L$^{low}$ cells using anti-CD62L mAb-coated microbeads and MACS™ system (Miltenyi Biotec, Auburn, CA) in accordance with the manufacturer's instruction. The purity of all cells was > 90%.

(4) Microarray analysis

CD62L$^{high}$CD4+ T cells and CD62L$^{low}$CD4+ T cells in PBMCs from two patients of each responder type were purified. Total RNA was isolated using TRIzol reagent (Thermo Fisher Scientific, Waltham, MA). cDNA and cRNA were then synthesized, and a single stranded cDNA (ssDNA) was labeled using a WT Plus Reagent Kit (Thermo Fisher Scientific) in accordance with the manufacturer's instruction. Total RNA (0.5 μg) was reverse-transcribed onto cDNA, and then cRNA was synthesized. From 15 μg of cRNA, ssDNA was reverse-transcribed and then labeled. 1.8 μg of labeled ssDNA was hybridized using a microarray (Clariom S Assay, human; Thermo Fisher Scientific) in a GeneChip Hybridization Oven 645. The hybridized array was scanned using a GCS3000 7G System (Thermo Fisher Scientific). The accession ID number of gene expression data is GSE103157.

The difference in gene expression between two sets was estimated as follows in order to identify a gene expression signature from two sets of gene expression data. First, outliers were tested for all values of probe. A z score was calculated for each probe using the mean and dispersion of the probe values excluding the outliers. To compare z scores of two gene sets, the z score of each gene was converted into a probability, and the difference in the probability of each gene between two sets (p$^d$) was calculated as follows.

[Numeral 1]

$$p_k^d = |p(z_k^a) - p(z_k^b)| = \left| \frac{1}{\sqrt{2\pi}} \int_{-\infty}^{z_k^a} e^{-\frac{z}{2}} dz - \frac{1}{\sqrt{2\pi}} \int_{-\infty}^{z_k^b} e^{-\frac{z}{2}} dz \right|$$

wherein the k-th genes between two gene sets (a and b) were compared. In this analysis, a gene resulting in

[Numeral 2]
$$p_k^d > 0.2$$

was selected as a gene signature.

(4) Statistical analysis

SAS 9.4 (SAS Institute Inc., Cary, NC) and Prism 8 (GraphPad, La Jolla, CA) were used for the statistical analysis. Unless noted otherwise, data is expressed as mean value $\pm$ standard error of the mean value. Student's t-test was used for testing the difference between two populations. One-way ANOVA was used for multi-group comparison. A prediction formula was developed using a logistic regression model and data for the discovery cohort. The performance of the prediction formula was evaluated using data for the independent validation cohort. The survival curve was estimated using the Kaplan-Meier method. All p values were two-sided. P < 0.05 was deemed statistically significant.

(Example 1: Results from nivolumab therapy)

The inventor studied the results for previously treated advanced lung cancer patients who received therapy using an anti-PD-1 antibody nivolumab, until 5 years after starting the therapy based on Brahmer et al. (Five-year follow-up from the CA209-003 study of nivolumab in previously treated advanced non-small cell lung cancer: clinical characteristics of long-term survivors. Presented at: 2017 AACR Annual Meeting; April 1-5, 2017; Washington, DC. Abstract CT077) shown in Figure **1**.

In the left diagram, the curve showing overall survival stops decreasing from about year 3. 5 year survival is observed in 16% of all cases. The blue bars in the swimmer plot in the right diagram show the therapeutic period. Although therapy is completed in two years in this clinical trial, 12 patients (#1, 2, 3, 4, 6, 7, 8, 9, 10, 11, 12, and 13) out of 16 patients who survived 5 years survived progression free for 3 years or more without receiving any therapy after completion of nivolumab therapy. It is known that there are patients who achieve a "therapy-like" effect without recurrence or relapse after discontinuation of therapy among such patients who received anti-PD-1 antibody therapy. It is reported that there are many patients who are already surviving from melanoma without recurrence with no treatment for 10 years.

Figure **2** is a graph modified from Brahmer et al. (N Eng J Med 2015: 373: 123-135). It can be understood that the progression free survival curve in Figure **2** for the long-term progression free survival group observed in Figure **1** forms a tail plateau observed after 18 months. The tail plateau indicates overall survival of about 5 years. Meanwhile, it can be understood that there is a "non-responder group", which experiences early disease progression within 3 months, and a "short-term responder group", which first achieves an antitumor effect and deviates away from a PFS curve of conventional therapy, but experiences progression in the disease thereafter.

It can be understood from analysis of these results that there is a "long-term progression free survival group" that exhibits a "therapy-like" effect over a long period of time without recurrence or relapse even after discontinuation of therapy at a ratio of about 10 to 20% of patients who received anti-PD-1 antibody therapy. It can be understood that if such a "long-term progression free survival group" (LS) can be identified, the presence/absence of the need for combination therapy, the timing of starting combination therapy, or the timing of suspension/discontinuation of combination therapy can be property determined.

(Example 2: FACS Calibur analysis)

FACS Calibur analysis was performed via the following procedures.

(1) From blood collection to cryopreservation

**[0223]**

　　*About 14 to 16 ml of blood was collected in two Becton Dickinson (BD) 7 to 8 mL Vacutainer spitz (containing heparin) tubes.
　　*Blood was centrifuged at 3200 rpm for 20 minutes at room temperature (18 to 25°C) within 2 hours to half day after blood collection.
　　*After collecting plasma, plasma components and cellular components remaining on the top layer of a gel barrier were stirred and collected by pipetting into a 50 mL centrifuge tube (two spitz tubes were consolidated into one tube). PBS (+10% FBS) was added at the same amount as the collected liquid volume (about 13 mL).
　　*The tubes were centrifuged at 1600 rpm for 5 minutes at 4°C.
　　*After centrifugation, the supernatant was discarded, and the rest was suspended in 4.5 ml of TakaraBio's Cellbanker 2 solution.
　　*Cells suspended in Cellbanker 2 solution was dispensed into 2.0 ml cryogenic vials (external thread cap, round bottom) at 1.5 ml each (total of three vials). The vials were then stored in a -80°C deep freezer in a BiCell (container for programmed freezing).
　　*The vials were stored in a liquid nitrogen tank when storing for over one week.

(2) From thawing to FMC analysis

**[0224]**

　　*The cryogenic vials were retrieved and quickly thawed with warm water with a temperature of about 37°C.
　　*Cells were collected in a 50 ml centrifugation tube, and HBSS were added until reaching 50 ml.
　　*The cells were centrifuged at 1600 rpm for 5 minutes at 4°C.
　　*The supernatant was discarded after centrifugation, and the cells were suspended in HBSS. HBSS was further added until reaching 50 ml. 50 μl was retrieved to count the cells.
　　*The cells were centrifuged at 1600 rpm for 5 minutes at 4°C.
　　*CM (RPMI 1640 + 10% FCS) was added so that the density would be $1 \times 10^6$ cells/ml. The cells were transferred to a culture dish and cultured for 36 to 48 hours in an incubator.

*The culture solution was transferred to a 50 ml centrifugation tube. The cells were counted and centrifuged at 1600 rpm for 5 minutes at 4°C.

*The supernatant was discarded after centrifugation, and the cells were suspended in a FACS buffer. FACS buffer was added so that the density would be 0.3 to $1 \times 10^6$ cells/ml based on the cell count. 1 ml of cell suspension was placed in each FACS tube.

*The tube was centrifuged at 1600 rpm for 5 minutes at 4°C.

*After centrifugation, the supernatant was discarded. About 100 μl of supernatant was kept. Cell pellets were broken up with a vortex or the like. After marking each FACS tube, an antibody solution was added in accordance with the protocol. The tubes were left standing at 4°C for 30 to 60 minutes.

*2 ml of FACS buffer was added to the FACS tubes, which were centrifuged at 1600 rpm for 5 minutes at 4°C.

*After centrifugation, the supernatant was discarded. 0.5 ml of 1% PFA was added. Pellets were broken up with a vortex or the like to perform FCM analysis.

*Cells were suspended in FACS buffer. FACS buffer was added so that the density would be 0.3 to $1 \times 10^6$ cells/ml based on the cell count. 1 ml of cell suspension was placed in each FACS tube.

The following are the combinations of antibodies and labels used in FACS Calibur analysis.

[Table 3]

| For T cells | | | |
|---|---|---|---|
| | FITC | PE | fl3 |
| 1 | CD4 | CD62L | CD8 |
| 2 | CD4 | PD-1 | CD62L |
| 3 | CD4 | LAG-3 | CD62L |
| 4 | CD4 | ICOS | CD62L |
| 5 | CD4 | CD62L | CD28 |
| 6 | CD4 | CD137 | CD62L |
| 7 | CD62L | PD-1 | CD8 |
| 8 | CD62L | CD137 | CD8 |
| 9 | CD8 | CD62L | CD28 |
| 10 | CD4 | foxp3 | CD25 |
| | | | |
| For DC | | | |
| | FITC | PE | fl3 |
| 1 | CD141 | isotype control | CD11c |
| 2 | CD141 | HLA-DR | CD11c |
| 3 | CD141 | CD80 | CD11c |

FACS Calibur analysis was performed. The left graph of Figure **3** shows the percentage of CD62L^low CD4^+ T cells in a non-responder group with early disease progression and in other responder groups. The responder groups had a higher percentage of CD62L^low CD4^+ T cells relative to the non-responder group, but it can be seen that there is one group with a percentage of CD62L^low CD4^+ T cells exceeding 40% among the responder groups (left group of Figure **3).**

The right graph of Figure **3** shows results of plotting the percentage of CD62L^low CD4^+ T cells by separating responders groups into a patient group maintaining a progression free state for 18 months or longer (LS group) and a once responding but later with disease progression group in which patients were once respondent but experienced disease progression later (R group). It can be seen that a long-term progression free survival group has appeared from a group having a percentage of CD62L^low CD4^+ T cells exceeding 40%.

(Example 3: ROC analysis and PFS plot)

The left graph of Figure **4** shows results of ROC analysis on data obtained in Example 2. Specifically, analysis from classifying 18 month or more progression free survival groups shows that predication was possible at sensitivity of 85.7% and specificity of 83.3% when using percentage of CD62L$^{low}$CD4$^+$ T cells of > 35.85% as a threshold value at a p-value of 0.0008. AUC was also very good at 0.896.
The right graph of Figure **4** shows results of plotting the percentage of CD62L$^{low}$CD4$^+$ T cells on the horizontal axis and number of days of progression free survival (PFS (days)) on the vertical axis. This shows that most are early disease progression groups when the percentage of CD62L$^{low}$CD4$^+$ T cells is less than 20%, but half or more are long-term survival groups at 35.85% or higher.
These results show that the percentage of CD62L$^{low}$CD4$^+$ T cells is an excellent biomarker for predicting long-term survival in cancer immunotherapy.

(Example 4: Difference in ratios of CD62L$^{low}$ T cell subpopulation between responders and non-responders)

171 consecutive patients with NSCLC participated in this study. The patients were treated with nivolumab from February 2016 to August 2018 at a single facility (Saitama Medical University International Medical Center) (Figure **10a**). Since this study is an observational study of actual therapy, all patients had been treated in advance by cell destructive chemotherapy in accordance with a PMDA package insert before nivolumab therapy. After registration, 28 patients were excluded because PBMC samples that can be assessed could not be obtained. 17 more patients were excluded because the antitumor effect could not be evaluated after 9 weeks from nivolumab therapy. Table 1 lists features of all patients included in the discovery cohort and validation cohort. To identify a biomarker that distinguishes patients exhibiting early disease progression, the inventor performed an evaluation by computer tomography (CT) at 9 weeks after nivolumab therapy. Patients exhibiting progression in disease were considered "non-responders", and patients exhibiting complete response (CR), partial response (PR), or stable disease (SD) were considered "responders". In particular, the survival period of patients exhibiting early disease progression by 9 weeks after therapy was very short, while SD patients and PR patients exhibited preferred overall survival (OS) (Figure **16**). Non-responders appear to include a patient group that mostly does not benefit from a life prolongation effect by nivolumab therapy. For this reason, 34 parameters (including leukocyte, lymphocyte, and neutrophil count; serum immunoglobulin levels of IgG, IgA, IgM, IgE, and IgD; carcinoembryonic antigen and cytokeratin 19 fragment tumor markers; and biochemical data on antinuclear antibody, rheumatoid factor, AST, ALT, LDH, and CRP) were analyzed from the study data. However, a significant difference was not found between responders and non-responders.
In recent years, immunity of systemic T cells consisting of a subpopulation of CD62L$^{low}$CD44$^+$CD69$^+$CD90$^+$CD27$^{low}$T-bet$^+$CD4$^+$ T cells is demonstrated to be required for antitumor immune responses in tumor, tumor draining LN, and peripheral blood. Furthermore, it was found that a significant number of phenotypes of CD4$^+$ T cells are in one of three antitumor T cell clusters in human melanoma infiltrating lymphocytes. Thus, the inventor investigated such T cell subpopulations, especially the CD62L$^{low}$ subpopulation in peripheral blood of NSCLC patients.
As a result, the ratio of CD62L$^{low}$ cells in all CD4$^+$ T cell populations (Figure **10b**, P < 0.0001) and the ratio of CD62L$^{low}$ cells in all CD8$^+$ T cell populations (Figure **10c**, P = 0.0020) were significantly higher in responders relative to non-responders. Meanwhile, the ratio of CD25$^+$FOXP3$^+$ cells in all CD4$^+$ T cell populations was significantly higher (P = 0.034) in non-responders (Figure **10d**). The inventor selected the ratio of CD62L$^{low}$ cells in all CD4$^+$ T cell populations as one of the independent factors, because a robust difference was observed. The ratio of CD25$^+$FOXP3$^+$ cells in all CD4$^+$ T cell populations was also selected as another factor that is negatively correlated with clinical results, constituting a T cell cluster that is different from a CD62L$^{low}$CD4$^+$ T cell cluster. To detect non-responder patients, a logistic regression model comprising the two selected factors was used to obtain the following formula. [-31.3 + 12.0 × log [%CD62L$^{low}$ T cells in all CD4$^+$ T cell populations: X] - 6.1 × log[%CD25$^+$FOXP3$^+$ T cells in all CD4$^+$ T cell populations: Y]].
This formula can be approximated as [-31.3 + 6.0 × log (X$^2$/Y)] . Thus, the inventor obtained a prediction formula using X$^2$/Y as a variable in the formula.

(Example 5: Value of formula predicting nivolumab response)

The inventor determined a value of a prediction formula for responders and non-responders (Figure **10e**, P < 0.0047) and performed receiver operating characteristic (ROC) analysis to detect non-responders within the discovery cohort at 9 weeks (Figure **10f**). When the threshold value of the prediction formula was set to 192 (this is the point where the likelihood ratio of the ROC curve is at the maximum), sensitivity and specificity were 85.7% and 100%, respectively. Progression free survival (PFS) curves and OS curves were plotted for patients identified as a responder type (X$^2$/Y ≥ 192) and patients identified as a non-responder type (X$^2$/Y < 192) by analysis of PBMCs collected before nivolumab therapy (Figures **10g** and **10h**). Responders and non-responders in the discovery cohort (threshold value = 192) were significantly different in terms of both PSF and OS (P < 0.0001). Next, the inventor tested whether the threshold value of the prediction formula ($X^2/Y$ < 192) can distinguish non-responders in an independent validation cohort including 86 consecutive patients. The value of the prediction formula was significantly (P = 0.0008) higher for responders relative to non-responder patients in the validation cohort (Figure **10i**). Responder-type patients exhibited significantly longer PSF (Figure **10j**, P < 0.0001) and OS (Figure **10k,** P < 0.0001) relative to non-responder type patients in the validation cohort. All survival data (n = 143) including data for patients whose tumor response could not be evaluated at week 9 exhibited a significant difference between non-responder type patients and responder type patients (Figure **16c** and **16d).** ROC analysis was performed on the prediction formula to detect non-responders in the validation cohort (n = 86) and in all patients who could be evaluated (n = 126) at week 9 (Figures **16e** and **16f**). When the threshold value of the prediction formula was set to 192, sensitivity and specificity were 92.9% and 72.1% in the validation cohort (P < 0.0001), and 87.5% and 81.2% in all patients who could be evaluated (P < 0.0001). The following Table 4 shows the relationship between histological observation obtained from the patients (n = 126) and objective response and prediction formula results.

[Table 4]

| Histology, Driver | Objective response at 8 weeks | no. (%) | Responder type |
|---|---|---|---|
| Sq (n = 35) | Complete or patial response | 9 (25.7) | 8 |
|  | Stable disease | 17 (48.6) | 13 |
|  | Progressive disease | 9 (25.7) | 2 |
| non-Sq (Driver wt) (n = 71) | Complete or patial response | 12 (16.9) | 12 |
|  | Stable disease | 26 (36.6) | 21 |
|  | Progressive disease | 33 (46.5) | 3 |
| Driver mt+ (n = 20) | Complete or patial response | 1 (0.5) | 1 |
|  | Stable disease | 4 (20) | 1 |
|  | Progressive disease | 15 (79.5) | 2 |

Objective responses and prediction formula results are shown in the table described above. Non-responder type and responder type indicate patients whose results of calculation in accordance with the prediction formula are less than 192 and greater than or equal to 192, respectively. Sq refers to squamous epithelial cancer. Driver wt means that EGFR and ALK are wild-type. Driver mt+ included 19 patients with an EGFR activating mutation and one patient with an ALK fusion gene mutation.

As shown in the following Table 5, multivariate analysis revealed that the prediction formula functions as an independent factor that is correlated with PFS and OS.

[Table 5]

| Overall survival (OS) | | Univariate analysis | | Multivariate analysis | | |
|---|---|---|---|---|---|---|
|  |  | 1-yr survival rate (%) | p-value | HR | 95%CI | *p*-value |
| Age | ≤69 / > 69 | 56/63 | 0.75 | 1.03 | 0.78-1.35 | 0.83 |
| Gender | Male / Female | 59 / 57 | 0.83 | 1.11 | 0.80-1.57 | 0.52 |
| PS | 0 / 1 or 2 or 3 | 78 / 39 | <0.01 | 0.69 | 0.52-0.90 | <0.01 |
| $X^2/Y$ | High / Low | 81 / 34 | <0.01 | 0.46 | 0.34-0.61 | <0.01 |

| Progression-free survival (PFS) | | Univariate analysis | | Multivariate analysis | | |
|---|---|---|---|---|---|---|
|  |  | 1-yr survival rate (%) | *p*-value | HR | 95%CI | *p*-value |
| Age | ≤69 / > 69 | 29 / 27 | 0.83 | 1.08 | 0.87-1.36 | 0.45 |
| Gender | Male / Female | 31 / 20 | 0.04 | 0.87 | 0.68-1.13 | 0.30 |
| PS | 0 / 1 or 2 or 3 | 32/23 | 0.04 | 0.91 | 0.73-1.14 | 0.44 |

(continued)

| Progression-free survival (PFS) | | Univariate analysis | | Multivariate analysis | | |
|---|---|---|---|---|---|---|
| | | 1-yr survival rate (%) | *p*-value | HR | 95%CI | *p*-value |
| $X^2$/Y | High / Low | 46/11 | <0.01 | 0.44 | 0.34-0.56 | <0.01 |

Abbreviation: PS, performance status; HR, hazard ratio; 95%CI, 95% confidential interval.
PS refers to performance status. HR refers to the hazard ratio. 95% CI refers to 95% confidence interval.

(Example 6: CD62L$^{low}$ CD4$^+$ T cell subpopulation and other T cell subpopulations)

It is still unknown how a single marker for CD62L can distinguish subpopulations of CD4$^+$ T cells that predict antitumor response of PD-1 blocking therapy. In this regard, CD62L$^{low}$CD4$^+$ T cell subpopulations were defined, and the inventor performed mass cytometry and microarray analysis in addition to FCM analysis in order to study the relationship between CD62L$^{low}$CD4$^+$ T cell subpopulations and other T cell subpopulations. First, the correlation between the ratios of T cell subpopulations was analyzed. Both CCR7 and CD45RA are used as a baseline for distinguishing CCR7$^+$CD45RA$^+$ naive T cells, CCR7$^+$CD45RA$^-$ central memory cells (CM), CCR7$^-$CD45RA$^-$ effector memory T cells (EM), and CCR7$^-$CD45RA$^+$ effector T cells (EMRA). For this reason, the inventor studied the correlation thereof with CD62L$^{low}$CD4$^+$ T cell subpopulations. CD8$^+$ T cells were distinctly classified into four subpopulations with respect to expression of CD45RA and CCR7, and CD4$^+$ T cells in peripheral blood exhibited different patterns lacking a CD45RA$^+$CCR7$^-$ subpopulation (Figures **11a** and **11b**). The ratio of CD62L$^{low}$CD4$^+$ T cells had a positive correlation with a CCR7$^-$CD45RA$^-$EM subpopulation (P < 0.0001), but had a significantly negative correlation with other CCR7$^+$CD45RA$^{-/+}$ subpopulations (Figures **11c** and **11d**). It appears that the CCR7$^-$CD45RA$^-$CD4$^+$ T cell subgroup and CD62L$^{low}$CD4$^+$ T cell subgroup include a similar T cell subpopulation. However, clinical results after nivolumab therapy were not associated with the ratio of CCR7$^-$CD45RA$^-$ CD4$^+$ T cells (Figures **17a** and **17b**). Next, the inventor studied the correlation with type 1 (Th1) T cells, type 2 (Th2) T cells, and type 17 (Th17) helper T cells, and T follicular helper (Tfh) cells. CD62L$^{low}$CD4$^+$ T cell subpopulations had a strong correlation with a classical Th1 subpopulation of CXCR3$^+$CCR4$^-$CCR6$^-$ (P < 0.0001), but had a negative correlation with a Th2 subpopulation of CXCR3$^-$ CCR4$^+$CCR6$^-$ (Figures **11e** to **11h**) (P = 0.0013). A CD62L$^{low}$CD4$^+$ T cell subpopulation also had a positive correlation with CD8$^+$ T cells (Figure **11i**)) (P < 0.0001), and a positive correlation with the ratio of an effector CD8$^+$ T cell subpopulation (Figure **11j**)) (P = 0.0091). Interestingly, a CCR7$^-$CD45RA$^-$CD4$^+$ subpopulation had a weak correlation with a Th1 subpopulation (P = 0.01), but did not have a correlation with a Th2 population (Figures **17c** to **17e).** In agreement with these results, mass cytometry analysis revealed that a CD62L$^{low}$CD4$^+$ cluster and a CCR7$^-$CD4$^+$ cluster are not identical (Figure **12a**). Unsupervised cluster analysis on gated CD4$^+$CD3$^+$ T cells revealed that most CD62L$^{low}$CD4$^+$ T cell subpopulations belong to a CD27$^-$T-bet$^+$FOXP3$^-$CXCR3$^+$CCR4$^-$CCR6$^-$ subpopulation. Meanwhile, a CD45RA$^-$CCR7$^-$ subpopulation more broadly included CD27$^+$, T-bet$^-$, and FOXP3$^+$ subpopulations. It was found from heat map analysis for showing the mean expression level of molecules that T-bet and CXCR3 were significantly more strongly expressed, and CD27 was significantly more weakly expressed in CD62L$^{low}$CD4$^+$ T cells relative to CD45RA$^-$CCR7$^-$CD4$^+$ T cells (Figures **12b** and **12c**). It appears that instead of CD45RA$^-$CCR7$^-$, a single marker of CD62L$^{low}$ can distinguish relatively similar subpopulations of CD62L$^{low}$CD45RA$^-$CCR7$^-$CD27$^-$ T-bet$^+$CXCR3$^+$CD4$^+$ T cells. The inventor studied the correlation between CD62L$^{low}$CD4$^+$ T cells and PD-1, LAG-3, and CTLA-4 expression. Instead of CCR7$^-$CD45RA$^-$CD4$^+$ T cells, CD62L$^{low}$CD4$^+$ T cells had a positive correlation with the expression of PD-1 and LAG-3 in CD62L$^{low}$CD4$^+$ cells, and a positive correlation with expression of PD-1 in CD8$^+$ T$_{EMRA}$ cells (Figure **13a** to **13c,** and Figure **18a** to **18c**). They also had a negative correlation with the expression of CTLA-4 in CD62L$^{low}$CD4$^+$ T cells (Figure **13d** and Figure **18d**) .

(Example 7: Gene expression of CD62L$^{low}$CD4$^+$ T cells in responders and non-responders)

Next, the inventor performed microarray analysis to study the difference in CD62L$^{low}$CD4$^+$ T cells at the molecular level between responders and non-responders. To do so, first, the difference in the gene expression in CD62L$^{high}$CD4$^+$ T cells and CD62L$^{low}$CD4$^+$ T cells was elucidated. As a result, CD62L$^{high}$CD4$^+$ T cells and CD62L$^{low}$CD4$^+$ T cells had different gene expression profiles (Figure **14a**). In agreement with a previous report, it is understood that most CD62L$^{high}$CD4$^+$ T cells are naive T cells because genes of C-C chemokine receptor type 7 (CCR7), CD28, and transcription factor 7 (TCF7) were strongly expressed in CD62L$^{high}$CD4$^+$ T cells of all patients. CD62L$^{low}$CD4$^+$ T cells were strongly expressing aurora kinase A (AURKA), C-C motif chemokine ligand 17 (CCL17), granzyme A and H (GZMA and GZMH), NADH dehydrogenase 2 (ND2), and interleukin 21 (IL-21). Signature genes compared between PR (partial response) and SD (stable disease), PR and PD (progressive disease), SD and PD, PR + SD and PD, and PR and SD + PD derived cells (1884, 1826, 1410, 1167, and 1513 genes, respectively) were combined with all 3458 genes. The

40

expression of 30 of the 53 genes known to be associated with T cell-mediated immunity among them is shown from the viewpoint of responses to nivolumab therapy (Figure **14b**). This indicates that C-type lectin domain family 2 member A (CLEC2A), interleukin 7 (IL7), transforming growth factor beta receptor 3 (TGFBR3), Interferon alfa (IFNA), C-X-C chemokine receptor type 3 (CXCR3), and histone deacetylase 9 (HDAC9) were preferentially expressed in $CD62L^{low}CD4^+$ T cells derived from responders.

(Example 8: Change after nivolumab therapy)

The change in various markers after nivolumab therapy relative to before nivolumab therapy was measured. The percentage of $CD62L^{low}CD8^+$ T cells, percentage of $CD28^+CD62L^{low}CD8^+$ T cells, percentage of $CD62L^{low}CD4^+$ T cells, percentage of $ICOS^+CD62L^{low}CD4^+$ T cells, and percentage of $LAG3^+CD62L^{low}CD4^+$ T cells were used as the tested percentage of cells. Among them, the percentage of $CD62L^{low}CD4^+$ T cells had the best correlation, which decreased significantly after nivolumab therapy (P = 0.0001) (Figure **5).**

Next, $CD4^+$ T cells were prepared from a responder group (Responder, left graph in Figure **6)** and non-responder group (Non-responder, right graph in Figure **6)** at before nivolumab therapy and 4 weeks after therapy to test the percentage of $CD62L^{low}CD4^+$ T cells. As a result, a decrease in the percentage of $CD62L^{low}CD4^+$ T cells in the responder group had a better correlation than a decrease in the percentage of $CD62L^{low}CD4^+$ T cells in the non-responder group (Figure **6**: responder group P = 0.0016, non-responder group P = 0.32). The correlation of the responder group was better than the correlation for the percentage of $LAG3^+CD62L^{low}CD4^+$ T cells or the correlation for the percentage of $CD28^+CD62L^{low}CD8^+$ T cells (data no shown).

Next, PBMCs were prepared from patient groups at 12 to 92 weeks after the start of nivolumab therapy. Specifically, PBMCs were prepared for each of a group of 6 patients at an average of 63.3 weeks (28 to 92 weeks) after the start of therapy who have acquired therapeutic resistance after the start of therapy (Acquired resistance, middle of graph in Figure **7)** and a group of 8 patients at an average of 64.5 weeks (12 to 92 weeks) after the start of therapy who are still responsive to therapy after the start of therapy (Ongoing response, left side of graph in Figure **7).** As a control, PBMCs were prepared from a group of 5 patients who are resistant to therapy at the start of therapy (Primary resistance, right side of graph in Figure **7).** The percentage of $CD62L^{low}CD4^+$ T cells (i.e., percentage of $CD62L^{low}$ T cells in all $CD4^+$ T cell populations, the left graph in Figure **7)** and $X^2/Y$ wherein the percentage of $CD62L^{low}CD4^+$ T cells is "X" and the percentage of $CD25^+FOXP3^+CD4^+$ T cells (i.e., percentage of $CD25^+FOXP3^+$ T cells in all $CD4^+$ T cell populations) is "Y" (right graph in Figure **7)** are shown. The group of patients who are still responsive to therapy after the start of therapy exhibited a much higher value in both the percentage of $CD62L^{low}CD4^+$ T cells (left graph of Figure **7)** and $X^2/Y$ wherein the percentage of $CD62L^{low}CD4^+$ T cells is "X" and the percentage of $CD25^+FOXP3^+CD4^+$ T cells is "Y" (right graph of Figure **7).** This result revealed that the percentage of $CD62L^{low}CD4^+$ T cells and $X^2/Y$ wherein the percentage of $CD62L^{low}CD4^+$ T cells is "X" and the percentage of $CD25^+FOXP3^+CD4^+$ T cells is "Y" are both excellent prediction indicators for whether a patient is still responsive to therapy even after therapy with a checkpoint inhibitor such as nivolumab. When the percentage of $CD62L^{low}CD4^+$ T cells was used as an indicator, P = 0.0088. When $X^2/Y$ was used as an indicator, P = 0.017.

(Example 9: Marker characteristic of long-term progression free survival)

As described above, it can be understood that the progression free survival curve in Figure **2** forms a tail plateau observed after 18 months in a long-term progression free survival group observed in Figure **1.** In this regard, the inventor defined a patient with a progression free survival period of > 500 days as a long-term responder, and defined a patient who was initially responsive to therapy but acquired resistance to exhibit progression in disease within 500 days after the nivolumab therapy as a short-term responder.

PBMCs derived from a patient in a group with long-term progression free survival of 500 days or longer after the start of nivolumab therapy and PBMCs of a group of patients who were responders at the start of therapy but subsequently exhibited exacerbation were prepared and compared. Figure **8** shows the results. Figure **8** is a graph showing the percentage of $CD62L^{low}CD4^+$ T cells for long-term progression free survival group (LR), short-term responder group (SR), and non-responder group (NR) (left graph in Figure **8)** and a graph showing $X^2/Y$ wherein "X" is the percentage of $CD62L^{low}CD4^+$ T cells and "Y" is the percentage of $CD25^+FOXP3^+CD4^+$ T cells (right graph in Figure **8).** The long-term progression free survival group (LR) indicates a group of patients who did not exhibit exacerbation over 500 days or longer. The short-term responder group (SR) indicates a group of patients who were a part of a partial responder group (PR) or stable disease group (SD) for at least 9 weeks from the start of therapy, but subsequently exhibited progression in disease. The non-responder group (NR) indicates a group of patients with progression in disease within 9 weeks after the start of nivolumab therapy. Paired Student's t-test was used for statistical processing.

It was found that both the percentage of $CD62L^{low}CD4^+$ T cells (left graph in Figure **8)** and $X^2/Y$ wherein the percentage of $CD62L^{low}CD4^+$ T cells is "X" and the percentage of $CD25^+FOXP3^+CD4^+$ T cells is "Y" (right graph in Figure **8**) are excellent indicators for predicting a long-term progression free survival group (LR) and a short-term responder group (SR) and thus are excellent indicators for predicting long-term survival.

In view of these results, if 500-day progression free survival is calculated as a long-term survivor, sensitivity of 50% and specificity of 88.1% were achieved and P < 0.0001 when %CD62L$^{low}$CD4$^+$ T cell > 35.3%. Sensitivity of 62.5% and specificity of 84.2% were achieved and P < 0.0001 when X$^2$/Y > 404.5.

Furthermore, if samples were increased and the threshold value of the prediction formula was set to 323.5, where the likelihood ratio of the ROC curve was at the maximum, sensitivity and specificity were 68.2% and 81.7%, respectively (Figure **15**).

In view of the above results, long-term survival by cancer immunotherapy is predicted if X$^2$/Y is greater than or equal to a certain numerical value (e.g., X$^2$/Y > 323.5, X$^2$/Y > 404.5, or the like).

As described in WO 2018/147291, a patient is predicted to be a part of a non-responder group if X$^2$/Y is less than a certain numerical value. Thus, when administering therapy by cancer immunotherapy, X (percentage of CD62L$^{low}$CD4$^+$ cells) and Y (percentage of CD25$^+$FOXP3$^+$CD4$^+$ T cells) can used and constant "$\alpha$" can be determined in advance to determine, if X$^2$/Y < $\alpha$, that an effect from cancer immunotherapy cannot be expected and/or therapy should be changed to another therapeutic method and/or concomitant use with another therapeutic method should be considered. Furthermore, "$\beta$" can be determined in advance in addition to "a" to determine, if $\beta$ < X$^2$/Y, that long-term survival from cancer immunotherapy is expected so that, for example, therapy can or should be ended after one administration. Alternatively, it can be determined that at a numerical value therebetween, i.e., $\alpha \leq$ X$^2$/Y $\leq \beta$, cancer immunotherapy achieves somewhat of an effect, but continuation of therapy or concomitant use with another therapeutic method should be considered in order to attain long-term survival.

A cutoff value from a ROC curve can be determined using a method known in the art. Examples thereof include an approach of using the "value where the likelihood ratio is at the maximum" described above as a threshold value, as well as a method of using a value of a point resulting in the minimum distance from the top left corner of the graph, and a method of using a value of a point that maximizes the Youden Index (sensitivity + specificity - 1) (http://www.med.osaka-u.ac.jp/pub/kid/clinicaljournalclub6.html, http://www.snap-tck.com/room04/c01/stat/stat09/stat0902.html) .

(Discussion 1)

It is understood that a CD62L$^{low}$CD4$^+$ cell subpopulation is a CD4$^+$ T cell subpopulation correlated with dendritic cell stimulation in an antitumor immune response, with decreased expression of a homing molecule to a secondary lymphoid organ. It is understood that an ICOS$^+$CD62L$^{low}$CD4$^+$ T cell subpopulation is also a CD4$^+$ T cell subpopulation correlated with dendritic cell stimulation in an antitumor immune response. Since a HLA-DR$^+$CD141$^+$CD11c$^+$ dendritic cell subpopulation is a dendritic cell subpopulation that increases due to an increase in a cell subpopulation with decreased expression of a homing molecule in a CD4$^+$ T cell population, it is understood to be a dendritic cell subpopulation correlated with dendritic cell stimulation in an antitumor immune response .

The results described above suggest that long-term survival in cancer immunotherapy can be predicted by using a CD4$^+$ T cell subpopulation correlated with dendritic cell stimulation in an antitumor immune response and/or a dendritic cell subpopulation correlated with dendritic cell stimulation in an antitumor immune response.

Further, as shown in Examples 8 and 9, a patient who continues to be a part of a responder group after cancer immunotherapy can be selected by using the percentage of CD62L$^{low}$CD4$^+$ T cells or X$^2$/Y wherein the percentage of CD62L$^{low}$CD4$^+$ T cells is "X" and the percentage of CD25$^+$FOXP3$^+$CD4$^+$ T cells is "Y".

T cell subpopulations that are strongly positively correlated with a CD62L$^{low}$CD4$^+$ cell subpopulation are type 1 helper CD4$^+$ T cells (Th1), effector memory CD4$^+$ T cells, CD8$^+$ T cells, and effector CD8$^+$ T cells. They are cell subpopulations that are important for the cell killing function in cell-mediated immunity. Meanwhile, type 2 helper CD4$^+$ T cells (Th2) and regulatory T cells have a negative correlation. These are known as cell subpopulations that suppress cell-mediated immunity. Accordingly, an increase in the CD62L$^{low}$CD4$^+$ cell subpopulation indicates activation of antitumor cell-mediated immunity and a decrease in a cell subpopulation that obstructs such activation. The CD62L$^{low}$CD4$^+$ cell subpopulation controls the antitumor immune function by having a significant correlation with LAG3, ICOS, PD-1, or CTLA-4 expression on CD4$^+$ T cells or CD8$^+$ T cells. Specifically, an increase in the CD62L$^{low}$CD4$^+$ cell subpopulation correlates with an increase in PD-1, LAG-3, or ICOS expression and a decrease in CTLA-4 expression. This indicates that antitumor immunity is primarily regulated by PD-1 or LAG-3, and is thus understood to be associated with the efficacy of immune checkpoint inhibition therapy thereof. Furthermore, the HLA-DR$^+$CD141$^+$CD11c$^+$ dendritic cell subpopulation and CD62L$^{low}$CD4$^+$ cell subpopulation have a positive correlation. This is understood such that expression of an MHC class II restricted antigen by an activated dendritic cell results in an increase in the CD62L$^{low}$CD4$^+$ cell subpopulation which recognizes MHC class II restricted antigens. It is understood that the cell subpopulation is a CD4$^+$ T cell subpopulation correlated with dendritic cell stimulation in tumor immune response. It is understood that the HLA-DR$^+$CD141$^+$CD11c$^+$ dendritic cell subpopulation is a dendritic cell subpopulation correlated with dendritic cell stimulation in a tumor immune response.

(Discussion 2)

Study on cancer immunotherapy has focused on CD8[+] T cells in a tumor microenvironment. This is due to CTL differentiating from CD8[+] T cells and inducing tumor cell death by recognizing tumor antigens. However, the inventor demonstrated that: the immunological state of CD4[+] T cells in peripheral blood is an important factor that determines the clinical result of PD-1 blocking therapy in NSCLC patients; and a formula that is dependent on the ratio between the ratio of CD62L[low] T cells and the ratio of CD25[+]FOXP3[+]CD4[+] T cells can distinguish not only non-responders, but also long-term survivors. Since support of CD4[+] T cells promotes CTL priming, motility, cytotoxic activity, and survival, evidence indicating the need of CD4[+] T cells in effective antitumor immunity is mounting. In addition, it appears that CD4[+] T cells need to be present systemically in order to enhance CTL production, delivery, and cytotoxic activity. Spitzer et al. (Spitzer MH et al., Cell 2017; 168(3): 487-502 e15), by using mass cytometry, studied tumor infiltrating lymphocytes, tumor-draining lymph nodes, peripheral blood, spleen, and bone marrow of mice that have established antitumor immunity sufficient to eradicate tumor to find that the CD62L[low]CD27-T-bet[+]CD44[+]CD69[+]CD90[+]CD4[+] T cell cluster is highly concentrated at all studied sites and mediates antitumor responses. The authors also demonstrated that continuous recruitment of antitumor T cells via peripheral blood is required for persistent antitumor responses. Meanwhile, Wei et al. (Wei SC et al., Cell 2017; 170(6): 1120-33 e17) studied melanoma infiltrating lymphocytes to demonstrate that CD4[+] T cells (CD62L[low]CD27-FOXP3-CD44[+]CXCR3[+]ICOS[+]T-bet[+]) of nearly the same phenotype correlate with responses to an immune checkpoint inhibitor. Consistent with these studies, the study of the inventor using mass cytometry demonstrated that most CD62L[low]CD4[+] T cells are T-bet[+], CD27[-], FOXP3[-], and CXCR3[+] in a CD4[+] population. Meanwhile, CCR7-CD4[+] T cell subpopulations that do not exhibit a difference between non-responders and responders included broader subpopulations such as T-bet[-], CD27[+], and FOXP3[+] subpopulations. A CD62L[low]CD4[+] T cell subpopulation has a strong correlation with CXCR3[+]CCR4-CCR6- cells, i.e., a CD62L[low]CD4[+] T cell subpopulation serves an important role as Th1 cells in cell-mediated immunity. Thus, CD62L[low]CD4[+] T cell subpopulations had a positive correlation with the ratio of effector CD8[+] T cells. Interestingly, CD62L[low]CD4[+] T cell subpopulations had a positive correlation with PD-1 expression, but had a negative correlation with CTLA-4 expression. Thus, it is understood that CD62L[low]CD27- FOXP3-CXCR3[+]T-bet[+]CD4[+] T cell sub groups constitute a cell-mediated immunity T cell network including Th1 T cells and effector CD8[+] T cells, suggesting that these cells are regulated by PD-1, not CTLA-4.

It was unexpected to observe that the ratio of CD62L[low]CD4[+] T cells decreases in responders, but not in non-responders after nivolumab therapy. This is because it is demonstrated that PD-1[+] effector CD8[+] T cells increase in peripheral blood after effective anti-PD-1 therapy. However, Wei et al. (Wei SC et al., Cell 2017; 170(6): 1120-33 e17) demonstrated that PD-1 blocking therapy increased only CD8[+] antitumor T cell clusters in melanoma infiltrating lymphocytes while not decreasing CD4[+] antitumor T cell clusters. Thus, PD-1 blocking therapy may not promote antitumor CD4[+] T cell proliferation. It was previously reported that tumor-mutation burden decreases in responders after nivolumab therapy. It appears that an effective PD-1 blocking therapy results in activation of the immunoediting process and reduction in cancer clones characterized by high mutation burden. For this reason, the discovery of the inventor may indicate a loss of specific effector T cells as a result of a loss of cancer associated antigens. Since only patients who had CD62L[low]CD4[+] T cells exhibited persistent antitumor responses during nivolumab therapy, one of the fundamental mechanisms of acquiring resistance may include loss of tumor associated antigen, thus depleting support of CD4[+] T cells. The study of the inventor found that patients who exhibit a high CD62L[low]CD4[+] T cell ratio before nivolumab therapy and maintain CD62L[low]CD4[+] T cell subpopulations tend to experience no progression in disease and survive for 500 days or longer. Thus, promising therapy would necessarily involve an increase in CD62L[low]CD4[+] T cell subpopulations by therapy (e.g., anti-CTLA-4 therapy) together with monitoring of peripheral blood T cell subpopulations.

Gene expression analysis elucidated that gene expression profiles differ between CD62L[high] T cells and CD62L[low]CD4[+] T cells (Figure **14a**). CD62L[low]CD4[+] T cells expressed genes encoding Aurora A, CD101, granzyme A and H, ND2, and IL-21. AURORA A is a mitotic cell, which is expressed during the G2-M phase and is required for maintaining Lck activity after TCR engagement in T cells. CD101 is expressed in T cells activated by CD3. Granzyme A and H are expressed in cells with cytotoxic activity such as CLT and natural killer cells. It is reported that activated CD4[+] T cells can express granzymes and mediate antitumor responses (Hirschhorn-Cymerman D et al., J Exp Med 2012; 209(11): 2113-26). ND2 is one of seven subunits encoded in mitochondria of NADH dehydrogenase. IL-21 is demonstrated to enhance and maintain CD8[+] T cell responses, resulting in persistent antitumor immunity. In summary, CD62L[low]CD4[+] T cell subgroups include T cells that proliferate after activation due to TCR engagement, which exhibited an effector function and enhanced cytotoxic activity of CD8[+] T cells. In particular, some genes such as CCL19, IL7, CXCR3, CLEC2A, TGFBR3, and HDAC9 were preferentially expressed in CD62L[low]CD4[+] T cells derived from PR and/or SD (Figure **14b**). CCL19 binds to CCR7 and induces certain cells in the immune system including dendritic cells and CCR7[+] central memory cells. Signaling by IL-7 (non-redundant cytokine for T cell proliferation) promotes antitumor T cell-mediated immunity. Interestingly, it has been reported that expression of IL-7 and CCL19 in CAR-T cells improves immune cell infiltration and CAR-T survival in tumor (Adachi K et al., Nat Biotechnol 2018; 36(4): 346-51). CLEC2A enhances proliferation of T cells stimulated by TCR by improving the survival rate thereof. TGFβ has a broad range of regulatory activities affecting multiple types of immune cells. Soluble TGFBR3 may inhibit TGFβ signaling. HDAC9 regulates FOXP3 expression and suppresses Treg function. For this reason, these molecules appear to serve a role of promoting T cell activation, inhibiting a regulatory mechanism, and increasing the antitumor effector T cell count. These may represent promising targets for enhancing antitumor immunotherapy.

In conclusion, the inventor demonstrated that monitoring of systemic CD4$^+$ T cell-mediated immunity using peripheral blood is instrumental in predicting responses to anti-PD-1 therapy. The inventor developed a formula that can act as a biomarker for predicting therapeutic results based on the ratios of CD62L$^{low}$CD4$^+$ T cells and Treg. The discovery of the inventor can have critical clinical significance because the discovery assists in the preparation of anti-PD-1 therapy for all potential responders and provides the basis of new therapeutic strategies for patients exhibiting different CD4$^+$ T cell immunological state.

The above results show that long-term survival in cancer immunotherapy can be predicted by using a CD4$^+$ T cell subpopulation correlated with dendritic cell stimulation in an antitumor immune response and/or a dendritic cell subpopulation correlated with dendritic cell stimulation in an antitumor immune response.

(Example 10: Therapy with pembrolizumab)

It was confirmed that therapy using pembrolizumab also achieves the same result as nivolumab therapy as follows.

10-1 Materials and methods

(1) Patients

The following experiment was conducted on 54 18-year-old or older stage IV or IIIB-C PD NSCLC patients with a TPS (Tumor Proportion Score) of PD-L1 of 50% or greater without an EGFR mutation/ALK translocation from March 2017 to November 2018 at a single facility (Saitama Medical University International Medical Center). TPS of PD-L1 was calculated in accordance with a conventional method using PD-L1 IHC 22C3 pharmDx.

Patients were administered with a dose of 200 mg/kg of pembrolizumab every 3 weeks as a first-line therapy. Samples were collected after obtaining a written informed consent approved by the institutional review board of the Saitama Medical University International Medical Center.

49 patients were analyzed after excluding 5 patients who could not be evaluated. The overall response rate (ORR) combining complete response (CR) and partial response (PR) was 45.7%, and the disease control rate (DCR) combining complete response (CR), partial response (PR), and stable disease (SD) was 73.9% (Figure **21A**).

(2) Analysis of blood samples and (4) Statistical analysis were performed in the same manner as (Materials and methods) in Examples 1 to 9 described above.

10-2. Result of pembrolizumab therapy

Figures **21B** and **21C** show a progression free survival (PFS) curve and overall survival (OS) curve created by the same method as Example 1, respectively. The PFS curve and OS curve reached a tail plateau after 490 days and 637 days, respectively. The following analysis deemed groups reaching each tail plateau as long-term survival groups.

10-3. FACS Calibur analysis

Analysis was performed by the same method as Example 2. Results of finding the ratios of various cell subpopulations and finding the correlation between said ratios and PFS are shown in Table 6A, and results of studying the correlation with OS are shown in Table 6B.

[Table 6A]

## A.

| | PFS vs. CD4 | PFS vs. CD62Llow /CD4+CD3+ | PFS vs. CD62Llow CD4+ /CD3+ | PFS vs. CCR7+CD45RA+ /CD4+CD3+ | PFS vs. CCR7+CD45RA- /CD4+CD3+ | PFS vs. CCR7-CD45RA+ /CD4+CD3+ | PFS vs. CCR7-CD45RA- /CD4+CD3+ |
|---|---|---|---|---|---|---|---|
| Pearson r | -0.01606 | 0.4885 | 0.527 | -0.3445 | 0.03298 | 0.1314 | 0.4463 |
| 95% confidence interval | -0.2959 to 0.2663 | 0.2403 to 0.6767 | 0.2885 to 0.7039 | -0.5704 to -0.07006 | -0.2505 to 0.3113 | -0.1556 to 0.3979 | 0.1888 to 0.6464 |
| R squared | 0.0002579 | 0.2386 | 0.2777 | 0.1187 | 0.001088 | 0.01726 | 0.1992 |
| P value (two-tailed) | 0.9128 | 0.0004 | 0.0001 | 0.0154 | 0.822 | 0.3682 | 0.0013 |
| P value summary | ns | *** | *** | * | ns | ns | ** |
| Significant? (alpha = 0.05) | No | Yes | Yes | Yes | No | No | Yes |
| Number of XY Pairs | 49 | 49 | 49 | 49 | 49 | 49 | 49 |

| | PFS vs. CD8+ | PFS vs. CD62Llow /CD8+CD3+ | PFS vs. CCR7+CD45RA+ /CD8+ | PFS vs. CCR7+CD45RA- /CD8+ | PFS vs. CCR7-CD45RA+ /CD8+ | PFS vs. CCR7-CD45RA- /CD8- |
|---|---|---|---|---|---|---|
| Pearson r | -0.07892 | -0.0829 | -0.1024 | 0.1377 | -0.1275 | 0.1812 |
| 95% confidence interval | -0.3523 to 0.2069 | -0.3558 to 0.2030 | -0.3728 to 0.1841 | -0.1493 to 0.4033 | -0.3946 to 0.1594 | -0.1054 to 0.4400 |
| R squared | 0.006228 | 0.006873 | 0.01048 | 0.01896 | 0.01626 | 0.03283 |
| P value (two-tailed) | 0.5899 | 0.5712 | 0.4839 | 0.3455 | 0.3826 | 0.2128 |
| P value summary | ns | ns | ns | ns | ns | ns |
| Significant? (alpha = 0.05) | No | No | No | No | No | No |
| Number of XY Pairs | 49 | 49 | 49 | 49 | 49 | 49 |

| | PFS vs. CXCR5+ /EM+CM CD4+ | PFS vs. CXCR3+CCR6- /EM+CM CD4+ | PFS vs. CCR4+CXCR3-CCR6- /EM+CM CD4+ | PFS vs. CXCR3-CCR6+ /EM+CM CD4+ | PFS vs. CD25+Foxp3- /CD4+ |
|---|---|---|---|---|---|
| Pearson r | 0.1555 | 0.1264 | -0.3015 | 0.2637 | -0.1569 |
| 95% confidence interval | -0.1315 to 0.4184 | -0.1605 to 0.3936 | -0.5372 to -0.02221 | -0.01885 to 0.5073 | -0.4195 to 0.1301 |
| R squared | 0.02417 | 0.01597 | 0.09091 | 0.06956 | 0.0246 |
| P value (two-tailed) | 0.2861 | 0.3869 | 0.0353 | 0.0671 | 0.2818 |
| P value summary | ns | ns | * | ns | ns |
| Significant? (alpha = 0.05) | No | No | Yes | No | No |
| Number of XY Pairs | 49 | 49 | 49 | 49 | 49 |

[Table 6B]

## B.

| | OS vs. CD4 | OS vs. CD62Llow /CD4+CD3+ | OS vs. CD62L lowCD4+ /CD3+ | OS vs. CCR7+CD45RA+ /CD4+CD3+ | OS vs. CCR7+CD45RA- /CD4+CD3+ | OS vs. CCR7-CD45RA+ /CD4+CD3+ | OS vs. CCR7-CD45RA- /CD4+CD3+ |
|---|---|---|---|---|---|---|---|
| Pearson r | 0.08876 | 0.3664 | 0.3788 | -0.1914 | -0.09773 | 0.02026 | 0.3406 |
| 95% confidence interval | -0.1974 to 0.3610 | 0.09496 to 0.5871 | 0.1092 to 0.5964 | -0.4485 to 0.09490 | -0.3688 to 0.1887 | -0.2624 to 0.2997 | 0.06567 to 0.5674 |
| R squared | 0.007879 | 0.1342 | 0.1435 | 0.03663 | 0.009551 | 0.0004103 | 0.116 |
| P value (two-tailed) | 0.5442 | 0.0096 | 0.0073 | 0.1877 | 0.5041 | 0.8901 | 0.0166 |
| P value summary | ns | ** | ** | ns | ns | ns | * |
| Significant? (alpha = 0.05) | No | Yes | Yes | No | No | No | Yes |
| Number of XY Pairs | 49 | 49 | 49 | 49 | 49 | 49 | 49 |

| | OS vs. CD8+ | OS vs. CD62Llow /CD8+CD3+ | OS vs. CCR7+CD45RA+ /CD8+ | OS vs. CCR7+CD45RA- /CD8+ | OS vs. CCR7-CD45RA+ /CD8+ | OS vs. CCR7-CD45RA- /CD8+ |
|---|---|---|---|---|---|---|
| Pearson r | -0.1509 | -0.1546 | 0.07195 | 0.09578 | -0.179 | 0.0643 |
| 95% confidence interval | -0.4145 to 0.1360 | -0.4177 to 0.1323 | -0.2136 to 0.3461 | -0.1905 to 0.3671 | -0.4381 to 0.1076 | -0.2209 to 0.3394 |
| R squared | 0.02278 | 0.02391 | 0.005176 | 0.009174 | 0.03203 | 0.004135 |
| P value (two-tailed) | 0.3006 | 0.2887 | 0.6232 | 0.5127 | 0.2185 | 0.6607 |
| P value summary | ns | ns | ns | ns | ns | ns |
| Significant? (alpha = 0.05) | No | No | No | No | No | No |
| Number of XY Pairs | 49 | 49 | 49 | 49 | 49 | 49 |

| | OS vs. CXCR5+ /EM+CM CD4+ | OS vs. CXCR3+CCR6- /EM+CM CD4+ | OS vs. CCR4+CXCR3-CCR6- /EM+CM CD4 | OS vs. CXCR3-CCR6+ /EM+CM CD4 | OS vs. CD25+Foxp3+ /CD4+ |
|---|---|---|---|---|---|
| Pearson r | 0.1847 | 0.08262 | -0.3053 | 0.2268 | -0.2237 |
| 95% confidence interval | -0.1018 to 0.4429 | -0.2033 to 0.3556 | -0.5401 to -0.02633 | -0.05815 to 0.4775 | -0.4750 to 0.06132 |
| R squared | 0.03412 | 0.006826 | 0.09319 | 0.05142 | 0.05006 |
| P value (two-tailed) | 0.2039 | 0.5725 | 0.0329 | 0.1172 | 0.1223 |
| P value summary | ns | ns | * | ns | ns |
| Significant? (alpha = 0.05) | No | No | Yes | No | No |
| Number of XY Pairs | 49 | 49 | 49 | 49 | 49 |

While significant correlation was found in CD62L$^{Low}$/CD4$^+$CD3$^+$, CD62L$^{low}$CD4$^+$/CD3$^+$, and CCR7$^-$CD45RA$^-$/CD4$^+$, the strongest correlation was found in CD62L$^{low}$CD4$^+$/CD3$^+$, and a negative correlation was found with Th2.

Table 7 shows the results of comparing the ratio of a cell subpopulation in a long-term survival group on a PFS curve, the ratio of a cell subpopulation in a long-term survival group on an OS curve, and the ratio of a cell subpopulation in a non-long-term survival group.

[Table 7]

| % of / P value (two-tailed) | | | | | | | |
|---|---|---|---|---|---|---|---|
| % of | CD4+/CD3+ | CD62LlowCD4+/CD3+ | CD62Llow/CD4+CD3+ | CTLA-4/CD62LlowCD4 | ICOS/CD62LlowCD4 | LAG3/CD62LlowCD4 | PD-1/CD62LlowCD4 |
| P value (two-tailed) | 0.92 | 0.000032 | 0.0026 | 0.16 | 0.061 | 0.35 | 0.89 |
| % of | | | CCR7-CD45RA+ (EMRA)/CD4+CD3+ | CTLA-4/CCR7-CD45RA+CD4 | ICOS/CCR7-CD45RA+CD4 | LAG3/CCR7-CD45RA+CD4 | PD-1/CCR7-CD45RA-CD4 |
| P value (two-tailed) | | | 0.99 | 0.47 | 0.65 | 0.85 | 0.99 |
| % of | | | CCR7-CD45RA- (CM)/CD4+CD3+ | CTLA-4/CCR7-CD45RA+CD4 | ICOS/CCR7-CD45RA+CD4 | LAG3/CCR7-CD45RA+CD4 | PD-1/CCR7-CD45RA-CD4 |
| P value (two-tailed) | | | 0.58 | 0.088 | 0.057 | 0.27 | 0.83 |
| % of | | | CCR7-CD45RA- (EM)/CD4+CD3+ | CTLA-4/CCR7-CD45RA-CD4 | ICOS/CCR7-CD45RA-CD4 | LAG3/CCR7-CD45RA-CD4 | PD-1/CCR7-CD45RA-CD4 |
| P value (two-tailed) | | | 0.0063 | 0.12 | 0.062 | 0.39 | 0.64 |
| % of | CD8+/CD3+ | CD62LlowCD8+/CD3+ | CD62Llow/CD8+CD3+ | CTLA-4/CD62LlowCD8 | ICOS/CD62LlowCD8 | LAG3/CD62LlowCD8 | PD-1/CD62LlowCD8 |
| P value (two-tailed) | 0.77 | 0.55 | 0.93 | 0.089 | 0.28 | 0.22 | 0.23 |
| % of | | | CCR7-CD45RA+ (EMRA)/CD8+ | CTLA-4/CCR7-CD45RA+CD8 | ICOS/CCR7-CD45RA+CD8 | LAG3/CCR7-CD45RA+CD8 | PD-1/CCR7-CD45RA-CD8 |
| P value (two-tailed) | | | 0.29 | 0.10 | 0.31 | 0.35 | 0.36 |
| % of | | | CCR7+CD45RA- (CM)/CD8+ | CTLA-4/CCR7-CD45RA+CD8 | ICOS/CCR7-CD45RA+CD8 | LAG3/CCR7-CD45RA+CD8 | PD-1/CCR7-CD45RA-CD8 |
| P value (two-tailed) | | | 0.57 | 0.83 | 0.27 | 0.14 | 0.22 |
| % of | | | CCR7-CD45RA- (EM)/CD8+ | CTLA-4/CCR7-CD45RA-CD8 | ICOS/CCR7-CD45RA-CD8 | LAG3/CCR7-CD45RA-CD8 | PD-1/CCR7-CD45RA-CD8 |
| P value (two-tailed) | | | 0.013 | 0.10 | 0.11 | 0.17 | 0.13 |
| % of | CXCR3+CCR4-CCR6-(Th1)/EM+CM CD4+ | CXCR3-CCR4+CCR6-(Th2)/EM+CM CD4 | CXCR3-CCR4+CCR6-(Th17)/EM+CM CD4 | nTreg/CD4+ | eTreg/CD4+ | CD25+Foxp3 (Treg)/CD4- | |
| P value (two-tailed) | 0.33 | 0.016 | 0.44 | 0.48 | 0.52 | 0.42 | |

Data comparing patients who are long-term responders reaching a tail-plateau where PFS curve does not decrease, and patients who are not. While a significant difference is found in CD62L$^{Low}$CD4$^+$, CCR7$^-$CD45RA$^-$ CD4$^+$, and CCR7$^-$CD45RA$^-$CD8$^+$, the most significant difference is in CD62L$^{low}$CD4$^+$/CD3$^+$ .

While a significant difference was found in CD62L$^{Low}$CD4$^+$ /CD3$^+$, CD62L$^{low}$/CD4$^+$CD3$^+$, CCR7$^-$CD45RA$^-$/CD4$^+$CD3$^+$, and CCR7$^-$CD45RA$^-$/CD8$^+$, the strongest correlation was found in CD62L$^{low}$CD4$^+$ /CD3$^+$.

10-4. ROC analysis, and PFS and OS plots Analysis was conducted by the same method as Example 3. The results of plotting CD62L$^{low}$CD4$^+$/CD3$^+$ on the horizontal axis and PFS or OS on the vertical axis are shown in Figure 21D and Figure 21E, respectively. Correlation was found between CD62L$^{low}$CD4$^+$ /CD3$^+$ and PFS or OS. The results of plotting CD62L$^{low}$CD4$^+$ /CD3$^+$ for PFS < 490 and PFS ≥ 490 groups and OS < 637 and OS ≥ 637 groups are shown in Figures 21F and 21H, respectively. Correlation was found in both figures.

Figure 21G shows results of ROC analysis using CD62L$^{low}$CD4$^+$ /CD3$^+$ > 17.6 as a threshold value for PFS, and Figure 21I shows results of ROC analysis using CD62L$^{low}$CD4$^+$/CD3$^+$> 15.6 as a threshold value for OS, from the results of Figures 21F and 21H.

PFS had a p value of 0.0002, sensitivity of 72.7%, specificity of 86.7%, and AUC of 0.88, and OS had a p value of 0.0065, sensitivity of 66.7%, specificity of 81.8%, and AUC of 0.77, which were all excellent. The ratios of CD62L$^{low}$CD4$^+$CD3$^+$ cell populations include ratios using CD4$^+$CD3$^+$ as the parent population (CD62L$^{low}$/CD4$^+$CD3$^+$) and ratios using CD3$^+$ as the parent population (CD62L$^{low}$CD4$^+$/CD3$^+$) (cells described in the numerator comprise all of the features of the cells described in the denominator), which exhibited similar results. The results indicate that sensitivity and specificity are better with CD3$^+$ as the parent population than with CD4$^+$CD3$^+$ as the parent population.

10-5. Comparison of first-line therapy using pembrolizumab with second-line therapy using nivolumab

Results of plotting CD62L$^{low}$/CD4$^+$ on the horizontal axis and PFS or OS on the vertical axis for patients subjected to first-line therapy using pembrolizumab (•) and patients subjected to second-line therapy using nivolumab (o) are shown in Figures 22A and 22B, respectively.

While PFS was better in the pembrolizumab therapy group, the rate of increase in PFS for each CD62L$^{low}$/CD4$^+$ was the same for both PFS and OS. When evaluated using CD62L$^{low}$/CD4$^+$ as an indicator, it was found that the therapeutic effect exhibited a tendency very similar to nivolumab. This result shows that the present invention provides prediction of long-term survival by any cancer immunotherapy (e.g., any immune checkpoint inhibition therapy).

[Industrial Applicability]

The present invention can be utilized in cancer therapy. The present invention can indicate whether a patient is in a long-term survival immunological state before cancer immunotherapy. The present invention can also indicate that a long-term survival immunological state is persisting after the start of cancer immunotherapy. This allows properly determining the presence/absence of a need for combination therapy, determining the timing of starting combination therapy, or determining the timing for suspending/discontinuing combination therapy.

**Claims**

1. A method of using a composition of a cell subpopulation in a sample obtained from a subject as an indicator for predicting long-term survival in cancer immunotherapy in the subject, comprising:

   a step of analyzing the composition of the cell subpopulation in the sample obtained from the subject;
   wherein long-term survival in cancer immunotherapy in the subject is predicted by comparing an amount of a CD4$^+$ T cell subpopulation correlated with dendritic cell stimulation in an antitumor immune response in the sample with a baseline.

2. A method of using a composition of a cell subpopulation in a sample obtained from a subject as an indicator for predicting long-term survival in cancer immunotherapy in the subject, comprising:

   a step of analyzing the composition of the cell subpopulation in the sample obtained from the subject;
   wherein long-term survival in cancer immunotherapy in the subject is predicted by comparing an amount of a dendritic cell subpopulation correlated with dendritic cell stimulation in an antitumor immune response in the sample with a baseline.

3. A method of using a composition of a cell subpopulation in a sample obtained from a subject as an indicator for predicting long-term survival in cancer immunotherapy in the subject, comprising:

   a step of analyzing the composition of the cell subpopulation in the sample obtained from the subject;
   wherein long-term survival in cancer immunotherapy in the subject is predicted by comparing an amount of a CD8$^+$ T cell subpopulation correlated with dendritic cell stimulation in an antitumor immune response in the sample with a baseline.

4. The method of any one of claims 1 to 3, wherein the long-term survival in cancer immunotherapy in the subject is predicted by comparing at least two amounts selected from the group consisting of an amount of a CD4$^+$ T cell subpopulation correlated with dendritic cell stimulation in an antitumor immune response, an amount of a dendritic cell subpopulation correlated with dendritic cell stimulation in an antitumor immune response, and an amount of a CD8$^+$ T cell subpopulation correlated with dendritic cell stimulation in an antitumor immune response in the sample with a baseline.

5. The method of claim 1 or 4, wherein the CD4$^+$ T cell subpopulation correlated with dendritic cell stimulation in an antitumor immune response is a cell subpopulation within a CD62L$^{low}$CD4$^+$ T cell population.

6. The method of claim 5, wherein the CD4$^+$ T cell subpopulation correlated with dendritic cell stimulation in an antitumor immune response is a CD62L$^{low}$CD4$^+$ T cell subpopulation.

7. The method of claim 5, wherein the CD4$^+$ T cell subpopulation correlated with dendritic cell stimulation in an antitumor immune response is an ICOS$^+$CD62L$^{low}$CD4$^+$ T cell subpopulation.

8. The method of claim 2 or 4, wherein the dendritic cell subpopulation correlated with dendritic cell stimulation in an antitumor immune response is an HLA-DR$^+$CD141$^+$CD11c$^+$ cell subpopulation.

9. The method of claim 3 or 4, wherein the CD8$^+$ T cell subpopulation correlated with dendritic cell stimulation in an antitumor immune response is a cell subpopulation within a CD62L$^{low}$CD8$^+$ T cell population.

10. The method of claim 9, wherein the CD8$^+$ T cell subpopulation correlated with dendritic cell stimulation in an antitumor immune response is a CD137$^+$CD62L$^{low}$CD8$^+$ T cell subpopulation.

11. A method of using a relative value with respect to amounts (X, Y) selected from the group consisting of:

an amount of a CD4$^+$ T cell subpopulation correlated with dendritic cell stimulation in an antitumor immune response;

an amount of a dendritic cell subpopulation correlated with dendritic cell stimulation by a CD4$^+$ T cell in an antitumor immune response;

an amount of a CD8$^+$ T cell subpopulation correlated with dendritic cell stimulation in an antitumor immune response;

an amount of regulatory T cells or a CD4$^+$ T cell subpopulation correlated with regulatory T cells; and

an amount of an ICOS$^+$CD62L$^{low}$CD4$^+$ T cell subpopulation; as an indicator for predicting long-term survival in cancer immunotherapy in the subject; comprising:

a step of measuring X; and

a step of measuring Y;

wherein comparison of a relative value of X to Y with a baseline is used as an indicator for predicting long-term survival in cancer immunotherapy in the subject.

12. The method of claim 11, wherein the amounts (x) and (Y) are each selected from the group consisting of:

an amount of a CD62L$^{low}$CD4$^+$ T cell subpopulation;

an amount of a CCR7$^-$CD4$^+$ T cell subpopulation;

an amount of a LAG-3$^+$CD62L$^{low}$CD4$^+$ T cell subpopulation;

an amount of an ICOS$^+$CD62L$^{low}$CD4$^+$ T cell subpopulation;

an amount of a PD-1$^+$CD62L$^{low}$CD4$^+$ T cell subpopulation;

an amount of a Foxp3$^+$CD25$^+$CD4$^+$ T cell subpopulation;

an amount of an HLA-DR$^+$ dendritic cell subpopulation;

an amount of a CD80$^+$ dendritic cell subpopulation;

an amount of a CD86$^+$ dendritic cell subpopulation;

an amount of a PD-L1$^+$ dendritic cell subpopulation;

an amount of a CD62L$^{low}$CD8$^+$ T cell subpopulation;

an amount of a CD137$^+$CD8$^+$ T cell subpopulation; and

an amount of a CD28$^+$CD62L$^{low}$CD8$^+$ T cell subpopulation.

13. The method of claim 11, wherein

the amount (X) is an amount of a CD62L$^{low}$CD4$^+$ T cell subpopulation, and

(Y) is an amount of a Foxp3$^+$CD25$^+$CD4$^+$ T cell subpopulation.

14. The method of any one of claims 11 to 13, wherein the relative value is X/Y.

15. The method of any one of claims 11 to 13, wherein the relative value is $X^2$/Y.

16. The method of any one of claims 1 to 15, wherein the sample is a peripheral blood sample.

17. The method of any one of claims 1 to 16, wherein the baseline is an amount of the cell subpopulation in the sample of the subject before the cancer immunotherapy or a predetermined value.

18. The method of any one of claims 1 to 17, wherein the amount of the cell subpopulation in the sample which is greater than the baseline indicates that long-term survival in cancer immunotherapy in the subject is predicted.

19. The method of any one of claims 1 to 17, wherein the amount of the cell subpopulation in the sample which is less than the baseline indicates that long-term survival in cancer immunotherapy in the subject is not predicted.

20. The method of any one of claims 1 to 19, wherein no prediction of long-term survival in cancer immunotherapy in the subject further indicates that combination therapy should be administered to the subject.

21. The method of any one of claims 1 to 20 further defined as a method of using a composition of a cell subpopulation in a sample obtained at a plurality of points in time from a subject as an indicator for predicting long-term survival in cancer immunotherapy in the subject, the method comprising a step of analyzing the composition of the cell subpopulation in the sample obtained at the plurality of points in time from the subject.

22. The method of claim 15, wherein long-term survival is predicted if $X^2/Y$ is about 324 or greater.

23. A pharmaceutical composition comprising an immune checkpoint inhibitor for treating cancer in a subject, wherein the pharmaceutical composition is administered to a subject predicted to have long-term survival in cancer immunotherapy in the subject by the method of any one of claims 1 to 18 and 21 to 22.

24. The pharmaceutical composition of claim 23, wherein the immune checkpoint inhibitor is a PD-1 inhibitor and/or a PD-L1 inhibitor.

25. A combination drug comprising an immune checkpoint inhibitor for treating cancer in a subject, wherein the combination drug is administered to a subject not predicted to have long-term survival in cancer immunotherapy in the subject by the method of any one of claims 1 to 22.

26. The combination drug of claim 25, wherein the immune checkpoint inhibitor is a PD-1 inhibitor and/or a PD-L1 inhibitor.

27. The combination drug of claim 25, comprising a drug selected from the group consisting of a chemotherapeutic agent and additional cancer immunotherapy.

28. A kit for determining whether long-term survival in cancer immunotherapy in a subject is predicted, comprising a detecting agent for a combination or markers selected from the group consisting of:

    *a combination of CD4 and CD62L;
    *a combination of CD4 and CCR7;
    *a combination of CD4, CD62L, and LAG-3;
    *a combination of CD4, CD62L, and ICOS;
    *a combination of CD4, CD62L, and CD25;
    *a combination of CD4, CD127, and CD25;
    *a combination of CD4, CD45RA, and Foxp3;
    *a combination of CD4, CD25, and Foxp3;
    *a combination of CD11c, CD141, and HLA-DR;
    *a combination of CD11c, CD141, and CD80;
    *a combination of CD11c, CD123, and HLA-DR;
    *a combination of CD11c, CD123, and CD80;
    *a combination of CD8 and CD62L;
    *a combination of CD8 and CD137; and
    *a combination of CD28, CD62L, and CD8.

29. A kit for determining whether therapeutic intervention is needed in cancer immunotherapy in a subject, comprising a detecting agent for a combination or markers selected from the group consisting of:

    *a combination of CD4 and CD62L;
    *a combination of CD4 and CCR7;
    *a combination of CD4, CD62L, and LAG-3;
    *a combination of CD4, CD62L, and ICOS;
    *a combination of CD4, CD62L, and CD25;
    *a combination of CD4, CD127, and CD25;
    *a combination of CD4, CD45RA, and Foxp3;
    *a combination of CD4, CD25, and Foxp3;
    *a combination of CD11c, CD141, and HLA-DR;
    *a combination of CD11c, CD141, and CD80;
    *a combination of CD11c, CD123, and HLA-DR;
    *a combination of CD11c, CD123, and CD80;
    *a combination of CD8 and CD62L;
    *a combination of CD8 and CD137; and
    *a combination of CD28, CD62L, and CD8.

30. A method of using a composition of a subpopulation in a sample obtained from a subject as an indicator of a need for therapeutic intervention in cancer immunotherapy in the subject, comprising:

a step of analyzing the composition of the cell subpopulation in the sample obtained from the subject;
wherein an indicator of a need for therapeutic intervention in cancer immunotherapy in the subject is provided by comparing an amount of a CD4$^+$ T cell subpopulation correlated with dendritic cell stimulation in an antitumor immune response in the sample with a baseline.

31. The method of claim 30, wherein the CD4$^+$ T cell subpopulation correlated with dendritic cell stimulation in an antitumor immune response is a cell subpopulation within a CD62L$^{low}$CD4$^+$ T cell population.

32. The method of claim 30, wherein the CD4$^+$ T cell subpopulation correlated with dendritic cell stimulation in an antitumor immune response is a CD62L$^{low}$CD4$^+$ T cell subpopulation.

33. A method of using a relative value with respect to amounts (X, Y) selected from the group consisting of:

an amount of a CD4$^+$ T cell subpopulation correlated with dendritic cell stimulation in an antitumor immune response;
an amount of a dendritic cell subpopulation correlated with dendritic cell stimulation by a CD4$^+$ T cell in an antitumor immune response;
an amount of a CD8$^+$ T cell subpopulation correlated with dendritic cell stimulation in an antitumor immune response;
an amount of regulatory T cells or a CD4$^+$ T cell subpopulation correlated with regulatory T cells; and
an amount of an ICOS$^+$CD62L$^{low}$CD4$^+$ T cell subpopulation; as an indicator of a need for therapeutic intervention in cancer immunotherapy in the subject; comprising:

a step of measuring X; and
a step of measuring Y;
wherein comparison of a relative value of X to Y with a baseline is used as an indicator of a need for therapeutic intervention in cancer immunotherapy in the subject.

34. The method of claim 33, wherein the amounts (x) and (Y) are each selected from the group consisting of:

an amount of a CD62L$^{low}$CD4$^+$ T cell subpopulation;
an amount of a CCR7$^-$CD4$^+$ T cell subpopulation;
an amount of a LAG-3$^+$CD62L$^{low}$CD4$^+$ T cell subpopulation;
an amount of an ICOS$^+$CD62L$^{low}$CD4$^+$ T cell subpopulation;
an amount of a PD-1$^+$CD62L$^{low}$CD4$^+$ T cell subpopulation;
an amount of a Foxp3$^+$CD25$^+$CD4$^+$ T cell subpopulation;
an amount of an HLA-DR$^+$ dendritic cell subpopulation;
an amount of a CD80$^+$ dendritic cell subpopulation;
an amount of a CD86$^+$ dendritic cell subpopulation;
an amount of a PD-L1$^+$ dendritic cell subpopulation;
an amount of a CD62L$^{low}$CD8$^+$ T cell subpopulation;
an amount of a CD137$^+$CD8$^+$ T cell subpopulation; and
an amount of a CD28$^+$CD62L$^{low}$CD8$^+$ T cell subpopulation.

35. The method of claim 33, wherein

the amount (X) is an amount of a CD62L$^{low}$CD4$^+$ T cell subpopulation, and
(Y) is an amount of a Foxp3$^+$CD25$^+$CD4$^+$ T cell subpopulation.

36. The method of any one of claims 33 to 35, wherein the relative value is X/Y.

37. The method of any one of claims 33 to 35, wherein the relative value is X$^2$/Y.

38. The method of any one of claims 30 to 37, wherein the therapeutic intervention is radiation therapy.

39. The method of any one of claims 30 to 37, wherein the therapeutic intervention is chemotherapeutic agent therapy.

40. The method of claim 37, wherein X$^2$/Y of less than about 324 is an indicator of a need for therapeutic intervention.

41. The method of claim 37, wherein $X^2/Y$ of about 174 or greater and less than about 324 is an indicator of a need for therapeutic intervention, wherein the therapeutic intervention comprises chemotherapy, radiation therapy, a surgical procedure, hyperthermia therapy, or additional cancer immunotherapy in addition to cancer immunotherapy being administered.

42. The method of claim 37, wherein $X^2/Y$ of less than about 174 is an indicator of a need for therapeutic intervention, wherein the therapeutic intervention comprises discontinuation of cancer immunotherapy being administered, or chemotherapy, radiation therapy, a surgical procedure, hyperthermia therapy, or additional cancer immunotherapy in addition to cancer immunotherapy being administered.

43. A combination drug comprising an immune checkpoint inhibitor for treating cancer in a subject, wherein the combination drug is administered to a subject determined as needing therapeutic intervention in cancer immunotherapy in the subject by the method of any one of claims 30 to 42.

44. The combination drug of claim 43, wherein the immune checkpoint inhibitor is a PD-1 inhibitor and/or a PD-L1 inhibitor.

45. The combination drug of claim 43, comprising a drug selected from the group consisting of a chemotherapeutic agent and additional cancer immunotherapy.

46. A method of using a composition of a cell subpopulation in a sample obtained from a subject who is a cancer patient before therapy as an indicator for determining a therapeutic strategy for the subject, comprising:

a step of measuring an amount of a $CD62L^{low}CD4^+$ T cell subpopulation in the sample obtained from the subject (X) and an amount of a $Foxp3^+CD25^+CD4^+$ T cell subpopulation (Y);
a step of finding a relative value $X^2/Y$; and
a step selected from the group consisting of:

(a) a step of setting threshold value $\alpha$ for relative value $X^2/Y$ and determining a subject as a non-responder to cancer immunotherapy if $X^2/Y$ is less than threshold value $\alpha$;
(b) a step of setting threshold values $\alpha$ and $\beta$ for relative value $X^2/Y$ wherein $\alpha < \beta$, and determining a subject as a short-term responder to cancer immunotherapy if $X^2/Y$ is threshold value $\alpha$ or greater and less than threshold value $\beta$; or
(c) a step of setting threshold value $\beta$ for relative value $X^2/Y$ and determining a subject as a long-term responder to cancer immunotherapy if $X^2/Y$ is threshold value $\beta$ or greater.

47. The method of claim 46, wherein threshold value $\beta$ is a value that is at least 50 greater than threshold value a.

48. The method of claim 47, wherein

threshold value $\alpha$ is a value within a range from 100 to 400, and
threshold value $\beta$ is a value within a range from 150 to 450.

49. A product comprising a package insert describing the method of any one of claims 46 to 48, and an immune checkpoint inhibitor.

## FIG.1A

## FIG.1B

**Subsequent Treatment of 5-Year Survivors (n = 16)**
CA209-003 5-Year Update: Phase 1 Nivolumab in Advanced NSCLC

## FIG.2

Progression-free Survival

# FIG.3

%CD62L$^{low}$ CD4$^+$ cell (n = 91)

LS: >18m Progression free long-term survival group
R: Once responding but later with disease progression group

$P = 5.1 \times 10^{-11}$

$P = 0.022$

# FIG.4

Progression free for 18 months or longer

AUC 0.896
$P = 0.0008$
%CD62L$^{low}$/CD4+>35.85%
Sensitivity 85.7%
Specificity 83.3%

# FIG.5

%CD62L$^{low}$ CD8$^+$ T cell

$P = 0.034$

%CD28$^+$ CD62L$^{low}$CD8+

$P = 0.014$

pre-Nivo  post-Nivo

pre-Nivo  post-Nivo

%CD62L$^{low}$ CD4$^+$ T cell

$P = 0.0001$

%ICOS$^+$ CD62L$^{low}$CD4+

$P = 0.0025$

%LAG3$^+$ CD62L$^{low}$CD4+

$P = 0.0495$

pre-Nivo  post-Nivo

pre-Nivo  post-Nivo

pre-Nivo  post-Nivo

FIG.6

Responder

$P = 0.0016$

Non-responder

$P = 0.32$

FIG.7

Weeks post Nivo    64.5W (12-92W)    63.3W (28-92W)

FIG.8

$P < 0.0001$
$P = 0.04$    $P < 0.0001$

$P < 0.0001$
$P = 0.02$    $P < 0.0001$

FIG.9

# FIG.10

# FIG.11

FIG.12

# FIG.13

FIG.14

EP 3 928 793 A1

a.

CD62L^high CD4+          CD62L^low CD4+

PR   SD   PD    PR   SD   PD

| CD62L^low > CD62L^high | CD62L^low < CD62L^high |
|---|---|
| AURAKA | BACH2 |
| CCL17 | CCL28 |
| CD101 | CCR7 |
| CD24 | CD27 |
| FOXF1 | CD28 |
| GZMA | CD62L |
| GZMH | CSNK1D |
| IL18RAP | FOXP1 |
| IL21 | FOXP3 |
| IL5RA | IGF1R |
| ND2 | IL16 |
| SMAD5 | IL27RA |
| SMAD7 | IL6R |
| VEGFA | LEF1 |
|  | MAL |
|  | TCF7 |

b.

2.0    0.0    2.0

CD62L^low CD4+

PR   SD   PD

CCL19
CCL3
CCL7
CD80
CLEC2A
CTAGE1
EPS8
ERBB3
FGF5
FKBP14
FOXA1
FOXB1
IFNA10
IFNA17
IFNA4
IL11
IL13RA2
IL23R
IL7
PDCD1LG2
TGFBR3
TIAM2

CXCR3
FKBP7
HDAC9
IFNA8
IL34
NRCAM
XCL1
XCR1

| | PR > PD | PR > SD | PR+SD > PD |
|---|---|---|---|
| CCL19 |  | + |  |
| CCL3 | + | + | + |
| CCL7 |  | + |  |
| CD80 | + | + |  |
| CLEC2A | + | + | + |
| CTAGE1 | + | + |  |
| EPS8 | + | + |  |
| ERBB3 | + | + | + |
| FGF5 | + | + |  |
| FKBP14 |  | + |  |
| FOXA1 | + | + | + |
| FOXB1 | + |  |  |
| IFNA10 | + | + | + |
| IFNA17 | + |  |  |
| IFNA4 | + | + | + |
| IL11 |  | + |  |
| IL13RA2 | + | + | + |
| IL23R | + | + |  |
| IL7 |  | + |  |
| PDCD1LG2 | + | + |  |
| TGFBR3 | + |  | + |
| TIAM2 | + | + |  |
| CXCR3 | + |  | + |
| FKBP7 | + |  | + |
| HDAC9 | + |  | + |
| IFNA8 | + |  | + |
| IL34 | + |  | + |
| NRCAM |  |  | + |
| XCL1 | + |  | + |
| XCR1 |  |  | + |

# FIG.15

AUC 0.79
P < 0.0001
threshold < 323.5
sensitivity 68.2 %
specificity 81.7 %

# FIG.16

FIG.17

FIG.18

# FIG.19

# FIG.20

FIG.21

FIG.22

<div align="center">

**INTERNATIONAL SEARCH REPORT**

</div>

| | International application No. |
|---|---|
| | PCT/JP2020/006620 |

**A. CLASSIFICATION OF SUBJECT MATTER**
A61K 39/395(2006.01)i; A61K 45/00(2006.01)i; A61P 35/00(2006.01)i; A61P
43/00(2006.01)i; C07K 16/28(2006.01)i; C12Q 1/02(2006.01)i; C12Q
1/06(2006.01)i; G01N 33/48(2006.01)i; G01N 33/49(2006.01)i; G01N
33/68(2006.01)i
FI: G01N33/48 M; G01N33/68; C12Q1/06; A61K39/395 T; A61K39/395 U;
A61K45/00; A61P35/00; A61P43/00 121; C12Q1/02; C07K16/28;
G01N33/49 A

According to International Patent Classification (IPC) or to both national classification and IPC

**B. FIELDS SEARCHED**

Minimum documentation searched (classification system followed by classification symbols)
A61K39/395; A61K45/00; A61P35/00; A61P43/00; C07K16/28; C12Q1/02;
C12Q1/06; G01N33/48; G01N33/49; G01N33/68

Documentation searched other than minimum documentation to the extent that such documents are included in the fields searched
Published examined utility model applications of Japan          1922–1996
Published unexamined utility model applications of Japan        1971–2020
Registered utility model specifications of Japan                1996–2020
Published registered utility model applications of Japan        1994–2020

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | WO 2018/147291 A1 (SAITAMA MEDICAL UNIVERSITY) 16.08.2018 (2018-08-16) claims, paragraphs [0001], [0060]–[0062], [0135]–[0138], [0242]–[0273] example 1, paragraphs [0279]–[0282] example 3, paragraphs [0290]–[0301] examples 6-8, paragraphs [0310]–[0314] example 10, fig. 10, 20, 30 | 1-49 |
| X | WO 2017/140826 A1 (INSTITUT GUSTAVE ROUSSY) 24.08.2017 (2017-08-24) claims, pp. 45-48, 62-70, fig. 1-21 | 1, 3-4, 11-12, 16-21, 23-30, 33-34, 38-39, 43-45 |
| A | | 2, 5-10, 13-15, 22, 31-32, 35-37, 40-42, 46-49 |

☒ Further documents are listed in the continuation of Box C.    ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 24 April 2020 (24.04.2020) | 12 May 2020 (12.05.2020) |

| Name and mailing address of the ISA/ Japan Patent Office 3-4-3, Kasumigaseki, Chiyoda-ku, Tokyo 100-8915, Japan | Authorized officer |
|---|---|
| | Telephone No. |

Form PCT/ISA/210 (second sheet) (January 2015)

**INTERNATIONAL SEARCH REPORT**

| International application No. |
| --- |
| PCT/JP2020/006620 |

C (Continuation).    DOCUMENTS CONSIDERED TO BE RELEVANT

| Category* | Citation of document, with indication, where appropriate, of the relevant passages | Relevant to claim No. |
| --- | --- | --- |
| X | WO 2018/212237 A1 (ITO, Kyogo) 22.11.2018 (2018-11-22) claims 1, 12, paragraphs [0013], [0202]-[0203], [0211]-[0212], fig. 26 | 1, 16-20, 28-30, 38-39 |
| X | WO 2018/145023 A1 (NOVARTIS AG) 09.08.2018 (2018-08-09) claims, example 3, fig. 23a-b | 1, 3, 17-21, 23-30, 38-39, 43-45 |
| A |  | 2, 4-16, 22, 31-37, 40-42, 46-49 |
| X | JP 2017-538664 A (THE REGENTS OF THE UNIVERSITY OF CALIFORNIA) 28.12.2017 (2017-12-28) example 11 | 2, 8, 17-19, 23-24, 28 |
| X | MANJARREZ-ORDUNO, N. et al., "Circulating T Cell Subpopulations Correlate With Immune Responses at the Tumor Site and Clinical Response to PD1 Inhibition in Non-Small Cell Lung Cancer", | 1, 3-7, 9-12, 14, 16-21, 23-36, 38-39, 43-45 |
| A | Frontiers in Immunology, 2018, vol.9, article 1613, pp. 1-9 in particular, abstract | 2, 8, 13, 15, 22, 37, 40-42, 46-49 |
| X | MARTENS, A. et al., "Baseline Peripheral Blood Biomarkers Associated with Clinical Outcome of Advanced Melanoma Patients", Clinical Cancer | 1, 16-21, 23-30, 38-39, 43-45 |
| A | Research, 19 January 2016 in particular, abstract | 2-15, 22, 31-37, 40-42, 46-49 |
| A | ZHANG, Met al., "D-1 blockade augments humoral immunity through ICOS-mediated CD4+ T cell instruction", International Immunopharmacology, 2019, vol. 66, pp. 127-138 in particular, abstract | 1-49 |
| A | JP 2018-530747 A (INSERM (INSTITUT NATIONAL DE LA SANTE ET DE LA RECHERCHE MEDICALE)) 18.10.2018 (2018-10-18) claims | 1-49 |

Form PCT/ISA/210 (continuation of second sheet) (January 2015)

**INTERNATIONAL SEARCH REPORT**
Information on patent family members

International application No.

PCT/JP2020/006620

| Patent Documents referred in the Report | Publication Date | Patent Family | Publication Date |
|---|---|---|---|
| WO 2018/147291 A1 | 16 Aug. 2018 | JP 6664684 B1<br>CN 110446928 A<br>KR 10-2019-0112027 A | |
| WO 2017/140826 A1 | 24 Aug. 2017 | US 2019/0331682 A1 | |
| WO 2018/212237 A1 | 22 Nov. 2018 | (Family: none) | |
| WO 2018/145023 A1 | 09 Aug. 2018 | JP 2020-509351 A<br>CN 110461342 A | |
| JP 2017-538664 A | 28 Dec. 2017 | US 2017/0291946 A1<br>example 11<br>WO 2016/049641 A1<br>KR 10-2017-0061152 A<br>CN 106999585 A | |
| JP 2018-530747 A | 18 Oct. 2018 | US 2018/0252720 A1<br>claims<br>WO 2017/032867 A1 | |

Form PCT/ISA/210 (patent family annex) (January 2015)

## REFERENCES CITED IN THE DESCRIPTION

**Patent documents cited in the description**

- WO 2018147291 A

**Non-patent literature cited in the description**

- **BRAHMER et al.** *AARC,* 2017
- **BRAHMER et al.** *N Eng J Med,* 2015, vol. 373, 123-135
- **OKAZAKI, T. ; CHIKUMA, S. ; IWAI, Y. et al.** A rheostat for immune responses: the unique properties of PD-1 and their advantages for clinical application. *Nat. Immunol.,* 2013, vol. 14, 1212-1218
- *AACR Annual Meeting,* 01 April 2017
- **SPITZER MH et al.** *Cell,* 2017, vol. 168 (3), 487-502
- **WEI SC et al.** *Cell,* 2017, vol. 170 (6), 1120-33
- **HIRSCHHORN-CYMERMAN D et al.** *J Exp Med,* 2012, vol. 209 (11), 2113-26
- **ADACHI K et al.** *Nat Biotechnol,* 2018, vol. 36 (4), 346-51